# EUROPEAN PATENT APPLICATION

(11) **EP 2 270 032 A2**
(43) Date of publication of application: **05.01.2011**
(21) Application number: 10007543.1
(22) Date of filing: 18.09.1998
(51) Int. Cl.: C07K 14/29, C12N 15/31, G01N 33/569, A61K 39/02

(54) **Outer membrane protein of Ehrlichia canis and Ehrlichia chaffeenis**

(30) Priority: 19.09.1997 US 59353 P
(62) Divisional of application: 98949384.6
(71) Applicant: The Ohio State University Research Foundation, Columbus, OH 43212 (US)
(72) Inventor: Rikihisa, Yasuko, Worthington, OH 43085 (US); Ohashi, Norio, Columbus, OH 43212 (US)
(74) Representative: Kremer, Simon Mark

(57) **Abstract**

The present invention relates to diagnostic tools for veterinary and human use which are used for serodiagnosing ehrlichiosis in mammals, particularly in members of the Canidae family and in humans. The present invention also provides polynucleotides which encode the outer membrane proteins of E. chafeensis. The polynucleotides encode an OMP-1 family of proteins of E. chafeensis and P30 family of proteins of E. canis. The present invention also provides the following isolated proteins ofE. chafeensis OMP-1, OMP-1A, OMP-1B, OMP-1C, OMP-1D, OMP-1E, OMP-1F, OMP-1R, OMP-1S, OMP-1T, OMP-1U, OMP-1V, OMP-1W, OMP-1X, and OMP-1Z, referred to hereinafter collectively as the "OMP family". The present invention also provides the following isolated proteins of E. canis P30, P30-a, P30-1, P30-2, P30-3, P30-4, P30-5, P30-6, P30-7, P30-8, P30-9, and P30-10, referred to hereinafter as the P30 family. The present invention also relates to an assay for diagnosing ehrlichiosis in humans using a recombinant outer membrane protein of E. chafeensis, particularly OMP-1. The present invention also relates to an assay for diagnosing ehrlichiosis in humans and members of the family Canidae using a recombinant outer membrane protein of E. canis, particularly P30.

## Description

This work was supported by grant RO1 AI40934 from National Institutes of Health. The government has certain rights in this invention.

### BACKGROUND OF THE INVENTION

The ehrlichiae are obligate intracellular bacteria that infect circulating leucocytes, *Ehrlichia chafeensis* infects the monocytes and macrophages in humans and causes human monocytic ehrlichiosis. The clinical manifestations of ehrlichiosis in humans are nonspecific and similar to Rocky Mountain spotted fever. The clinical manifestations include fever, chills, headache mylagia or vomiting and weight loss. Most patients have a history of tick exposure.

*Ehrlichia canis* infects and causes ehrlichiosis in animals belonging to the family Canidae. Canine ehrlichiosis consists of an acute and a chronic phase. The acute phase is characterized by fever, serous nasal and ocular discharges, anorexia, depression, and loss of weight. The chronic phase is characterized by severe pancytopenia, epistaxis, hematuria, blood in feces in addition to more severe clinical signs of the acute disease. If treated early during the course of the disease, dogs respond well to doxycycline. However, chronically infected dogs do not respond well to the antibiotic. Therefore, early diagnosis is very important for treating canine ehrlichiosis.

The primary diagnostic test for diagnosing canine ehrlichiosis and human ehrlichiosis is the indirect fluorescent antibody (IFA) test. This test uses the etiologic agent *Ehrlichia canis* to diagnose canine ehrlichiosis. The IFA test uses *Ehrlichia chafeensis* as antigen for diagnosing human ehrlichiosis. The IFA test has, however, serious limitations. The IFA test is subject to false positives because the antigens are made of whole infected cells which comprise many nonspecific proteins which will cross-react with sera from some patients. The IFA test is also subject to false negatives because IFA antigens are unstable and may become inactivated during storage. In addition the IFA test requires a special equipment to perform the test. For example, the IFA test requires a tissue culture system for growing the bacterium that are used to prepare the antigen slides, a fluorescent microscope, and trained persons to evaluate the serum reactivity to the bacterial antigen on the slide.

Tools which permit simpler, more rapid, and objective serodiagnosis of canine ehrlichiosis or human ehrlichiosis are desirable.

### SUMMARY OF THE INVENTION

The present invention relates to improved diagnostic tools for veterinary and human use which are used for serodiagnosing ehrlichiosis in mammals, particularly in members of the Canidae family and in humans.

The present invention also provides polynucleotides or nucleic acids which encode the outer membrane proteins of E. chafeensis. The OMP-1 polynucleotide encodes an OMP-1 protein of E. chafeensis having a molecular weight of about 27.7 kDa and an amino acid sequence which is at least 85% homologous to the amino acid sequence shown in FIG.3B, SEQ ID NO: _. The OMP-1B polynucleotide encodes an OMP-1B protein of E. chafeensis having a molecular weight of about 28.2 kDa and an amino acid sequence which is at least 85% homologous to the amino acid sequence shown in FIG. 4B, SEQ ID NO: _. The OMP-IC polynucleotide encodes an OMP-1C protein of E. chafeensis having a molecular weight of about 27.6 kDa and an amino acid sequence which is at least 85% homologous to the amino acid sequence shown in FIG. 5B, SEQ ID NO: _. The OMP-1D polynucleotide encodes an OMP-1D protein of E. chafeensis having a molecular weight of about 28.7 kDa and an amino acid sequence which is at least 85% homologous to the amino acid sequence shown in FIG. 6B, SEQ ID NO: _. The OMP-1E polynucleotide encodes an OMP-1E protein of E. chafeensis having a molecular weight of about 27.8 kDa and an amino acid sequence which is at least 85% homologous to the amino acid sequence shown in FIG. 7B, SEQ ID NO: _. The OMP-1F polynucleotide encodes an OMP-1F protein of E. chafeensis having a molecular weight of about 27.9 kDa and an amino acid sequence which is at least 85% homologous to the amino acid sequence shown in FIG. 8B, SEQ ID NO: _. The OMP-1A polynucleotide encodes an OMP-1A protein of E. chafeensis having a molecular weight of about 29.6 kDa and an amino acid sequence which is at least 85% homologous to the amino acid sequence shown in FIG. 9B, SEQ ID NO: _. The OMP-1R polynucleotide encodes an OMP-1R protein of E. chafeensis having a molecular weight of at least 23 kDa and comprising an amino acid sequence which is at least 85% homologous to the amino acid sequence shown in FIG. 10B, SEQ ID NO: _. The OMP-1S S polynucleotide encodes an OMP-1S protein of E. chafeensis comprising an amino acid sequence which is at least 85% homologous to the amino acid sequence shown in FIG. 11B, SEQ ID NO: _. The OMP-1T polynucleotide encodes an OMP-1T protein of E. chafeensis comprising an amino acid sequence which is at least 85% homologous to the amino acid sequence shown in FIG. 12B, SEQ ID NO: _. The OMP-1U polynucleotide encodes an OMP-1U protein of E. chafeensis having a molecular weight of about 30.6 kDa and an amino acid sequence which is at least 85% homologous to amino acid sequence shown in FIG. 13B, SEQ ID NO: _. The OMP-1V polynucleotide encodes an OMP-1V protein of E. chafeensis having a molecular weight of about 28.0 kDa and an amino acid sequence which is at least 85% homologous to the amino acid sequence shown in FIG. 14B, SEQ ID NO: _. The OMP-1W polynucleotide encodes an OMP-1W protein of E. chafeensis having a molecular weight of about 28.8 kDa and an amino acid sequence which is at least 85% homologous to the amino acid sequence shown in FIG. 15B, SEQ ID NO: _. The OMP-1X polynucleotide encodes an OMP-1S protein of E. chafeensis having a molecular weight of about 27.8 kDa and an amino acid sequence which is at least 85% homologous to the amino acid sequence shown in FIG. 16B, SEQ ID NO: _. The OMP-1Y polynucleotide encodes an OMP-1Y protein of E. chafeensis having a molecular weight about 28.8 kDa and an amino acid sequence which is at least 85% homologous to the amino acid sequence shown in FIG. 17B, SEQ ID NO: _. The OMP-1Z polynucleotide encodes an OMP-1Z protein of E. chafeensis comprising an amino acid sequence which is at least 85% homologous to the amino acid sequence shown in FIG. 18B, SEQ ID NO: _.

The outer membrane proteins from E. chaffeensis, particularly a recombinant form of OMP-1, are immunogenic and, thus are useful for preparing antibodies. Such antibodies are useful for immunolabeling isolates of E. chafeensis and for detecting the presence of E. chafeensis in body fluids, tissues, and particularly in monocytes and macrophages. The isolated outer membrane proteins, particularly OMP-1, are also useful for detecting antibodies to E. chafeensis in the blood of patients with clinical signs of ehrlichiosis. The isolated outer membrane protein, particularly OMP-1, are also useful immunogens for raising antibodies that are capable of reducing the level of infection in an immunized mammal that has been infected with E. chafeensis. The isolated membrane proteins are also useful in a vaccine for protecting against infection with E. chafeensis.

The present invention also relates to isolated polynucleotides which encode 30 kDa outer membrane proteins from Ehrlichia canis. The proteins are designated P30 and P30a. The proteins, particularly P30, are immunogenic and are, thus, useful for preparing antibodies that are useful for immunolabeling isolates of E. canis. The P30 protein is also useful for diagnosing canine ehrlichiosis in mammals, particularly in members of the family Canidae, most particularly in dogs and for diagnosing infections with E. chafeensis in humans. The P30 protein is also a useful immunogen for raising antibodies that reduce the level of infection in an immunized mammal that has been infected with E. canis. The P30 protein is also useful in a vaccine for protecting animals against infection with E. canis.

The present invention also provides the following isolated proteins of E. chafeensis OMP-1 (also known as p28), OMP-1A, OMP-1B, OMP-1C, OMP-1D, OMP-1E, OMP-1F, OMP-1R, OMP-1S, OMP-IT, OMP -1U, OMP-1V, OMP-1W, OMP-1X, and OMP-1Z, referred to hereinafter collectively as the "OMP family". The present invention also provides the following isolated proteins of E. canis P30, P30-a, P30-1, P30-2, P30-3, P30-4, P30-5, P30-6, P30-7, P30-8, P30-9, and P30-10, referred to hereinafter as the P30 family.

The present invention also relates to an assay for diagnosing ehrlichiosis in humans using a recombinant outer membrane protein of E. chafeensis, particularly OMP-1. The present invention also relates to an assay for diagnosing ehrlichiosis in humans and members of the family Canidae using a recombinant outer membrane protein ofE. canis, particularly P30.

### Brief Description of the Figures

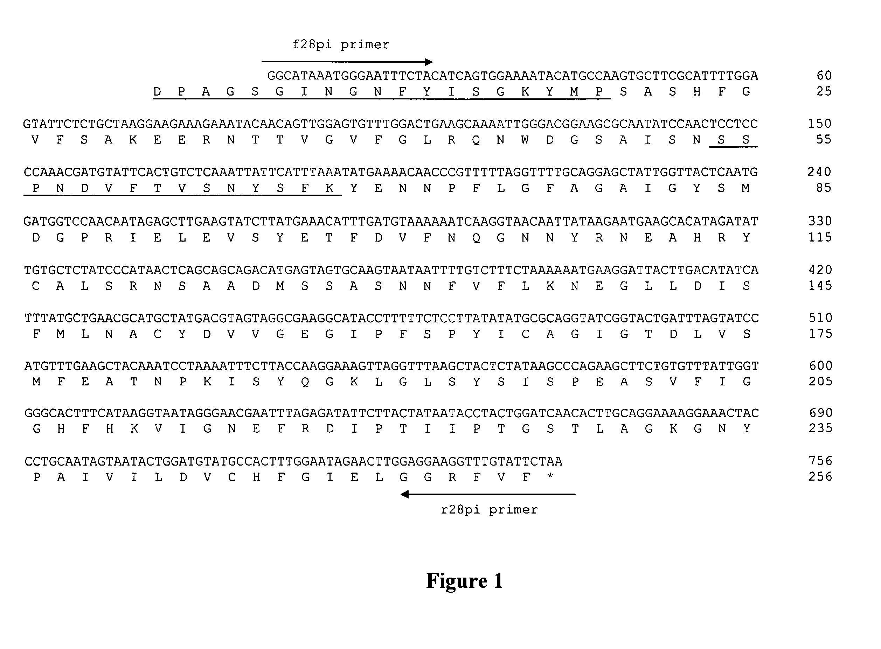

FIG. 1. shows the DNA sequence of and the amino acid sequence encoded by the *E. chafeensis (p28)* gene cloned in pCRII*p*28. The N-terminal amino acid sequence of native *omp-1* protein (P28) determined chemically is underlined. Five amino acid residues at the N terminus of P28 which were not included in the *p28* gene, are indicated by boldface. Arrows indicate annealing positions of the primer pair designed for PCR

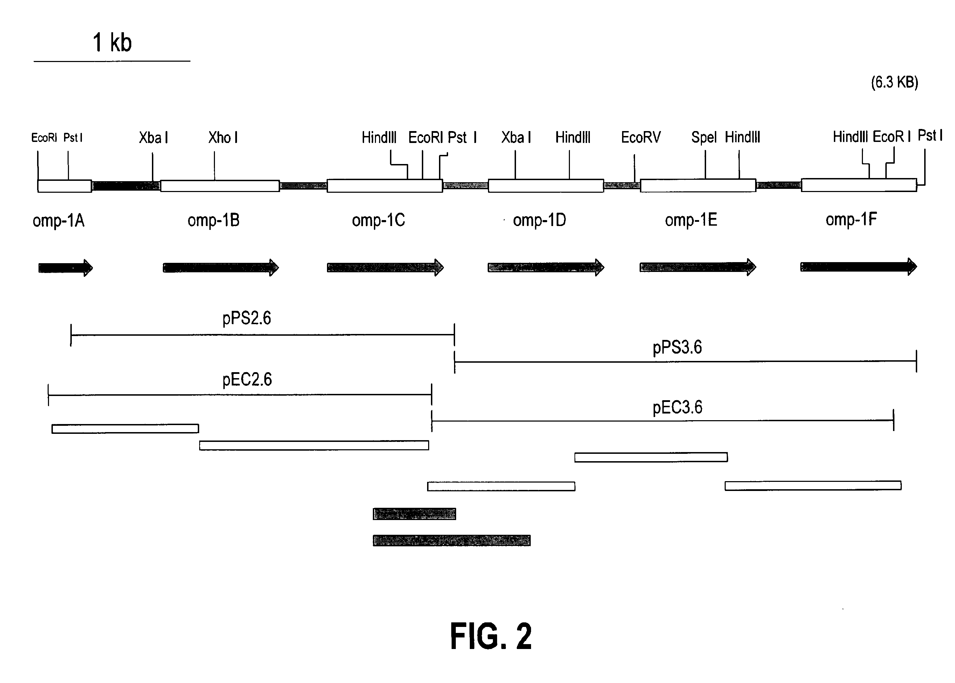

FIG. 2. shows the restriction map of 6.3-kb genomic DNA including the *omp-1* gene copies in E. *chafeensis.* The four DNA fragments were cloned from the genomic DNA (pPS2.6, pPS3.6, pEC2.6, and pEC3.6). A recombinant plasmid pPS2.6 has an overlapping sequence with that of pEC3.6. The closed boxes at the bottom show PCR-amplified fragments from the genomic DNA for confirmation of the overlapping area. Open boxes at the top indicate open reading frames (ORF) of *omp-1* gene copies with direction by arrows. Open boxes at the bottom show DNA fragments subcloned for DNA sequencing.

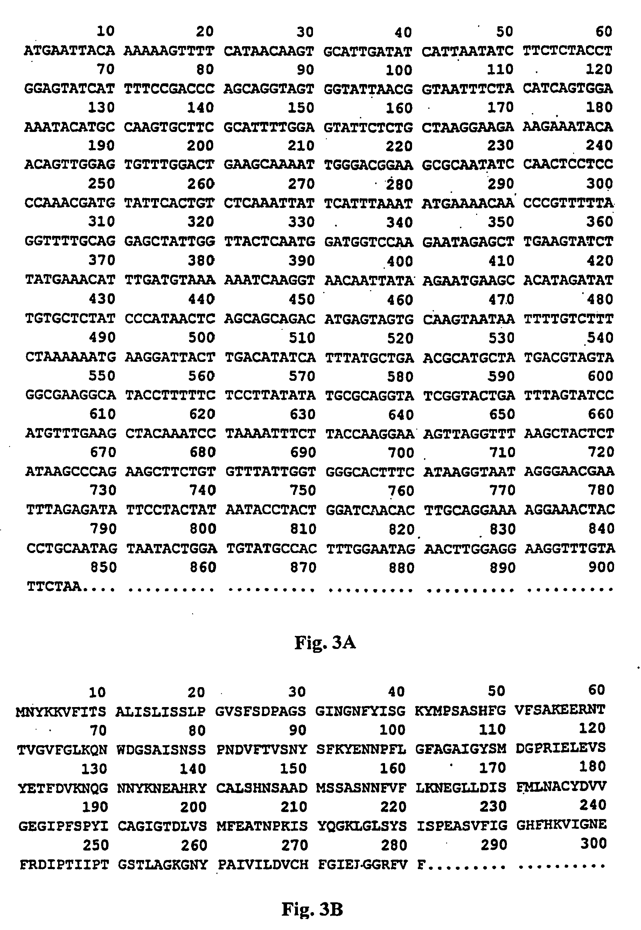

FIG. 3B shows one embodiment of the OMP-1 protein; FIG. 3A shows one embodiment of the OMP-1 polynucleotide.

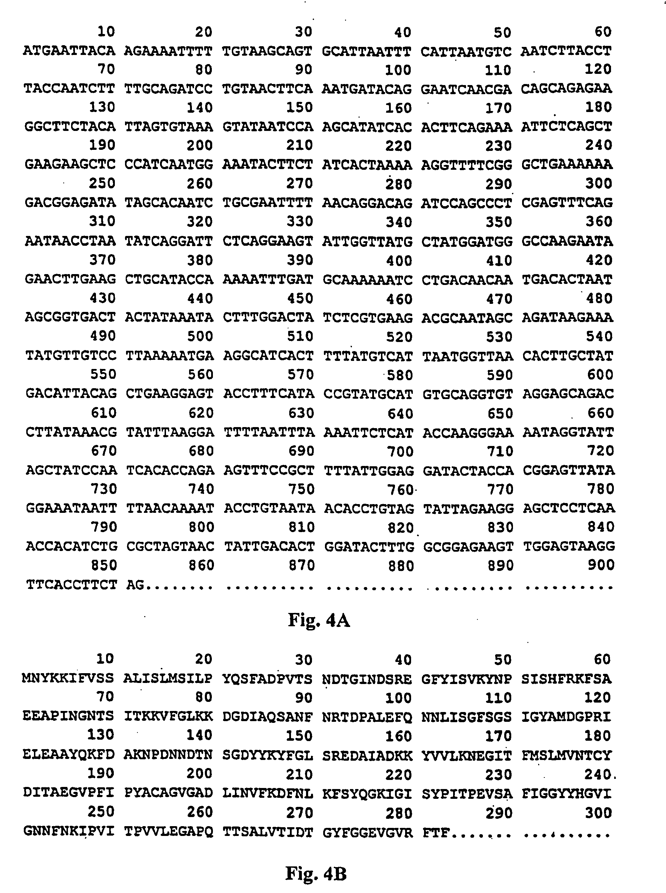

FIG. 4B shows one embodiment of the OMP-1B protein, FIG. 4A shows one embodiment of the OMP-1B polynucleotide

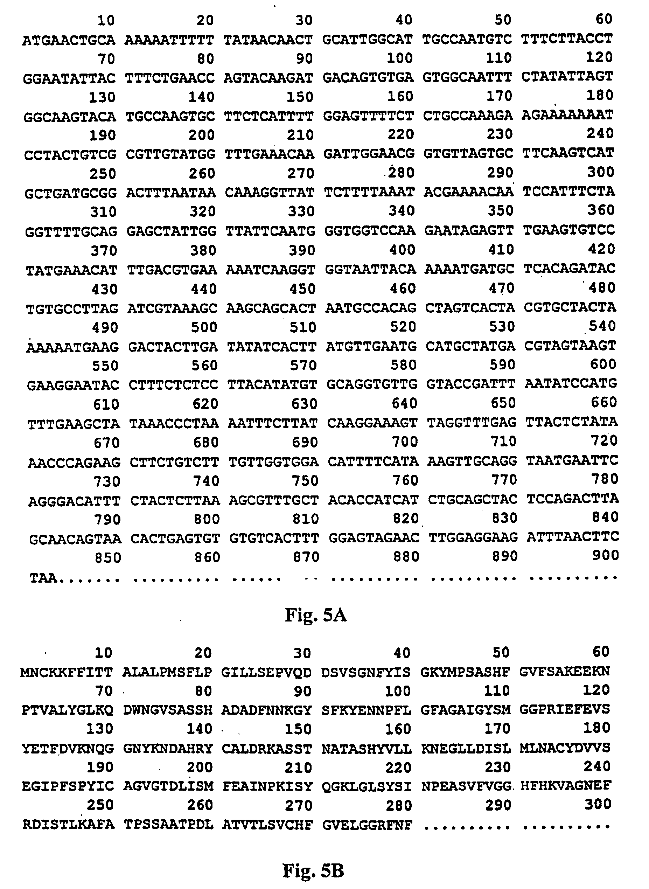

FIG. 5A shows one embodiment of the OMP-1C polynucleotide; FIG 5B shows one embodiment of the OMP-1C protein.

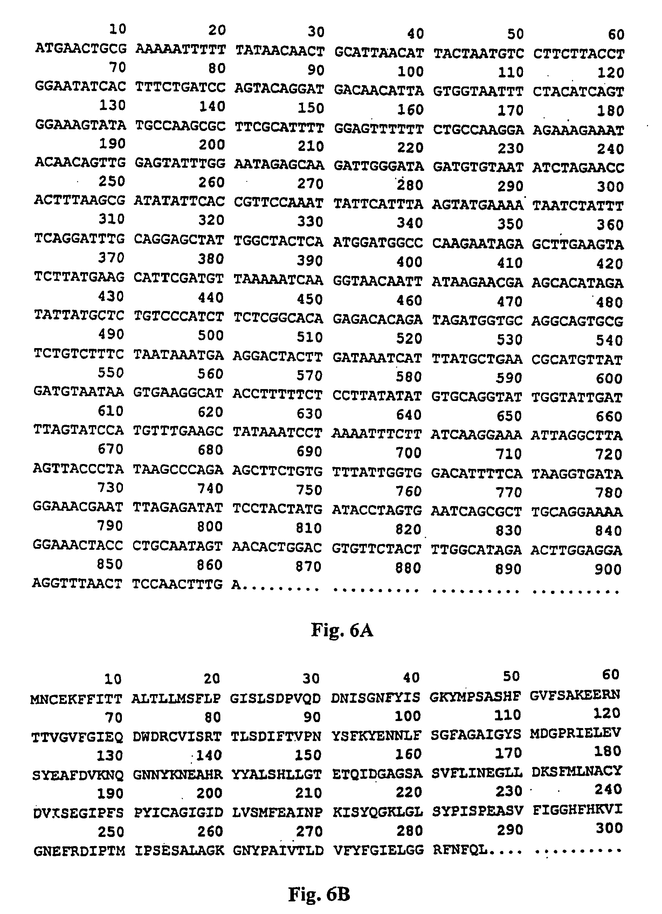

FIG. 6B shows one embodiment of the OMP-1D protein; FIG. 6A shows one embodiment of the OMP-1D polynucleotide.

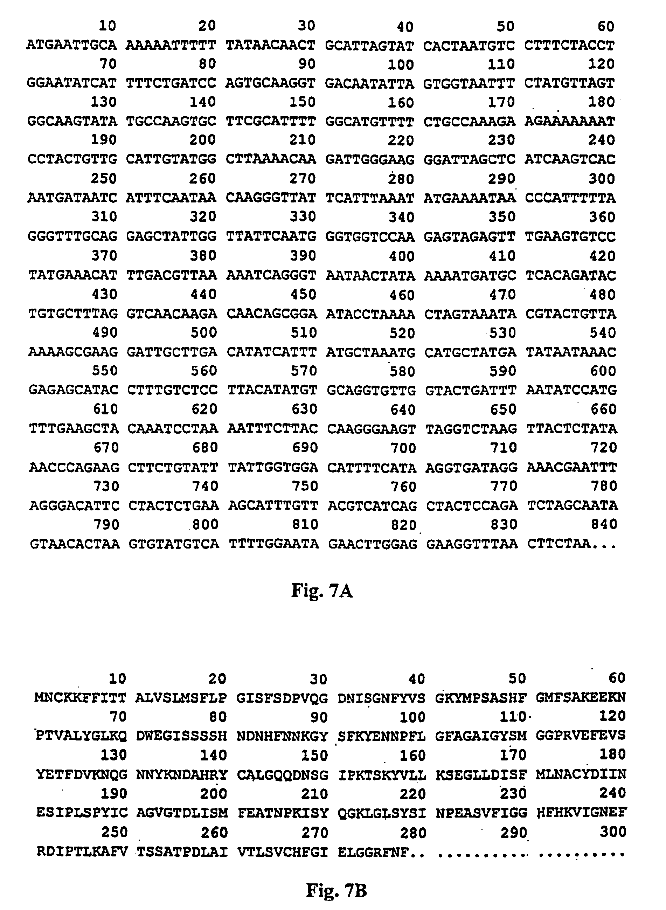

FIG. 7A shows one embodiment of the OMP-1E protein; FIG 7B shows one embodiment of the OMP-1E polynucleotide.

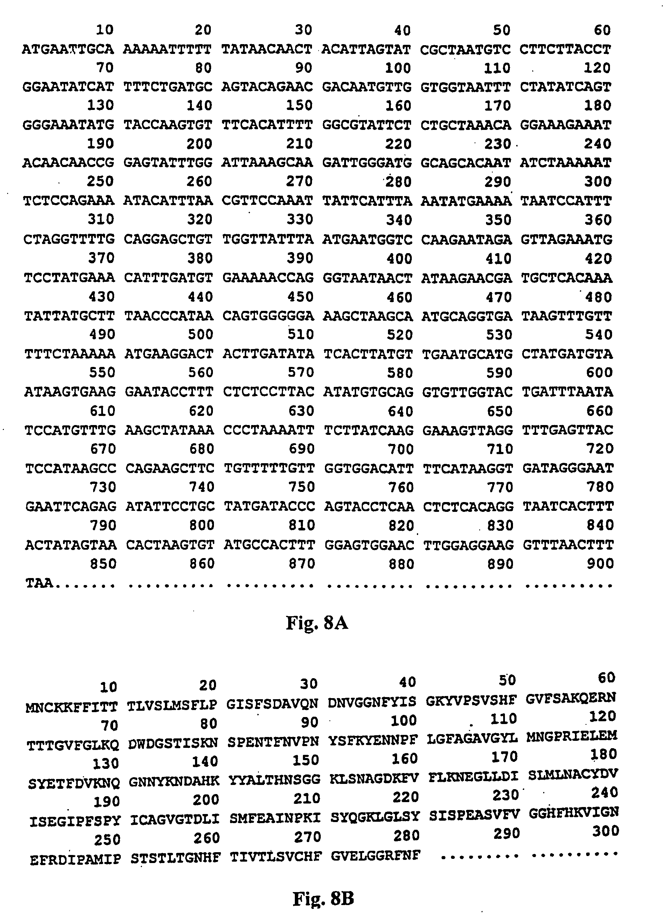

FIG. 8A shows one embodiment of the OMP-1F protein; FIG 8B shows one embodiment of the OMP-1F polynucleotide.

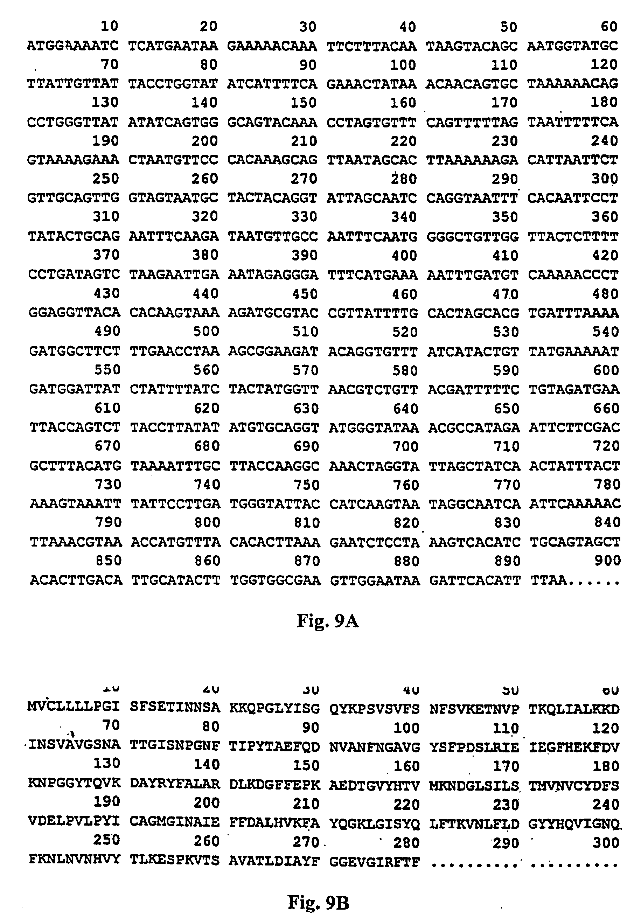

FIG. 9B shows one embodiment of the OMP-1A protein, FIG 9A shows one embodiment of the OMP-1A polynucleotide.

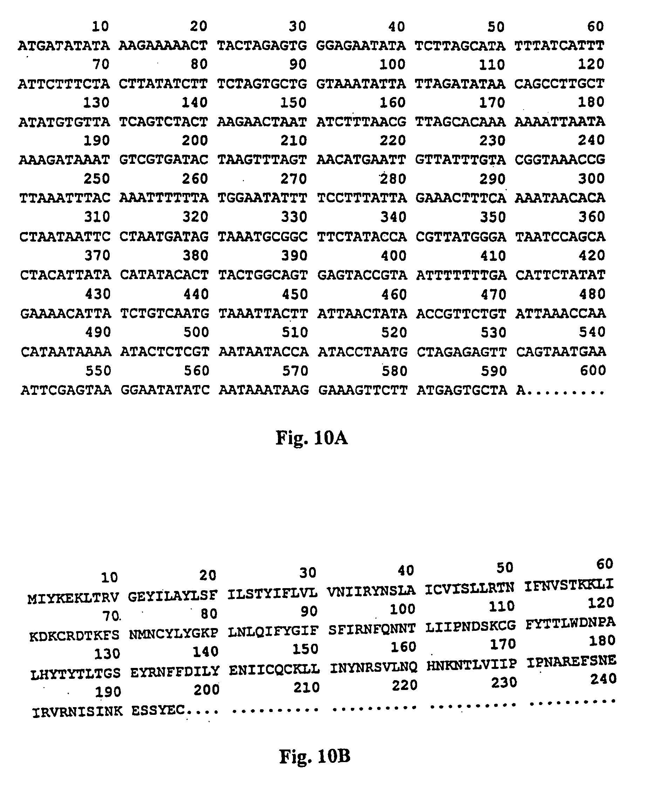

FIG. 10B shows one embodiment of a portion of the OMP-1R protein, FIG 10A shows one embodiment of an OMP-1R polynucleotide encoding such polypeptide.

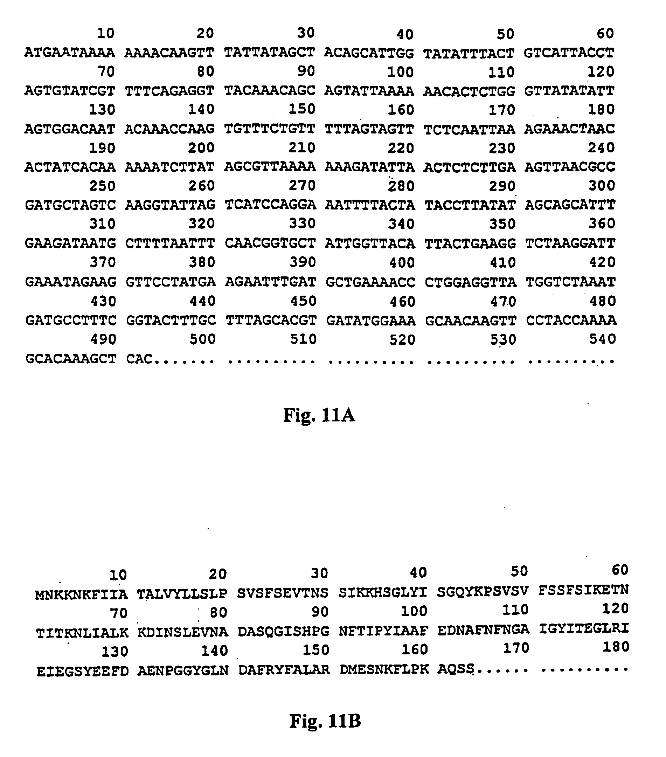

FIG. 11B shows one embodiment of a portion of the OMP-1S protein, FIG 11A shows one embodiment of the OMP-1S polynucleotide encoding such polypeptide.

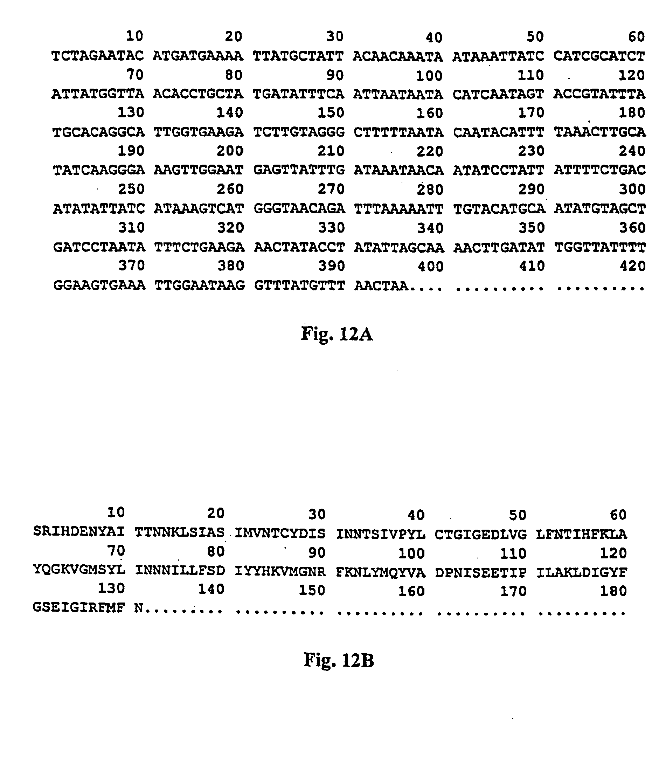

FIG. 12B shows one embodiment of a portion of the OMP-1T protein, FIG 12A shows one embodiment of the OMP-1T polynucleotide encoding such polypeptide.

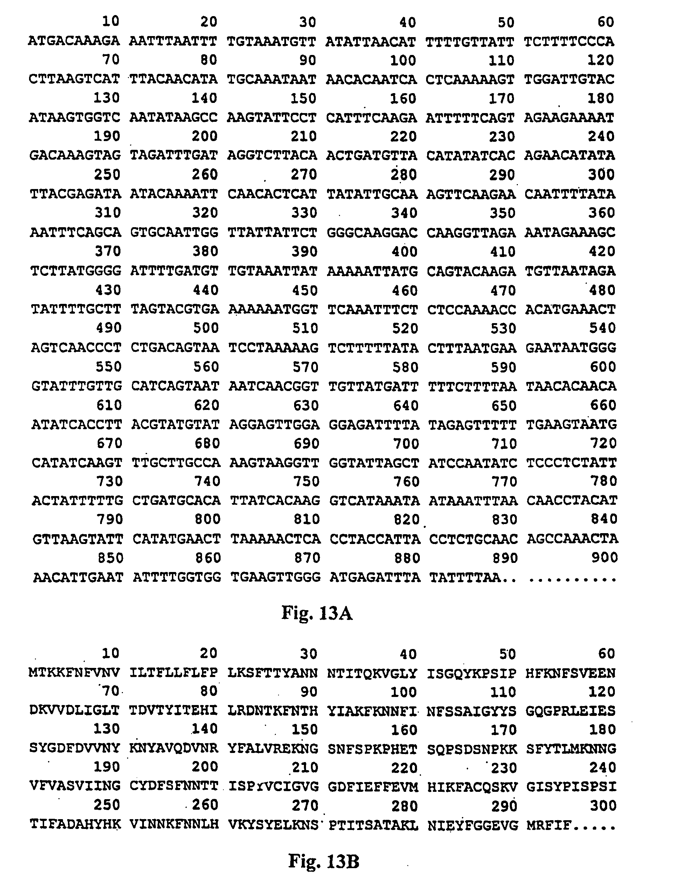

FIG. 13B shows one embodiment of the OMP-1U protein, FIG 13A shows one embodiment of the OMP-1U polynucleotide.

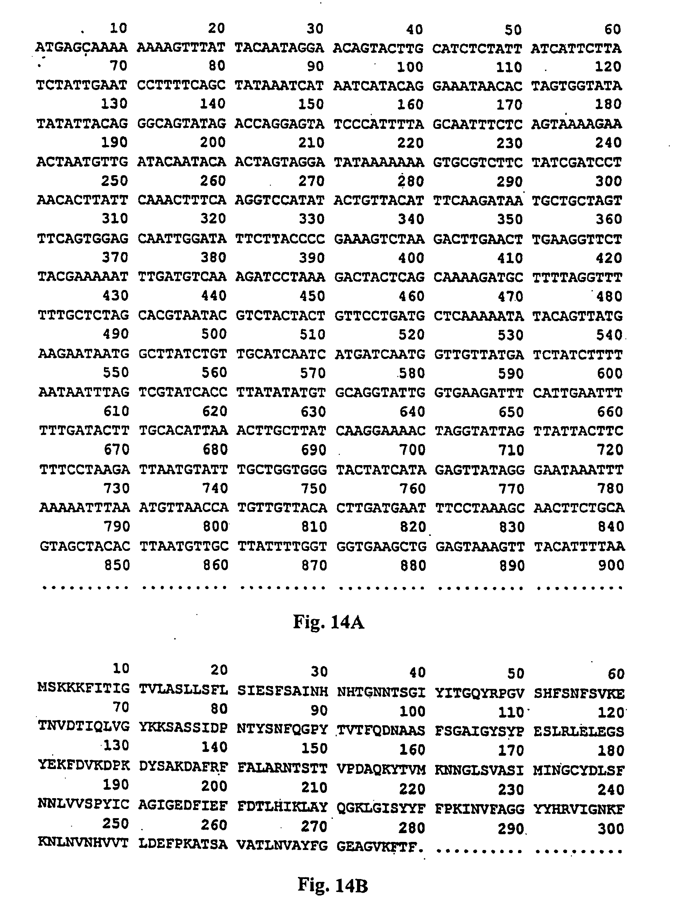

FIG. 14B shows one embodiment of the OMP-1V protein, FIG 14A shows one embodiment of the OMP-1V polynucleotide.

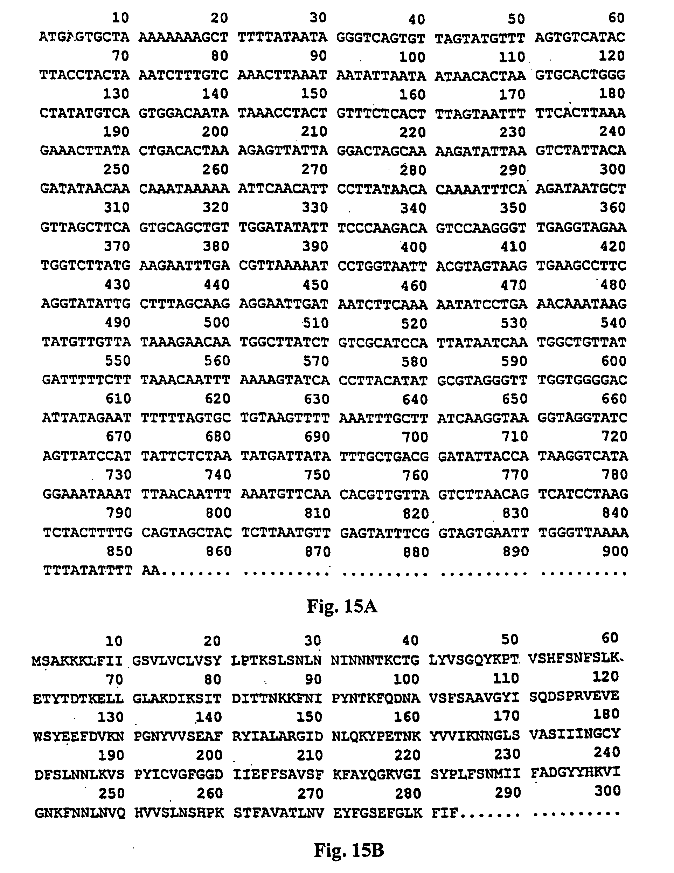

FIG. 15B shows one embodiment of the OMP-1W protein, FIG 15A shows one embodiment of the OMP-1W polynucleotide.

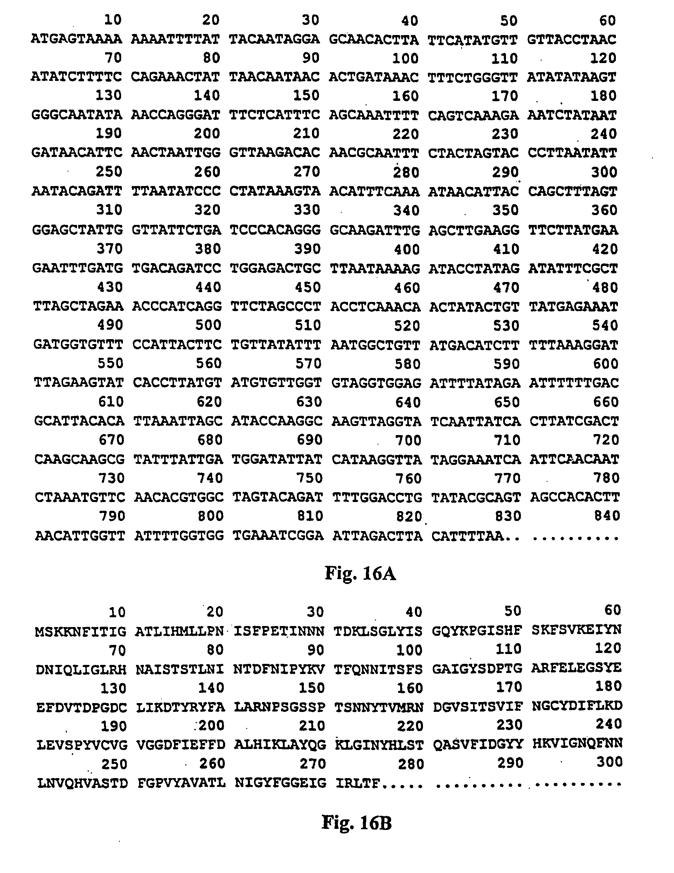

FIG. 16B shows one embodiment of the OMP-1X protein, FIG 16A shows one embodiment of the OMP-1W polynucleotide.

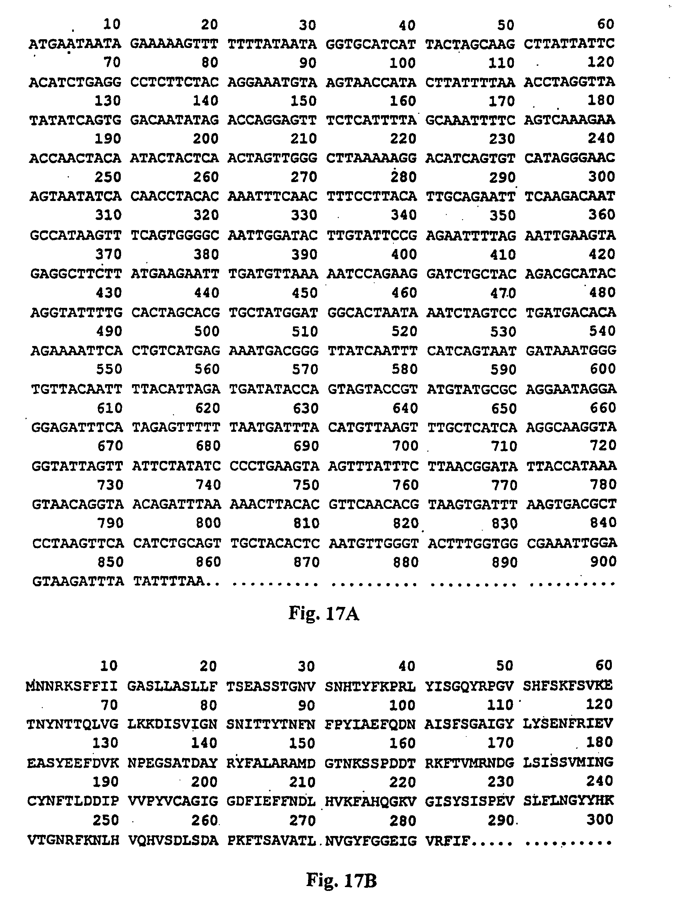

FIG. 17B shows one embodiment of the OMP-1Y protein, FIG 17A shows one embodiment of the OMP-1Y polynucleotide.

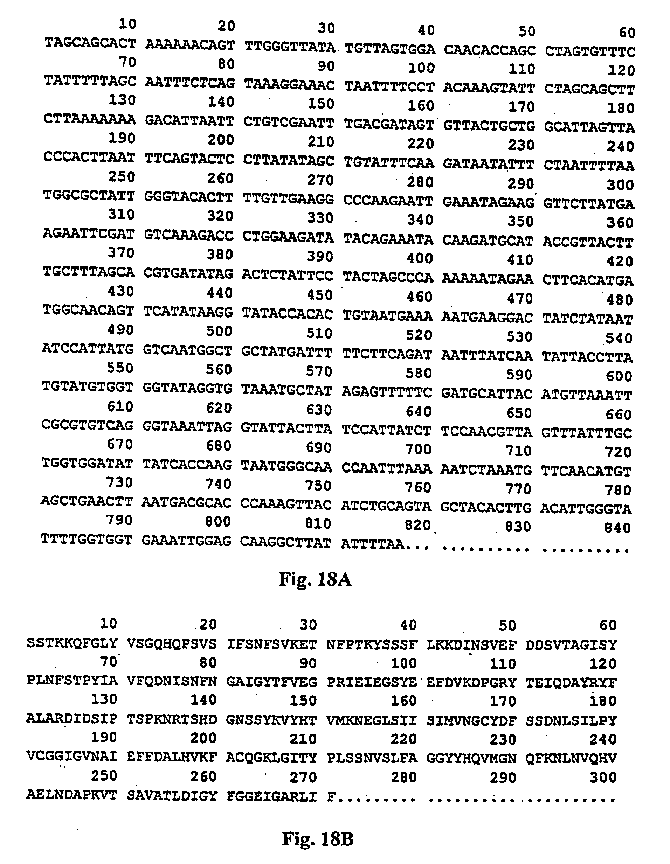

FIG. 18B shows one embodiment of the OMP-1Z protein, FIG 18A shows one embodiment of the OMP-1Z polynucleotide.

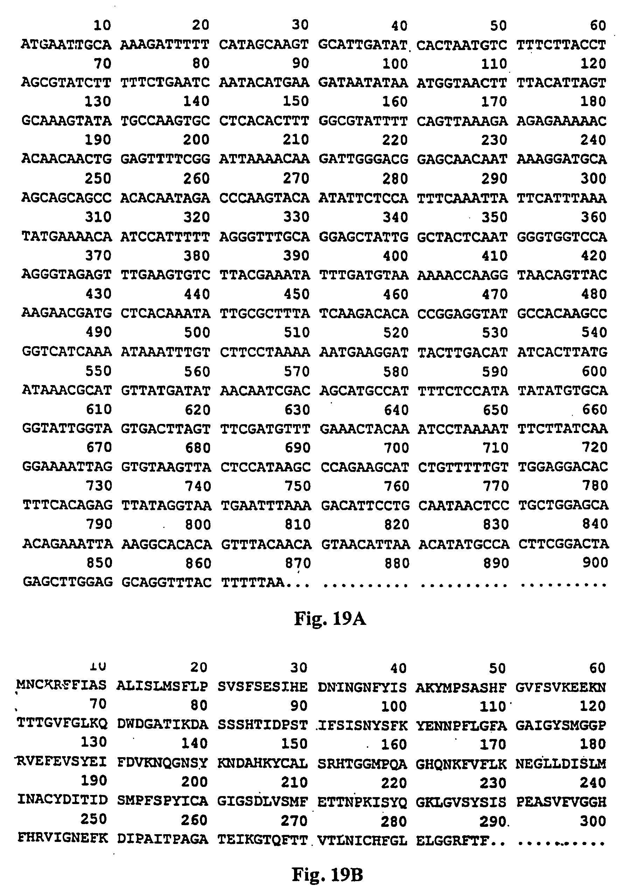

FIG. 19B shows one embodiment of the P30 protein, FIG 19A shows one embodiment of the P30 polynucleotide.

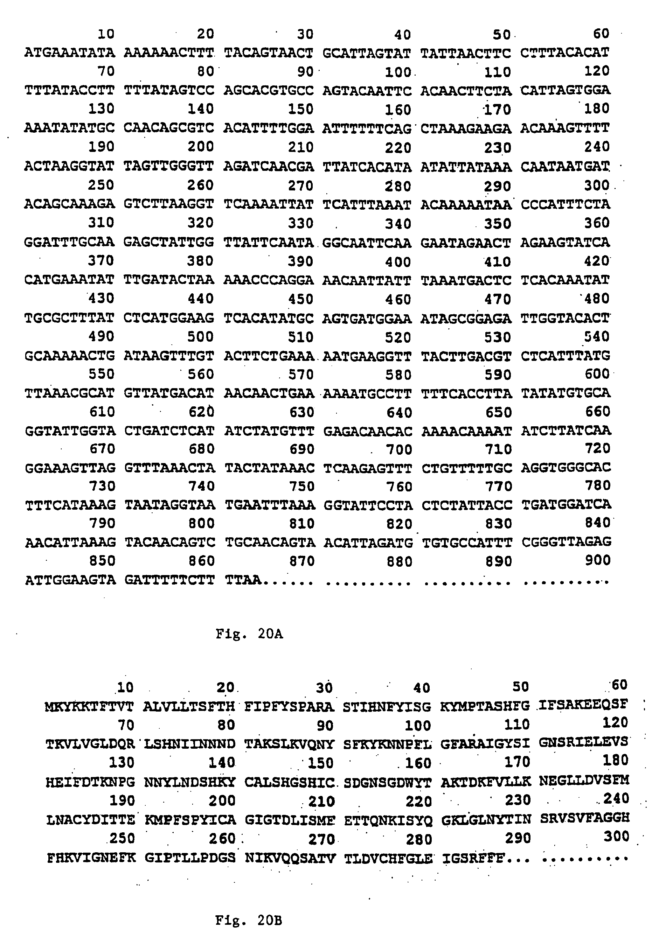

FIG. 20B shows one embodiment of the P30a protein, FIG 20A shows one embodiment of the p30A polynucleotide.

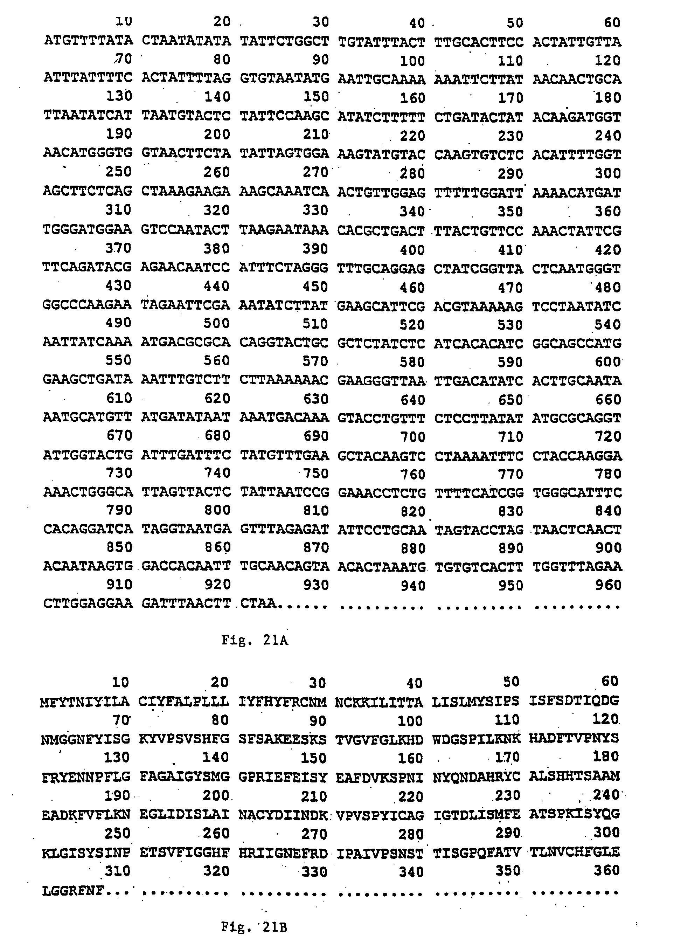

FIG. 21B shows one embodiment of the P30-1 protein, FIG 21A shows one embodiment of the p30-1 polynucleotide.

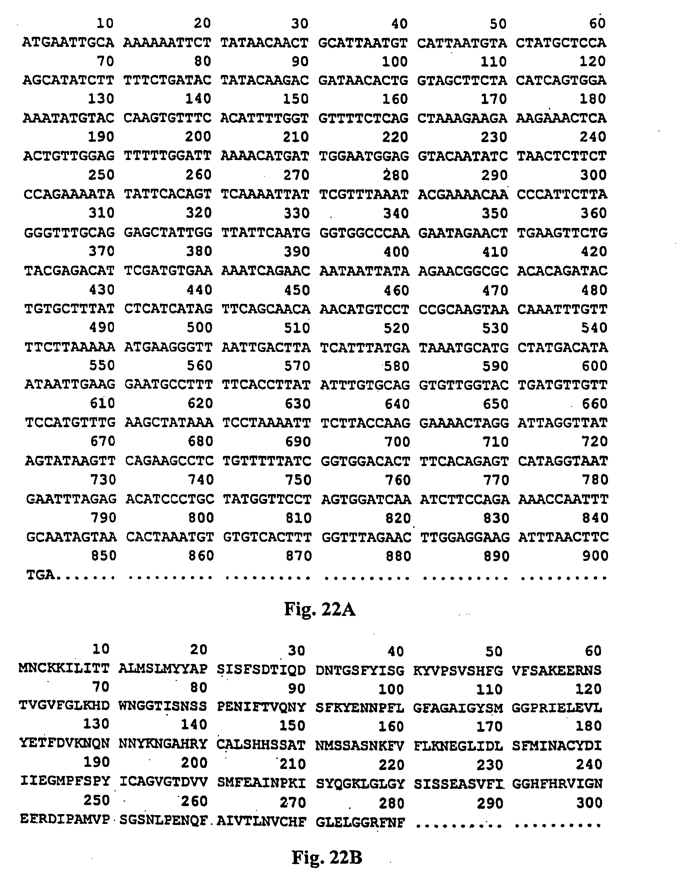

FIG. 22B shows one embodiment of the P30-2 protein, FIG 22A shows one embodiment of the p30-2 polynucleotide.

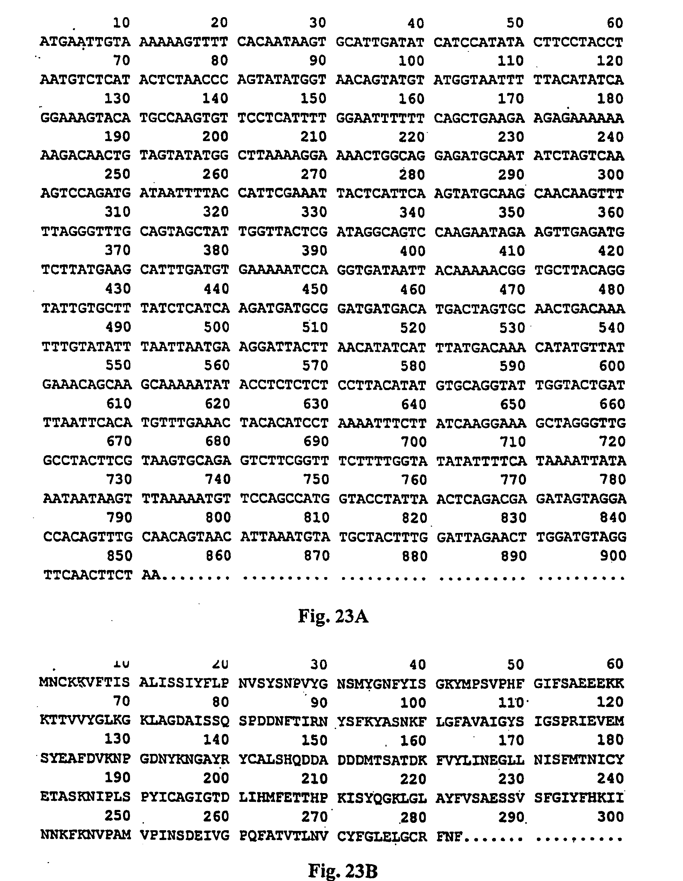

FIG. 23B shows one embodiment of the P30-3 protein, FIG 23A shows one embodiment of the p30-3 polynucleotide.

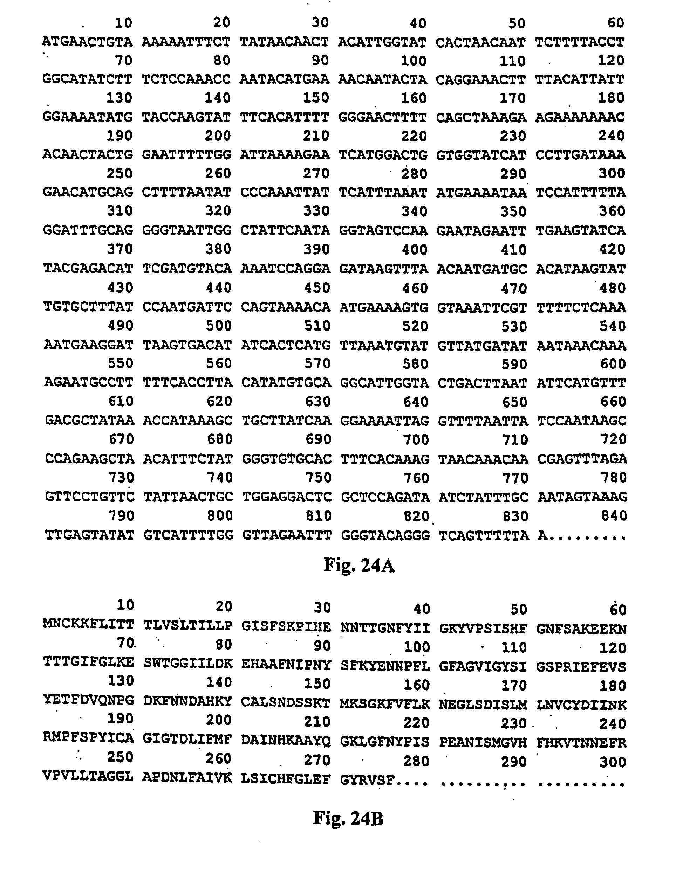

FIG. 24B shows one embodiment of the P30-4 protein, FIG 22A shows one embodiment of the p30-4 polynucleotide.

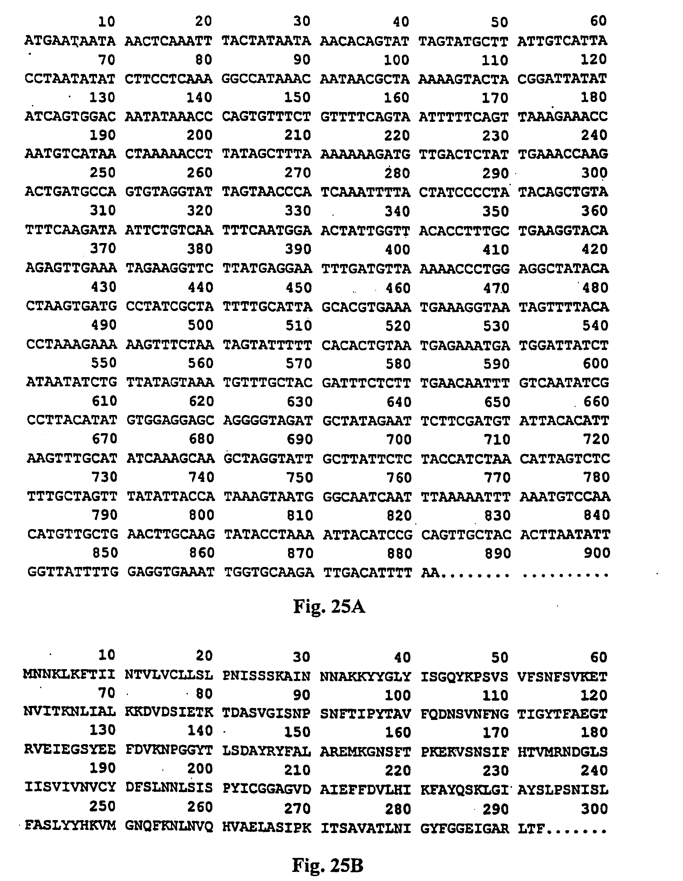

FIG. 25B shows one embodiment of the P30-5 protein, FIG 22A shows one embodiment of the p30-5 polynucleotide.

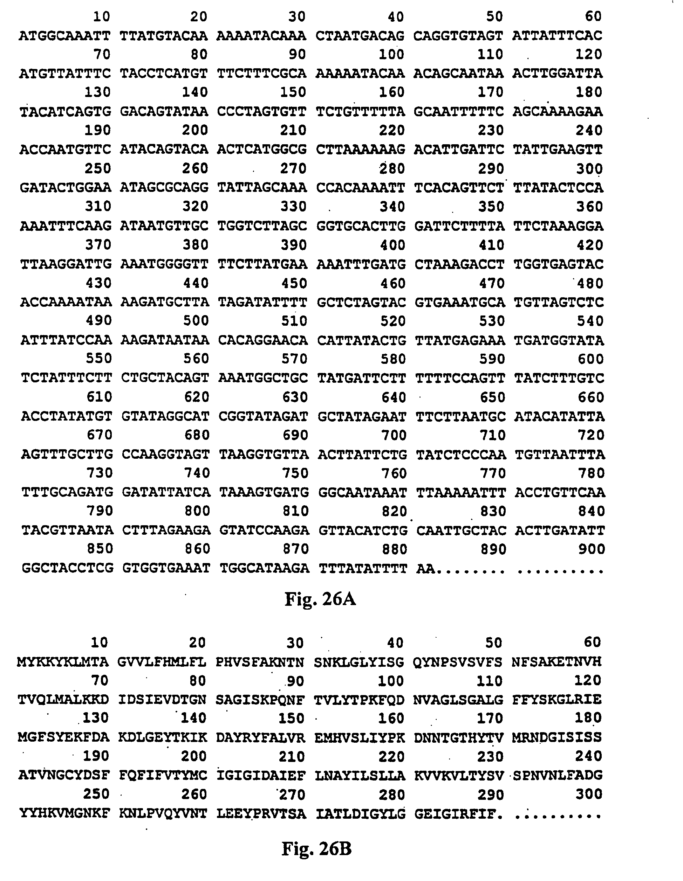

FIG. 26B shows one embodiment of the P30-6 protein, FIG 26A shows one embodiment of the p30-6 polynucleotide.

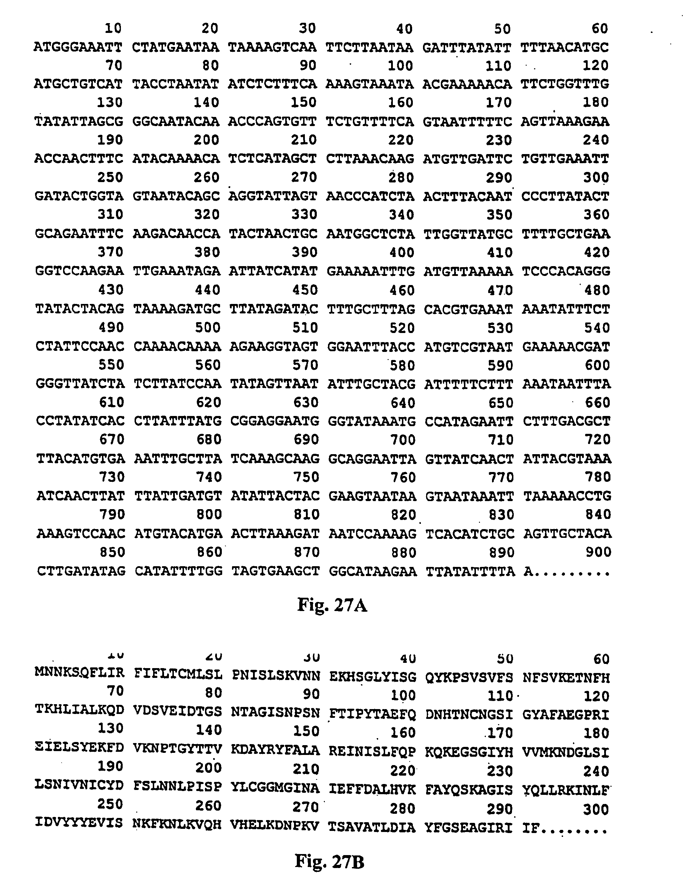

FIG. 27B shows one embodiment of the P30-7 protein, FIG 27A shows one embodiment of the p30-7 polynucleotide.

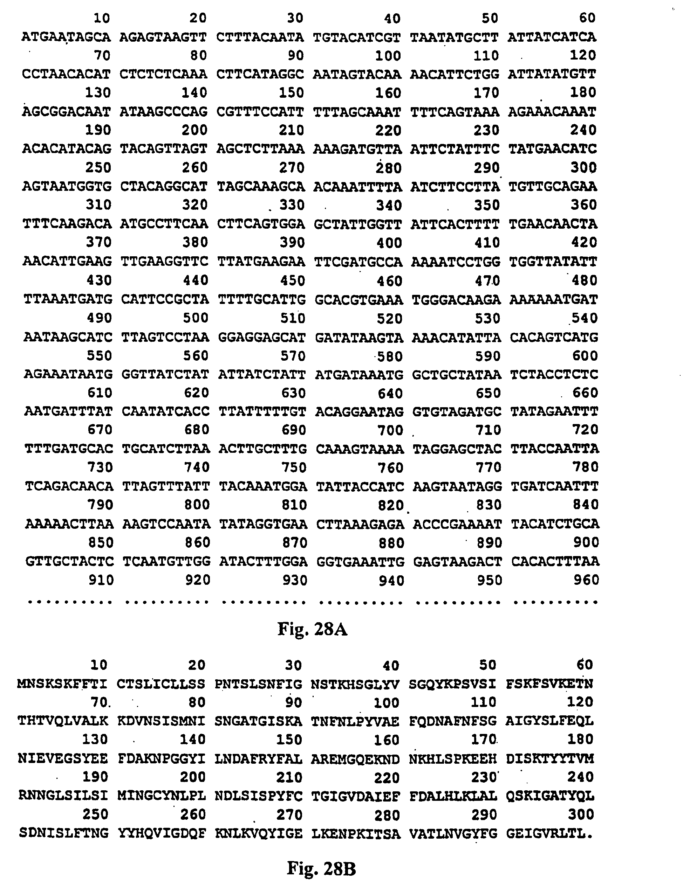

FIG. 28B shows one embodiment of the P30-8 protein, FIG 28A shows one embodiment of the p30-8 polynucleotide.

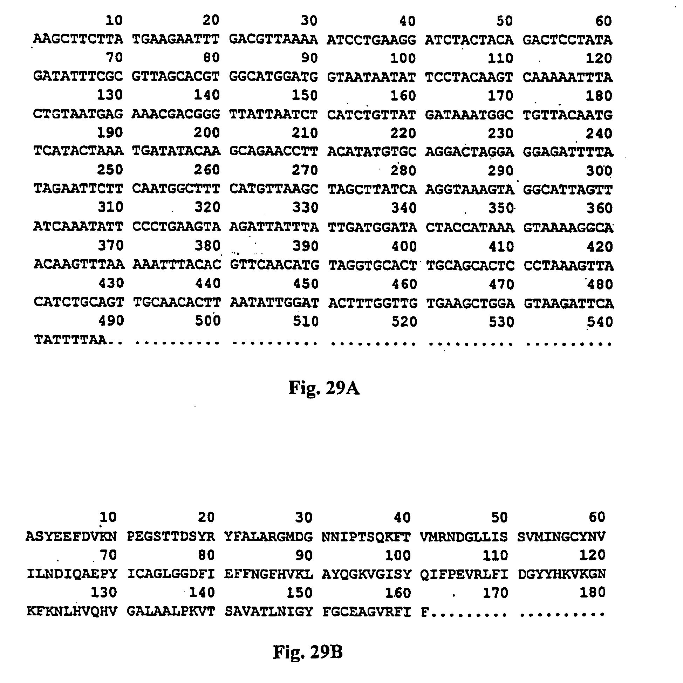

FIG. 29B shows one embodiment of a portion of the P30-9 protein, FIG 29A shows one embodiment of the p30-9 polynucleotide encoding such polypeptide.

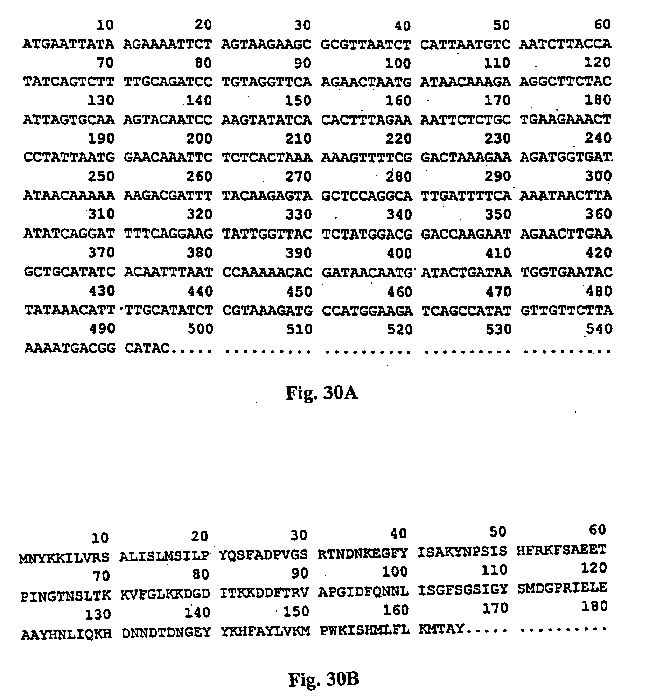

FIG. 30B shows one embodiment of a portion of the P30-10 protein, FIG 30A shows one embodiment of the p30-10 polynucleotide encoding such polypeptide.

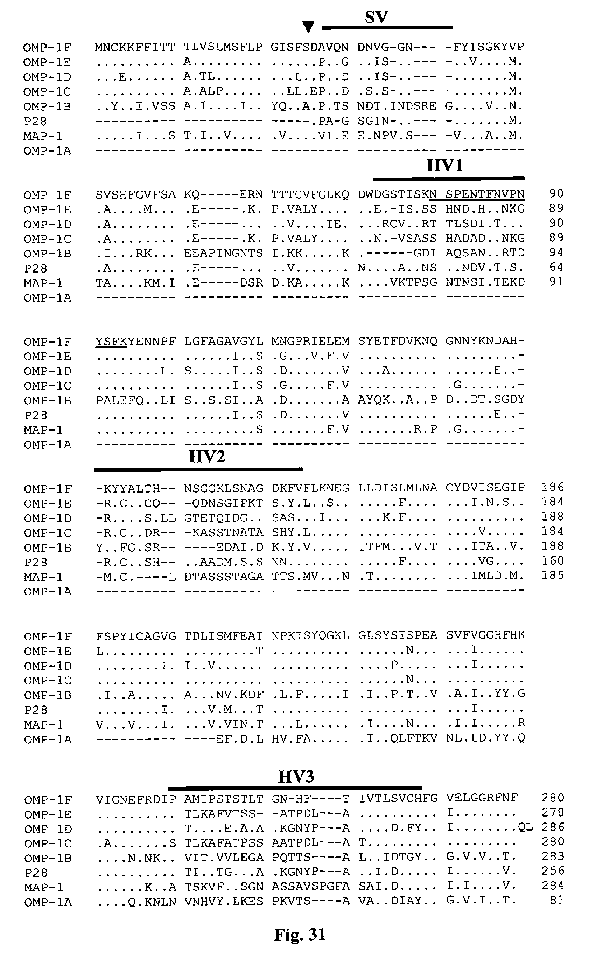

FIG. 31 depicts the amino acid sequences alignment of seven *E. chafeensis* OMP-1s and *Cowdria ruminantium* MAP-1. Aligned positions of identical amino acids with OMP-IF are shown with dots. The sequence of *C. ruminantium* MAP-1 is from the report of Van Vliet et al (1994) Molecular cloning, sequence analysis, and expression of the gene encoding the immunodominant 32-kilodalton protein of *Cowdria ruminantium.* Infect. Immun. 62:1451-1456. Gaps indicated by dashes were introduced for optimal alignment of all proteins. Bars indicates semivariable region (SV) and three hypervariable regions (HV1, HV2, and HV3).

### DETAILED DESCRIPTION OF THE INVENTION

### Isolated Polynucleotides Encoding OMP-1,OMP-1A, OMP-1B, OMP-1C, OMP-1D, OMP-1F and the OMP from E. Canis

In one aspect, the present invention, provides isolated polynucleotides that encode the outer membrane proteins, OMP-1 (or p28), OMP-1B, OMP-1C, OMP-1D, OMP- 1E, OMP-1F, OMP-1A, OMP-1R, OMP-1S, OMP-IT, OMP -1U, OMP-1V, OMP-1W, OMP-1X, OMP-1Y and OMP-1Z from E. chafeensis and the outer membrane proteins P30, P30-a, P-30-1, P30-2, P30-3, P30-4, P30-5, P30-6, P30-7, P30-8, P30-9, and P30-10 from E. Canis or an immunogenic fragment thereof.

The polynucleotide is single stranded or double stranded. The polynucleotide may be a DNA or RNA molecule, preferably a DNA molecule, and comprises a sequence which codes for the respective outer membrane protein. Preferably, the polynucleotide encodes at least the mature form of outer membrane protein. The polynucleotide optionally further comprises a leader sequence and encode an outer membrane preprotein that is processed in the cell to form the mature protein. The polynucleotide of the present invention may also be fused in frame to a marker sequence which allows for purification of the corresponding outer membrane protein.

The OMP-1 polynucleotide encodes an OMP-1 protein of E. chafeensis having a molecular weight of about 27.7 kDa and an amino acid sequence which is at least 85% homologous to the amino acid sequence shown in FIG. 3B SEQ ID NO: _; Figure 3B shows one embodiment of the OMP-1 protein, Figure 3A shows one embodiment of the OMP-1 polynucleotide. The OMP-1B polynucleotide encodes an OMP-1B protein of E. chafeensis having a molecular weight of about 28.2 kDa and an amino acid sequence which is at least 85% homologous to the amino acid sequence shown in FIG. 4B SEQ ID NO: _; Figure 4B shows one embodiment of the OMP-1B protein, Figure 4A shows one embodiment of the OMP-1B polynucleotide. The OMP-1C polynucleotide encodes an OMP-1C protein of E. chafeensis having a molecular weight of about 27.6 kDa and an amino acid sequence which is at least 85% homologous to the amino acid sequence shown in FIG. 5B SEQ ID NO: _; Figure 5B shows one embodiment of the OMP-1C protein, Figure 5A shows one embodiment of the OMP-1C polynucleotide. The OMP-1D polynucleotide encodes an OMP-1D protein of E. chafeensis having a molecular weight of about 28.7 kDa and an amino acid sequence which is at least 85% homologous to the amino acid sequence shown in FIG. 6B SEQ ID NO: _; Figure 6B shows one embodiment of the OMP-1D protein, Figure 6A shows one embodiment of the OMP-1D polynucleotide. The OMP-1E polynucleotide encodes an OMP-1E protein of E. chafeensis having a molecular weight of about 27.8 kDa and an amino acid sequence which is at least 85% homologous to the amino acid sequence shown in FIG. 7B SEQ ID NO: _; Figure 7B shows one embodiment of the OMP-1E protein, Figure 7A shows one embodiment of the OMP-1E polynucleotide. The OMP-1F polynucleotide encodes an OMP-1F protein of E. chafeensis having a molecular weight of about 27.9 kDa and an amino acid sequence which is at least 85% homologous to the amino acid sequence shown in FIG. 8B SEQ ID NO: _; Figure 8B shows one embodiment of the OMP-1F protein, Figure 8A shows one embodiment of the OMP-1F polynucleotide. The OMP-1A polynucleotide encodes an OMP-1A protein of E. chafeensis having a molecular weight of about 29.6 kDa and an amino acid sequence which is at least 85% homologous to the amino acid sequence shown in FIG. 9B SEQ ID NO: _; Figure 9B shows one embodiment of the OMP-1A protein, Figure 9A shows one embodiment of the OMP-1A polynucleotide. The OMP-1R polynucleotide encodes an OMP-1R protein of E. chafeensis having a molecular weight of at least 23 kDa and comprising an amino acid sequence which is at least 85% homologous to the amino acid sequence shown in FIG. 10B SEQ ID NO: _; Figure 10B shows one embodiment of a portion of the OMP-1R protein, Figure10A shows one embodiment of the OMP-1R polynucleotide encoding such polynucleotide. The OMP-1S polynucleotide encodes an OMP-1S protein of E. chafeensis comprising an amino acid sequence which is at least 85% homologous to the amino acid sequence shown in FIG. 11B SEQ ID NO: _; Figure 11B shows one embodiment of a portion of the OMP-1S protein, Figure 11A shows one embodiment of the OMP-1S polynucleotide encoding such polypeptide. The OMP-1T polynucleotide encodes an OMP-1T protein of E. chafeensis comprising an amino acid sequence which is at least 85% homologous to the amino acid sequence shown in FIG.12B SEQ ID NO: _; Figure 12B shows one embodiment of a portion of the OMP-1T protein, Figure 12B shows one embodiment of a polynucleotide encoding such polypeptide. The OMP-1U polynucleotide encodes an OMP-1U protein of E. chafeensis having a molecular weight of about 30.6 kDa and an amino acid sequence which is at least 85% homologous to amino acid sequence shown in FIG. 13B SEQ ID NO: _; Figure 13B shows one embodiment of the OMP-1U protein, Figure 13A shows one embodiment of the OMP-1U polynucleotide. The OMP-1V polynucleotide encodes an OMP-1V protein of E. chafeensis having a molecular weight of about 28.0 kDa and an amino acid sequence which is at least 85% homologous to the amino acid sequence shown in FIG. 14B SEQ ID NO: _; Figure 14B shows one embodiment of the OMP-1V protein, Figure 14A shows one embodiment of the OMP-1V polynucleotide. The OMP-1W polynucleotide encodes an OMP-1W protein of E. chafeensis having a molecular weight of about 28.8 kDa and an amino acid sequence which is at least 85% homologous to the amino acid sequence shown in FIG. 15B SEQ ID NO: _; Figure 15B shows one embodiment of the OMP-1W protein, Figure 15A shows one embodiment of the OMP-1W polynucleotide. The OMP-1X polynucleotide encodes an OMP-1S protein of E. chafeensis having a molecular weight of about 27.8 kDa and an amino acid sequence which is at least 85% homologous to the amino acid sequence shown in FIG. 16B SEQ ID NO: _; Figure 16B shows one embodiment of the OMP-1X protein, Figure 16A shows one embodiment of the OMP-1X polynucleotide. The OMP-1Y polynucleotide encodes an OMP-1Y protein of E. chafeensis having a molecular weight about 28.8 kDa and an amino acid sequence which is at least 85% homologous to the amino acid sequence shown in FIG. 17B SEQ ID NO: _; Figure 17B shows one embodiment of the OMP-1Y protein, Figure 17A shows one embodiment of the OMP-1Y polynucleotide. The OMP-1Z polynucleotide encodes an OMP-1Z protein of E. chafeensis comprising an amino acid sequence which is at least 85% homologous to the amino acid sequence shown in FIG. 18B SEQ ID NO: _; Figure 18B shows one embodiment of a portion of the OMP-1Z protein, Figure 18A shows one embodiment of an OMP-1Z polynucleotide encoding such polypeptide.

The p30 polynucleotide encodes a P30 protein of E. canis having a molecular weight of about 28.8 kDa and an amino acid sequence which is at least 85% homologous to the amino acid sequence shown in FIG. 19B SEQ ID NO: _; Figure 19B shows one embodiment of the P30 protein, Figure 19A shows one embodiment of the p30 polynucleotide. The p30A polynucleotide encodes a P30a protein of E. canis having a molecular weight of about 29.1 kDa and an amino acid sequence which is at least 85% homologous to the amino acid sequence shown in FIG. 20B SEQ ID NO: _; Figure 20B shows one embodiment of the P30a protein, Figure 20A shows one embodiment of the p30A polynucleotide. The p30-1 polynucleotide encodes a P30-1 protein of E. canis having a molecular weight of about 27.7 kDa and an amino acid sequence which is at least 85% homologous to the amino acid sequence shown in FIG. 21B SEQ ID NO: _; Figure 21B shows one embodiment of the P30-1 protein, Figure 21A shows one embodiment of the p30-1 polynucleotide. The p30-2 polynucleotide encodes a P30-2 protein of E. canis having a molecular weight of about 28.0 kDa and an amino acid sequence which is at least 85% homologous to the amino acid sequence shown in FIG. 22B SEQ ID NO: _; Figure 22B shows one embodiment of the P30-2 protein, Figure 22A shows one embodiment of the p30-2 polynucleotide. The p30-3 polynucleotide encodes a P30-3 protein of E. canis having a molecular weight of about 28.7 kDa and an amino acid sequence which is at least 85% homologous to the amino acid sequence shown in FIG. 23B SEQ ID NO: _; Figure 23B shows one embodiment of the P30-3 protein, Figure 23A shows one embodiment of the p30-3 polynucleotide. The p30-4 polynucleotide encodes a P30-4 protein of E. canis having a molecular weight of about 28.0 kDa and an amino acid sequence which is at least 85% homologous to the amino acid sequence shown in FIG. 24B SEQ ID NO: _; Figure 24B shows one embodiment of the P30-4 protein, Figure 24A shows one embodiment of the p30-4 polynucleotide. The p30-5 polynucleotide encodes a P30-5 protein of E. canis having a molecular weight of about 29.4 kDa and an amino acid sequence which is at least 85% homologous to the amino acid sequence shown in FIG. 25B SEQ ID NO: _; Figure 25B shows one embodiment of the P30-5a protein, Figure 25A shows one embodiment of the p30-5a polynucleotide. The p30-6 polynucleotide encodes a P30-6 protein of E. canis having a molecular weight of about 29.5 kDa and an amino acid sequence which is at least 85% homologous to the amino acid sequence shown in FIG. 26B SEQ ID NO: _; Figure 26B shows one embodiment of the P30-6 protein, Figure 26A shows one embodiment of the p30-6 polynucleotide. The p30-7 polynucleotide encodes a P30-7 protein of E. canis having a molecular weight of about 29.9 kDa and an amino acid sequence which is at least 85% homologous to the amino acid sequence shown in FIG. 29B SEQ ID NO: _; Figure 29B shows one embodiment of the P30-7 protein, Figure 29A shows one embodiment of the p30-7 polynucleotide. The p30-8 polynucleotide encodes a P30-8 protein of E. canis having a molecular weight of about 30.3 kDa and an amino acid sequence which is at least 85% homologous to the amino acid sequence shown in FIG. 28B SEQ ID NO: _; Figure 28B shows one embodiment of the P30-8 protein, Figure 28A shows one embodiment of the p30-8 polynucleotide. The p30-9 polynucleotide encodes a P30-9 protein of E. canis comprising an amino acid sequence which is at least 85% homologous to the amino acid sequence shown in FIG. 29B SEQ ID NO: _; Figure 29B shows one embodiment of a portion of the P30-9 protein, Figure 29A shows one embodiment of the p30-9 polynucleotide encoding such polypeptide. The p30-10 polynucleotide encodes a P30-10 protein of E. canis comprising an amino acid sequence which is at least 85% homologous to the amino acid sequence shown in FIG. 30B SEQ ID NO: _; Figure 30B shows one embodiment of a portion of the P30-10 protein, Figure 30A shows one embodiment of the p30-10 polynucleotide encoding such polypeptide.

The polynucleotides encoding an E. chafeensis outer membrane protein or an E. canis outer membrane protein have a sequence that is at least 85%, preferably at least 90%, more preferably at least 95% homologous to or similar to the amino acid sequences shown in Figures 3B through 30B, and thus embrace polynucleotides encoding outer membrane proteins from different strains of E. chafeensis and E. canis. The polynucleotides encode an outer membrane protein whose conserved regions collectively are at least 90%, preferably at 95%, more preferably at least 97% homologous to the conserved regions of the amino acid sequences of the present invention. The outer membrane proteins of E. chafeensis and E. canis have six conserved regions, which are separated by one semivariable region and three hypervariable regions. The conserved regions of the outer membrane proteins OMP-1, OMP-1A, OMP-1B, OMP1-C, OMP-1D, OMP1-F are depicted in Fig. 31. Preferably, the amino acid sequence of the outer membrane proteins of E. chafeensis and E. canis are at least 30% divergent from the amino acid sequence of MAP-1. Such sequences include allelic, strain variants and other amino acid sequence variants (e.g., including "muteins" or "mutant proteins"), whether naturally-occurring or biosynthetically produced. As used herein, "amino acid sequence homology" is understood to mean amino acid sequence similarity, and homologous sequences share identical or similar amino acids, where similar amino acids are conserved amino acids as defined by Dayoff et al., Atlas of Protein Sequence and Structure; vol. 5, Supp. 3, pp. 345-362 (M. O. Dayoff, ed., Nat'l BioMed. Research Fdn., Washington D.C. 1978.) Thus, a candidate sequence sharing 85% amino acid sequence homology with a reference sequence requires that, following alignment of the candidate sequence with the reference sequence, 85% of the amino acids in the candidate sequence are identical to the corresponding amino acid in the reference sequence, or constitute a conserved amino acid change thereto. "Amino acid sequence identity" is understood to require identical amino acids between two aligned sequences. Thus, a candidate sequence sharing 85% amino acid identity with a reference sequence requires that, following alignment of the candidate sequence with the reference sequence, 85% of the amino acids in the candidate sequence are identical to the corresponding amino acid in the reference sequence.

As used herein, all homologies and identities are calculated using the amino acid sequences shown in the cited Figure or SEQ ID NO as the reference sequence. Thus, to determine whether an amino acid sequence is 85% homologous to OMP-1, one uses the amino acid sequence shown in Fig. _, SEQ ID NO: _ as a reference.

Also as used herein, sequences are aligned for homology and identity calculations using the method of the software basic local alignment search tool in the BLAST network service (the National Center for Biotechnology Information, Bethesda, MD) which employs the method of Altschul, S. F., Gish, W., Miller, W., Myers, E. W. & Lipman, D. J. (1990) J. Mol. Biol. 215, 403-410. Identities are calculated by the Align program (DNAstar, Inc.) In all cases, internal gaps and amino acid insertions in the candidate sequence as aligned are ignored when making the homology/identity calculation.

In another aspect, the present invention provides a nucleotide sequence encoding a polypeptide which comprises a fragment of the OMP1 protein, hereinafter referred to as "rP28" . The rP28 polypeptide weighs approximately 31 kDa and comprises all but of the first 5 amino acids of mature OMP-1 protein. The rP28 polypeptide comprises the amino acid sequence extending from amino acid 6 through amino acid 251 of the amino acid sequence shown in Fig.1, SEQ ID NO. The present invention also embraces polypeptides where one or more of the amino acids in the sequence extending from amino acid 1 or 6 through amino acid 251 Fig. 1 are replaced by conservative amino acid residues. The present invention also relates to derivatives of rP28 that have an amino acid sequence identity of at least 85%, more preferably at least 90%, and most preferably of at least 95% with the amino acid sequence extending from amino acid 1 or 6 through amino acid 251 of the protein and which derivative binds to antibodies in sera from humans infected with E. chafeensis.

The polynucleotides are useful for producing the outer membrane proteins of E. chafeensis and E. canis. For example, an RNA molecule encoding the outer membrane protein OMP-1 is used in a cell-free translation systems to prepare OMP-1. Alternatively, a DNA molecule encoding the outer membrane protein is introduced into an expression vector and used to transform cells. Suitable expression vectors include for example chromosomal, nonchromosomal and synthetic DNA sequences, e.g., derivatives of SV40, bacterial plasmids, phage DNAs; yeast plasmids, vectors derived from combinations of plasmids and phage DNAs, viral DNA such as vaccinia, adenovirus, fowl pox virus, and pseudorabies. The DNA sequence is introduced into the expression vector by conventional procedures.

Accordingly, the present invention also relates to recombinant constructs comprising one or more of the polynucleotide sequences. Suitable constructs include, for example, vectors, such as a plasmid, phagemid, or viral vector, into which a sequence that encodes the outer membrane protein has been inserted. In the expression vector, the DNA sequence which encodes the outer membrane protein is operatively linked to an expression control sequence, i.e., a promoter, which directs mRNA synthesis. Representative examples of such promoters, include the LTR or SV40 promoter, the *E .coli* lac or trp, the phage lambda PL promoter and other promoters known to control expression of genes in prokaryotic or eukaryotic cells or in viruses. The promoter may also be the natural promoter of the outer membrane protein coding sequence. The expression vector also contains a ribosome binding site for translation initiation and a transcription terminator. Preferably, the recombinant expression vectors also include an origin of replication and a selectable marker, such as for example, the ampicillin resistance gene of *E*. *coli* to permit selection of transformed cells, i.e. cells that are expressing the heterologous DNA sequences. The polynucleotide sequence encoding the outer membrane protein is incorporated into the vector in frame with translation initiation and termination sequences. Optionally, the sequence encodes a fusion outer membrane protein which includes an N-terminal or C-terminal peptide or tag that stabilizes or simplifies purification of the expressed recombinant product. Representative examples of such tags include sequences which encode a series of histidine residues, the Herpes simplex glycoprotein D, or glutathione S-transferase.

Polynucleotides which encode portions of the outer membrane proteins of E. chafeensis and E. canus are useful as probes for isolating and identifying E. chafeensis genes and E. canis genes, particularly full-length genes from new strains or isolates of E. chafeensis and E. canis.

### The Outer Membrane Proteins of E. chafeensis and E. Canis

In addition to the outer membrane proteins OMP-1, OMP-1B, OMP-1C, OMP-1D, OMP-1 E, and OMP-1F, OMP-1R, OMP-1S, OMP-1T, OMP-1U, OMP-1V, OMP-1W, OMP-1X, OMP-1Y, and OMP-1Z from E. chafeensis and the proteins P30, P30A, P30-1, P30-2, P30-3, P30-4, P30-5, P30-6, P30-7, P30-8, P30-9, and P30-10 from E. Canis, the present inventions embraces non-naturally occurring allelelic forms or derivatives of the outer membrane proteins, where one or more of the amino acids have been replaced by conservative amino acid residues, typically by using direct synthesis or recombinant techniques.

### Preparing the Outer Membrane Proteins

The outer membrane proteins of the present invention are synthetically produced by conventional peptide synthesizers. The outer membrane proteins are also produced using cell-free translation systems and RNA molecules derived from DNA constructs that encode the outer membrane protein. Alternatively, the outer membrane protein is made by transfecting host cells with expression vectors that comprise a DNA sequence which encodes the outer membrane protein and then inducing expression of the outer membrane protein in the host cells.

The outer membrane protein is expressed in suitable host cells, preferably bacteria, under the control of suitable promoters. Host cells are transformed with the expression vectors of this invention and cultured in conventional nutrient media. Such media optionally contains additional compounds, such as for example compounds that induce promoters, such as for example isopropyl-β-D-thiogalactoside which induces the Lac promoter, or compounds, such as for example, ampicillin, which allows for selection of transformants.

Following transformation of the suitable host strain and growth of the host strain to an appropriate cell density, the cells are typically harvested by centrifugation, disrupted by physical or chemical means, and the resulting crude extract retained for further purification of the outer membrane protein. Such purification usually involves salting-out of the protein fraction, and one or more chromatography steps, including aqueous ion exchange chromatography, size exclusion chromatography steps, and high performance liquid chromatography (HPLC).

### Preparation of Antibodies

The isolated outer membrane proteins, particularly the recombinant forms of the outer membrane proteins, are used as immunogens to produce antibodies immunospecific for the corresponding protein. The term "immunospecific" means the antibodies have substantially greater affinity for the protein used as an immunogen than for other proteins. Such antibodies are generated using conventional techniques by administering the respective outer membrane protein or a portion thereof, i.e., the recombinant polypeptide, to an animal, preferably a nonhuman. collecting blood from the immunized animals and isolating the serum and/or the IgG fraction from the blood. Monoclonal antibodies are prepared by injecting animals with the immunogens, extracting antibody-producing B cells from the animal, fusing the B cells with a myeloma cells to produce hybridomas, obtaining the monoclonal antibodies from the hybridomas.

Antibodies to the outer membrane proteins of E. chafeensis and E. canis are useful research tools for identifying cells, particularly monocytes, infected with E.chafeensis or E. canis and for purifying the corresponding outer membrane protein of E.chafeensis or E. Canis from partially purified preparations by affinity chromatography. Such antibodies are also useful for identifying bacterial colonies, particularly colonies of genetically-engineered bacteria, that are expressing the major outer membrane protein .

### Diagnostic Method

The present invention also provides a method for detecting antibodies to the E. chafeensis or E.canis in a sample of a bodily fluid from a patient. The method comprises providing an isolated outer membrane protein of E. chafeensis or E. canis, particularly a recombinant form of the isolated protein, contacting the outer membrane protein or polypeptide with a sample taken from the patient; and assaying for the formation of a complex between the outer membrane protein or polypeptide and antibodies in the sample. For ease of detection, it is preferred that the isolated protein or polypeptide be attached to a substrate such as a column, plastic dish, matrix, or membrane, preferably nitrocellulose. The sample may be a tissue or a biological fluid, including urine, whole blood, or exudate, preferably serum. The sample may be untreated, subjected to precipitation, fractionation, separation, or purification before combining with the isolated protein or peptide. Interactions between antibodies in the sample and the isolated protein or peptide are detected by radiometric, colorimetric, or fluorometric means, size-separation, or precipitation. Preferably, detection of the antibody-outer membrane protein complex is by addition of a secondary antibody that is coupled to a detectable tag, such as for example, an enzyme, fluorophore, or chromophare. Formation of the complex is indicative of the presence of anti-E chafeensis or anti-E canis antibodies, either IgM or IgG, in the patient. Thus, the method is used to determine whether a patient is infected with E. chafeensis or E. canis.

Preferably, the method employs an enzyme-linked immunosorbent assay (ELISA) or a Western immunoblot procedure . Such methods are relatively simple to perform and do not require special equipment as long as membrane strips are coated with a high quality antigen. Accordingly, it is more advantageous to use a recombinant form of the outer membrane protein of E. chafeensis or E. canis since such proteins, typically, are more pure and consistent in quality than a purified form of such protein.

### Immunogenic Composition

The present invention also relates to immunogenic compositions comprising one or more of the isolated outer membrane proteins of E. chafeensis and a pharmaceutically acceptable adjuvant and to immunogenic compositions comprising an isolated P30 protein of E. canis and a pharmaceutically acceptable adjuvant, which, preferably, enhances the immunogenic activity of the outer membrane protein in the host animal.

### Preparation of a Polynucleotide which Encodes OMP-1(P28)

### A. Isolation of the Outer Membrane Proteins

*E. chafeensis* Arkansas strain and E. *canis* Oklahoma strain were cultivated in the DH82 dog macrophage cell line and purified by Percoll density gradient centrifugation. Purified ehrlichiae (100 µg) were suspended with 10 mM sodium phosphate buffer, pH 7.4, containing 0.1% Sodium *N*-lauroyl sarcosine (Sarkosyl) [Sigma, St. Louis, MO], 50 µg/ml each Dnase I (Sigma) and Rnase A (Sigma), and 2.5 mM MgCl₂. After incubation at 37° for 30 min, the sample was separated by centrifugation at 10,000 x g for I h into the soluble supernatant and the insoluble precipitate. The insoluble pellet was resuspended 2 to 3 times with 0.1% Sarkosyl and centrifuged. The final pellet was analyzed by sodium dodecyl sulfate (SDS)-polyacrylamide gel electrophoresis (PAGE) and by electron microscopy.

Transmission electron microscopy revealed that the purified ehrlichial fraction consists of a mixture of electron dense and light forms of *E. chafeensis* with slight disintegration of inner membrane. Ehrlichiae were not surrounded with the host inclusion membrane. Various sizes of membrane vesicles (< 1 µm) without significant ribosomes or nuclear materials were observed in the Sarkosyl-insoluble fraction from the organism. Succinic dehydrogenase (inner membrane marker enzyme of gram negative bacteria) activities were at less than the detection limit (1 n moles / min / mg of protein) in the Sarkosyl-insoluble fraction compared to approximately 10 n moles / min / mg of protein in the Percoll-purified organisms, suggesting that the insoluble fraction primarily consisted of the outer membrane of *E. chafeensis.*

Analysis of the Sarkosyl-soluble, and insoluble fraction of *E. chafeensis* by SDS-PAGE suggested that proteins of 30-kDa range in the insoluble fraction represent the major outer membrane proteins of this organism. Analysis of the Sarkosyl-soluble, and insoluble fraction of *E. canis* by SDS-PAGE suggested that proteins of 30-kDa range in the insoluble fraction represent the major outer membrane proteins of this organism also. *E. canis* was antigenically cross reactive with *E. chafeensis.* These findings indicate that the 30-kDa range proteins represent the major outer membrane proteins of these two *Ehrlichia* spp.

To improve resolution of the outer membrane proteins, proteins in the Sarkosyl-insoluble pellet prepared from 400 µg of purified *E. chafeensis* were separated by a reversed-discontinuous (Rd) SDS-PAGE (2.5-cm-long 17% gel on top of 11-cm-long 12% gel). At least five proteins of 30-kDa range in *E. chafeensis* (P23, P25, P27, P28, and P29) were resolved from the Sarkosyl-insoluble proteins.

### B. Cloning and sequencing of the p28 gene

The portion of the membrane containing bound proteins was excised and analyzed with an Applied Biosystems protein sequencer (Model 470). The N-terminal amino acid sequence of P28 was determined as D P A G S G I N G N F Y S G K Y M P, SEQ IN NO _. Based on 6th to 12th amino acids of this sequence, a forward primer, FECH1, having the sequence: 5'-CGGGATCCGAATTCGG(A/T/G/C)AT(A/T/C)AA(T/C)GG(A/T/G/C)AA(T/C)TT(T/C)TA-3'. SEQ ID NO was designed. Amino acids at the 1 to 5 positions of the N terminus of P28 were not included in this primer design. For insertion into an expression vector, a 14-bp sequence (underlined) was added at the 5' end of primer to create an *EcoRI* and a *Bam*HI site. The reverse primer, RECH2, which includes a *Not*I site at the 5' end for ligation into an expression vector had the sequence : 5'-AGCGGCCGCTTA(A/G)AA(T/C)A(C/G) (A/G)AA (C/T)CT T(C/G)C TCC-3'. SEQ ID NO _.

Genomic DNA of *E. chafeensis* was isolated from purified organisms. PCR amplification with FECH1 and RECH2 primers was performed using a Perkin-Elmer Cetus DNA Thermal Cycler (model 480). A 0.8-kb amplified product was cloned in the pCRII vector of a TA closing kit, as described by the manufacturer (Invitrogen Co., San Diego, CA). The clone obtained was designated pCRII*p*28. Both strands of the inserted DNA were sequenced by a dideoxy-termination method with an Applied Biosystems 373A DNA sequencer.

The 0.8-kb DNA fragment, cloned in pCRII*p*28, had an open reading frame (ORF) of 756 bp encoding a 251-amino acid recombinant protein (including both PCR primer regions) with a molecular mass of 27,685 Da. The nucleotide sequence of the open reading frame, SEQ ID NO: , and the amino acid sequence of the polypeptide of the OMP-1 protein, SEQ ID NO _, are shown in Figs _ and _, respectively.

A DNA fragment comprising the *p30* gene was prepared in a similar manner, i.e., by PCR amplification of genomic DNA of E. canis with the FECH1 and RECH2 primers.

### Preparation of Polynucleotides which encode OMP 1A, OMP1B, OMP1-C, OMP-1D, OMP-1F, and OMP1-E

A. Southern blot analysis. Genomic DNA extracted from the purified *E. chafeensis* (200 ng each) was digested with restriction endonucleases, electrophoresed, and transferred to Hybond-N⁺ nylon membrane (Amersham, Arlington Heights, IL), by a standard method. The 0.8-kb p28 gene fragment from the clone pCRII*p*28 was labeled with [α-³²P]dATP by the random primer method using a kit (Boehringer Mannheim, Indianapolis, IN) and the labeled fragment was used as a DNA probe. Hybridization was performed at 60°C in rapid hybridization buffer (Amersham) for 20 h. The nylon sheet was washed in 0.1 x SSC (1 x SSC containing 0.15M sodium chloride and 0.015M sodium citrate) with 1% SDS at 55°C and the hybridized probes were exposed to Hyperfilm (Amersham) at -80°C.

Genomic Southern blot analysis with several restriction enzymes resulted in one or more DNA fragment(s) of *E. chafeensis* which hybridized to ³²P-labeled *p28* gene probe. The restriction enzymes used did not cut within the *p28* gene portion of the pCRII*p*28 insert. *Xba* I, *Bgl* II, and *Kpn* I produced two bands, *Spe* I generated three bands, and *EcoR* V and *Pst* I produced multiple bands with different densities. *Eco*R I generated a broad band of 2.5 to 4kb. These *p28* homologous genes are designated as *omp-1* (outer membrane protein-1) family.

B. Cloning and sequencing of genomic copies of *E. chafeensis p28* gene. The *EcoR* I and *Pst* I fragments of DNA, detected by genomic Southern blot analysis as described above, were inserted into pBluescript II KS (+) vectors, and the recombinant plasmids were introduced into *E. coli* DH5α. Using the colony hybridization method with the ³²P-labeled *p28* gene probe, four positive clones were isolated from the transformant. The positive clones were designated pEC2.6, pEC3.6, pPS2.6, and pPS3.6. These contained the ehrlichial DNA fragments of 2.6-kb (EcoR I), 3.6 kb (EcoR I), 2.6 kb *(Pst* I), and 3.6 kb *(Pst* I), respectively. The inserts of the clones pEC3.6 and pPS2.6 overlapped as shown in Fig.__. The overlapping area was further confirmed by PCR of *E. chafeensis* genomic DNA with two pairs of primer sets interposing the junctions of the four clones. The 1.1- to 1.6-kb DNA fragments of *Hind*III*-Hind*III*, Hind*III*-EcoR*I*,* or *Xho*I-*Eco*RI in the pEC2.6 and pEC3.6 were subcloned for sequencing. DNA sequencing was performed with suitable synthetic primers by dideoxy-termination method as described above.

Four DNA fragments from 2.6 to 3.6 kb were cloned from the *Eco*RI-digested and the *Pst*I-digested genomic DNA of *E. chafeensis* by colony hybridization with radiolabeled p28 gene probe. The inserted DNA of the two recombinant clones, pEC3.6 and PPS2.6, were overlapped as shown in Fig. 7. Sequencing revealed one 5'-truncated ORF of 243 bp (designated *omp*-1A) and five complete ORF of 836-861 bp (designated *omp-1*B to *omp-*IF), which are tandemly-arrayed and are homologous to the p28 gene (but are not identical), in the ehrlichial genomic DNA of 6,292 bp. The intergenic spaces were 581 bp between *omp-1*A and *omp-1*B and 260-308 bp among others. Putative promoter regions and ribosome-binding sites were identified in the noncoding regions.

### Sequence analysis and GenBank accession number.

Nucleotide sequences were analyzed with the DNASIS program (Hitachi Software Engineering Co., Ltd., Yokohama, Japan). A homology search was carried out with databases of the GenBank, Swiss Plot, PDB and PIR by using the software basic local alignment search tool in the BLAST network service (the National Center for Biotechnology Information, Bethesda, MD). Phylogenetic analysis was performed by using the PHYLIP software package (version 3.5). An evolutional distance matrix, generated by using the Kimura formula in the PROTDIST, was used for construction of a phylogenetic tree by using the unweighted pair-group method analysis (UPGMA) (Felsenstein, J. 1989. PHYLIP-phylogeny inference package (version 3.3). Cladistics 5:164-166). The data were also examined using parsimony analysis (PROTPARS in PHYLIP). A bootstrap analysis was carried out to investigate the stability of randomly generated trees by using SEQBOOT and CONSENSE in the same package. The nucleotide sequence of the *p28* gene and its gene copies has been assigned GenBank accession numbers U72291 and AF021338, respectively.

### Proteins of the E. chafeensis omp-1 Family.

Five complete *omp-1* gene copies (*omp-1B* to *omp-1*F) encode 279 to 287-amino acid proteins with molecular masses of 30,320 - 31,508 Da. *Omp-1A* encodes an 82-amino acid partial protein (9,243 Da) which lacks the N-terminal region. The 25-amino acid sequence at the N-terminus of OMP-1B to OMP-IF (encoded in *omp-1*B to *omp-IF)* is predicted to be a signal peptide because three carboxyl-terminal amino acids of the signal peptides (Ser-X-Ala in OMP-1B, Leu-X-Ser for OMP-C, and Ser-X-Ser for OMP-1D and OMP-1F) are included in the preferred amino acid sequence of signal peptidase at the processing sites proposed by Oliver .. The calculated molecular masses of the mature OMP-1B to OMP-1F from the predicted amino acid sequences are 28,181 Da for OMP-1B, 27,581 Da for OMP-1C, 28,747 Da for OMP-1D, 27,776 Da for OMP-1E, and 27,933 Da for OMP-IF. The estimated isoelectric points are 4.76-5.76 in the mature OMP-1B to OMP-1F. An amino acid sequence in *omp-1*F gene (the 80th to 94th amino acids) was identical to the N-terminal amino acid sequences of *E. chafeensis* native P23 protein as determined chemically, which indicates that P23 is derived from the *omp-1F* gene. Amino acid sequences identical to the N-terminal sequences of P25, P27, and P29 were not found in those from *omp-1* gene copies cloned in this study.

Alignment of predicted amino acid sequences of the *E. chafeensis* OMP-1 family and *Cowdria ruminantium,* revealed substitutions or deletions of one or several contiguous amino acid residues throughout the molecules. The significant differences in sequences among the aligned proteins are seen in the regions indicated SV (semivariable region) and HV (hypervariable region) 1 to 3 in Fig 31. Computer analysis for hydropathy revealed that protein molecules predicted from all *omp-1* gene copies contain alternative hydrophilic and hydrophobic motifs which are characteristic of transmembrane proteins. The HV1 and HV2 were found to locate in the hydrophilic regions.

The amino acid sequences of 5 mature proteins without signal peptides (OMP-1C to OMP-1F and a P28) were similar to one another (71-83%) but the sequence of OMP-1B was dissimilar to those of the 5 proteins (45-48%). The amino acid sequences of the 5 proteins showed an intermediate degree of similarity with that of *C*. *ruminantium* MAP-1 (59-63%), but the similarity between that of the OMP-1B and the *C*. *ruminantium* MAP-1 was low (45%). These relations are shown in a phylogenetic tree which was obtained based on the amino acid sequence alignment by UPGMA method in the PHYLIP software package (Fig. 10). Three proteins (P28, OMP-1D, and OMP-1F) and two proteins (OMP-1C and OMP-IE) formed two separate clusters. The OMP-1B was located distantly from these two clusters. The *C*. *ruminantium* MAP-1 was positioned between the OMP-1B and other members in the OMP-1 family.

### Preparation of a Recombinant form of OMP-1 and P30

The 0.8-kb p28 gene was excised from the clone pCRII*p*28 by *Eco*RI*-Not*I double-digestion, ligated into *EcoR*I*-Not*I sites of a pET 29a expression vector, and amplified in *Escherichia coli* BL21 (DE3)pLysS (Novagen, Inc., Madison, WI). The clone (designated pET29*p*28) produced a fusion protein with a 35-amino acid sequence carried from the vector at the N terminus. The amino acid sequence of the OMP-1 portion of the fusion protein is depicted in Fig. 1.

An expression vector comprising the p30 gene was used to prepare the recombinant form of P30.

The following examples are for purposes of illustration only and are not intended to limit the scope of the claims which are appended hereto.

### Preparation of anti rP28 (anti-OMP1) antibody

The (r) P28 antigen was prepared by excising the gel band corresponding to the rP28 in SDS-PAGE, mincing the band in phosphate-buffered saline (PBS), pH 7.4, and mixing with an equal volume of Freund's incomplete adjuvant (Sigma). The rP28 mixture (1 mg of protein each time) was subcutaneously injected into a rabbit every 2 weeks four times. A serum sample was collected from the rabbit to provide the anti-rP28 antibody

The anti-rP28 antibody was examined by western immunoblots analysis. The results indicated that the rabbit anti-rP28 antibody recognized not only rP28 (31 kDa) and P28, but also. P29 and P25 of *E. chafeensis* and P30 of *E. canis.* These results indicate that P28 shares antigenic epitopes with P25 and P29 in *E. chafeensis* and P30 of *E. canis.*

### Example 1. Assaying for the presence of anti-OMP-1 antibody in a Patient

Convalescent-phase serum from a patient with clinical signs of human ehrlichiosis was used. Western blot analyses using the rP28 protein as antigen was performed with 1:1,000 dilutions of this serum. Alkaline phosphatase-conjugated affinity-purified anti-human, anti-rabbit or anti-mouse immunoglobulin G (Kirkegaard & Perry Laboratories, Inc., Gaithersburg, MD) were used at a 1:1,000 or 1:2,000 dilution as secondary antibodies. Results indicated that serum from a patient with clinical signs of human ehrlichiosis reacted strongly to rP28 (31 kDa).

### Example 2 Assaying for the presence of anti-OMP-1 antibody in a Patient

Convalescent-phase serum from a patient with clinical signs of human ehrlichiosis was reacted with the rP30 protein of E.canis as described in Example 1. The serum reacted strongly to rP30. These results indicate the rP30 is useful for diagnosing an infection with E. chafeensis in human patients.

### Example 3. Identifying E. chafeensis-infected cells usine anti-rP 28 antibody

*E. chafeensis*-infected DH82 cells were sonicated and centrifuged at 400 x g for 10 min. The supernatant was then centrifuged at 10,000 x g for 10 min to obtain ehrlichia-enriched pellet. The pellet was resuspended and incubated with rabbit anti-rP28 antibody or normal rabbit serum (1:100 dilution) at 37°C for 1h in PBS containing 1% bovine serum albumin (BSA-PBS). After washing, the ehrlichiae was incubated with gold-conjugated protein G (20 nm), Sigma) at 1:30 dilution for 1 h at room temperature in BSA-PBS. After washing again, the specimen was fixed with 1.25% formaldehyde, 2.5% glutaraldehyde, and 0.03% trinitrophenol in 0.1 M cacodylate buffer (pH 7.4) for 24h and postfixed in 1% osmium-1.5% potassium ferricyanide for 1 h (34). The section was then embedded in PolyBed 812 (Polysciences, Warraington, Pa). The specimen was ultrathin sectioned at 60 nm, stained with uranyl acetate and lead citrate, and observed with a Philips 300 transmission electron microscope at 60 kV.

Transmission immunoelectron microscopy with colloidal gold-conjugated protein G and rabbit anti-rP28 antibody revealed gold particles bound to *E. chafeensis* surface. The distribution of the particles was random, close to the surface, and appeared as if almost embedded in the membrane, suggesting that the antigenic epitope protrudes very little from the lipid bilayer. Nonetheless, the antigenic epitope was surface-exposed, and thus, could be recognized by rabbit anti-rP28 antibody. No gold particles were observed on host cytoplasmic membrane or *E. chafeensis* incubated with normal rabbit serum.

### Example 4. Immunization of mice and E. chafeensis challenge.

The rP28 band in SDS- PAGE was excised, minced, and mixed with an equal volume of Freund's incomplete or complete adjuvant. Nine BALB/c male mice (6 weeks old) were divided into two groups. Five mice were intraperitoneally immunized a total of four times at 10-day intervals; twice with a mixture of the minced gel with the rP28 (30 to 40 µg of protein per mouse each time) and incomplete adjuvant, and twice with a mixture of the recombinant protein (the same amount as before) and complete adjuvant. Four mice were intraperitoneally injected with a mixture of the minced gel without protein and the respective adjuvants. For ehrlichia-challenge, approximately 1 x 10⁷ DH82 cells heavily-infected with *E. chafeensis* were disrupted by sonication in serum-free DMEM (GIBCO-BRL) and centrifuged at 200 x g for 5 min. The supernatant was diluted to a final volume of 5 ml, and 0.3 ml was inoculated intraperitoneally into each mouse 10 days after the last immunization. Before challenge, all 5-immunized mice had a titer of 1:160 against *E. chafeensis* antigen by IFA and all 4-nonimmunized mice were negative.

At day 5 post-challenge, approximately 1 ml of blood was collected in an EDTA tube from each mouse and protection was assessed by PCR detection of *E. chafeensis* 16S rDNA in the buffy coat of the collected blood. *E. chafeensis* could not be reisolated in cell culture at day 10 postinfection. Day 5 post challenge is the optimum time at which establishment of ehrlichial infection can be examined by PCR without the influence of residual DNA from the ehrlichiae used as the challenge before the spontaneous clearance of organisms take place. The *E. chafeensis*-specific DNA fragment was observed in all nonimmunized mice but not in any immunized mice, indicating that immunization of rP28 apparently protects mice from ehrlichial infection and indicating that the P28 is a potential protective antigen

### Example 5 Assaying for the presence of anti-P30 antibody in Dogs

The rP30 protein was used as an antigen in a Western immunoblot analysis and dot blot analysis to detect the presence of antibody to E. canis in serum from E-canis infected dogs. The results of the Western immunoblot analysis indicated that reactivity of the sera with rP30 was stronger than the reactivity that was observed when purified E.canis was used as antigen. The results of the dot blot assay indicated that rP30 is a useful and sensitive tool for serodiagnosis of canine ehrlichiosis.
1. An isolated polynucleotide encoding an outer membrane protein of E. chafeensis or an immunogenic fragment thereof, wherein the outer membrane protein is selected from the group consisting of OMP-1, OMP-1B, OMP-1C, OMP-1D, OMP-1E, OMP-1F, OMP-1R, OMP-1S, OMP-IT, OMP-1U OMP-1V, OMP-1W OMP-1X OMP-1Y, and OMP-1Z.
2. The isolated polynucleotide of para 1 wherein the polynucleotide encodes an OMP-1 protein comprising a sequence which is at least 85% homologous to the amino acid sequence shown in Fig. 3B, SEQ. ID NO _.
3. The isolated polynucleotide of para 1 wherein the polynucleotide encodes an OMP-1B protein comprising a sequence which is at least 85% homologous to the amino acid sequence shown in Fig. 4B, SEQ. ID NO _.
4. The isolated polynucleotide of para 1 wherein the polynucleotide encodes an OMP-1C protein comprising a sequence which is at least 85% homologous to the amino acid sequence shown in Fig.5B, SEQ. ID NO _.
5. The isolated polynucleotide of para 1 wherein the polynucleotide encodes an OMP-1D protein comprising a sequence which is at least 85% homologous to the amino acid sequence shown in Fig. 6B , SEQ. ID NO _.
6. The isolated polynucleotide of para I wherein the polynucleotide encodes an OMP-1E protein comprising a sequence which is at least 85% homologous to the amino acid sequence shown in Fig. 7B, SEQ. ID NO _.
7. The isolated polynucleotide of para 1 wherein the polynucleotide encodes an OMP-1F protein comprising a sequence which is at least 85% homologous to the amino acid sequence shown in Fig. 8B, SEQ. ID NO _.
8. The isolated polynucleotide of para 1 wherein the polynucleotide encodes an immunogenic fragment of the OMP-1 protein, said fragment comprising a sequence which is at least 85% homologous to the amino acid sequence extending from amino acid 6 through amino acid 251 as shown in Fig. 1, SEQ. ID NO _.
9. An isolated polynucleotide encoding an outer membrane protein of E. canis or an immunogenic fragment thereof, wherein the outer membrane protein is selected from the group consisting of P30, P30-A, P30-1, P30-2, P30-3, P30-4, P30-5, P30-6, P30-7, P30-8, P30-9, P30-10.
10. The isolated polynucleotide of para 9 wherein said P30 protein comprises a sequence which is at least 85% homologous to the amino acid sequence shown in Fig, 19 SEQ ID NO.
11. An isolated outer membrane protein of E. chafeensis or an immunogenic fragment thereof, wherein said protein is selected from the group consisting of OMP-1, OMP-1B, OMP-1C, OMP-1D, OMP-1E, OMP-1F, OMP-1R, OMP-1S, OMP-1T, OMP-1U OMP-1V, OMP-1W OMP-1X OMP-1Y, and OMP-1Z.
12. The isolated OMP-1 protein of para11, wherein said protein comprises a sequence which is at least 85% homologous to the amino acid sequence shown in Fig. 4B, SEQ. ID NO _.
13. The isolated OMP-1B protein of Para 11 wherein said protein comprises a sequence which is at least 85% homologous to the amino acid sequence shown in Fig. 5B, SEQ. ID NO _.
14. The isolated OMP-1C protein of Para 11 wherein said protein comprises a sequence which is at least 85% homologous to the amino acid sequence shown in Fig. 6B, SEQ. ID NO _.
15. The isolated OMP-1D protein of para 11 wherein said protein comprises a sequence which is at least 85% homologous to the amino acid sequence shown in Fig. 7B, SEQ. ID NO _.
16. The isolated OMP-1E protein of para 11 wherein said protein comprises a sequence which is at least 85% homologous to the amino acid sequence shown in Fig. 8B, SEQ. ID NO _.
17. The isolated OMP-1F protein of para 11 wherein said protein comprises a sequence which is at least 85% homologous to the amino acid sequence shown in Fig. 9B, SEQ. ID NO _.
18. The isolated immunogenic fragment of the OMP-1 protein of para 11, said fragment comprising a sequence which is at least 85% homologous to the amino acid sequence extending from amino acid 6 through amino acid 251 as shown in Fig. 1, SEQ. ID NO _.
19. An isolated outer membrane protein of E. canis or an immunogenic fragment thereof, wherein the outer membrane protein is selected from the group consisting of P30, P30-A, P30-1, P30-2, P30-3, P30-4, P30-5, P30-6, P30-7, P30-8, P30-9, P30-10.
20. The isolated P-30 protein of para 19 wherein said protein comprises a sequence which is at least 85% homologous to the amino acid sequence shown in Fig 19, SEQ ID NO. _.
21. A method for diagnosing an infection with E. chafeensis in a patient comprising the steps of:
   (a) providing a serum sample from the patient;
   (b) providing an outer membrane protein selected from the group consisting of a protein of para 11, a protein of para 19, and mixtures thereof;
   (c) contacting the serum sample with the outer membrane protein; and
   (d) assaying for the formation of a complex between antibodies in the serum sample and
   the outer membrane protein, wherein formation of said complex is
   indicative of infection with E. chafeensis.
22. A method for diagnosing an infection with E. canis in a Canidae patient comprising the steps of:
   (a) providing a serum sample from the patient;
   (b) providing an outer membrane protein of Para 19;
   (c) contacting the serum sample with the outer membrane protein; and
   (d) assaying for the formation of a complex between antibodies in the serum sample and
   the outer membrane protein, wherein formation of said complex is
   indicative of infection with E. canis.

## Claims

1. An isolated outer membrane protein of *E. canis* or an immunogenic fragment thereof, wherein the outer membrane protein comprises an amino acid sequence which is at least 85% homologous to an amino acid sequence selected from the group consisting of:
the amino acid sequence shown in Fig. 19B showing P30,
the amino acid sequence shown in Fig. 20B showing P30-A,
the amino acid sequence shown in Fig. 21B showing P30-1,
the amino acid sequence shown in Fig. 22B showing P30-2,
the amino acid sequence shown in Fig. 23B showing P30-3,
the amino acid sequence shown in Fig. 24B showing P30-4,
the amino acid sequence shown in Fig. 25B showing P30-5,
the amino acid sequence shown in Fig. 26B showing P30-6,
the amino acid sequence shown in Fig. 27B showing P30-7,
the amino acid sequence shown in Fig. 28B showing P30-8,
the amino acid sequence shown in Fig. 29B showing a portion of P30-9,
the amino acid sequence shown in Fig. 30B showing a portion of P30-10.

2. An isolated outer membrane protein as claimed in claim 1 which comprises an amino acid sequence selected from the group consisting of:
the amino acid sequence shown in Fig. 19B showing P30,
the amino acid sequence shown in Fig. 20B showing P30-A,
the amino acid sequence shown in Fig. 21B showing P30-1,
the amino acid sequence shown in Fig. 22B showing P30-2,
the amino acid sequence shown in Fig. 23B showing P30-3,
the amino acid sequence shown in Fig. 24B showing P30-4,
the amino acid sequence shown in Fig. 25B showing P30-5,
the amino acid sequence shown in Fig. 26B showing P30-6,
the amino acid sequence shown in Fig. 27B showing P30-7,
the amino acid sequence shown in Fig. 28B showing P30-8,
the amino acid sequence shown in Fig. 29B showing a portion of P30-9,
the amino acid sequence shown in Fig. 30B showing a portion of P30-10.

3. An isolated outer membrane protein as claimed in claim 1 which comprises an amino acid sequence which is at least 85% homologous to the amino acid sequence shown in Fig 19B showing P30.

4. An isolated polynucleotide encoding an outer membrane protein of *E*. *canis* or an immunogenic fragment thereof as claimed in any one of claims 1 to 3.

5. An isolated polynucleotide as claimed in claim 4 encoding an amino acid sequence selected from the group consisting of :
the amino acid sequence shown in Fig. 19B showing P30,
the amino acid sequence shown in Fig. 20B showing P30-A,
the amino acid sequence shown in Fig. 21B showing P30-1,
the amino acid sequence shown in Fig. 22B showing P30-2,
the amino acid sequence shown in Fig. 23B showing P30-3,
the amino acid sequence shown in Fig. 24B showing P30-4,
the amino acid sequence shown in Fig. 25B showing P30-5,
the amino acid sequence shown in Fig. 26B showing P30-6,
the amino acid sequence shown in Fig. 27B showing P30-7,
the amino acid sequence shown in Fig. 28B showing P30-8,
the amino acid sequence shown in Fig. 29B showing a portion of P30-9,
the amino acid sequence shown in Fig. 30B showing a portion of P30-10.

6. An isolated polynucleotide of claim 4 which encodes an amino acid sequence which is at least 85% homologous to the amino acid sequence shown in Fig, 19B showing P30.

7. A method for diagnosing an infection with *E. canis* in a Canidae patient comprising the steps of:
(a) providing a serum sample from the patient;
(b) providing an outer membrane protein of claim 1;
(c) contacting the serum sample with the outer membrane protein; and
(d) assaying for the formation of a complex between antibodies in the serum sample and
the outer membrane protein, wherein formation of said complex is
indicative of infection with *E. canis.*

8. An immunogenic composition comprising
(a) an *E. canis* protein comprising an amino acid sequence which is at least 85% homologous to the amino acid sequence shown in Fig 19B showing P30, and
(b) a pharmaceutically acceptable adjuvant.

9. Use of an *E. canis* protein comprising an amino acid sequence which is at least 85% homologous to the amino acid sequence shown in Fig 19B showing P30 for preparing an antibody that is useful for immunolabeling isolates of *E*. *canis.*

10. A vaccine comprising an *E. canis* protein comprising an amino acid sequence which is at least 85% homologous to the amino acid sequence shown in Fig 19B showing P30, for protecting animals against infection with *E. canis.*


SEQUENCE LISTING

<110>  The Ohio State University Research Foundation

<120>  Outer Membrane Protein of Ehrlichia Canis and Ehrlichia Chaffeensis

<130>  SMK/FP6703466

<140>  EP 10007543.1
<141>  1998-09-18

<140>  EP 98949384.6

<141>  1998-09-18

<150>  PCT/US98/19600

<151>  1998-09-18

<150>  US 60/059,353

<151>  1997-09-19

<160>  64

<170>  PatentIn version 3.0

<210>  1

<211>  846

<212>  DNA

<213>  Ehrlichia chaffeensis

<220>

<221>  CDS

<222>  (1)..(846)

<400>  1
atg aat tac aaa aaa gtt ttc ata aca agt gca ttg ata tca tta ata        48
Met Asn Tyr Lys Lys Val Phe Ile Thr Ser Ala Leu Ile Ser Leu Ile
1               5                   10                  15

tct tct cta cct gga gta tca ttt tcc gac cca gca ggt agt ggt att 96
Ser Ser Leu Pro Gly Val Ser Phe Ser Asp Pro Ala Gly Ser Gly Ile
            20                  25                  30

aac ggt aat ttc tac atc agt gga aaa tac atg cca agt gct tcg cat 144
Asn Gly Asn Phe Tyr Ile Ser Gly Lys Tyr Met Pro Ser Ala Ser His
        35                  40                  45

ttt gga gta ttc tct gct aag gaa gaa aga aat aca aca gtt gga gtg 192
Phe Gly Val Phe Ser Ala Lys Glu Glu Arg Asn Thr Thr Val Gly Val
    50                  55                  60

ttt gga ctg aag caa aat tgg gac gga agc gca ata tcc aac tcc tcc 240
Phe Gly Leu Lys Gln Asn Trp Asp Gly Ser Ala Ile Ser Asn Ser Ser
65                  70                  75                  80

cca aac gat gta ttc act gtc tca aat tat tca ttt aaa tat gaa aac 288
Pro Asn Asp Val Phe Thr Val Ser Asn Tyr Ser Phe Lys Tyr Glu Asn
                85                  90                  95

aac ccg ttt tta ggt ttt gca gga gct att ggt tac tca atg gat ggt 336
Asn Pro Phe Leu Gly Phe Ala Gly Ala Ile Gly Tyr Ser Met Asp Gly
            100                 105                 110

cca aga ata gag ctt gaa gta tct tat gaa aca ttt gat gta aaa aat 384
Pro Arg Ile Glu Leu Glu Val Ser Tyr Glu Thr Phe Asp Val Lys Asn
        115                 120                 125

caa ggt aac aat tat aag aat gaa gca cat aga tat tgt gct cta tcc 432
Gln Gly Asn Asn Tyr Lys Asn Glu Ala His Arg Tyr Cys Ala Leu Ser
    130                 135                 140

cat aac tca gca gca gac atg agt agt gca agt aat aat ttt gtc ttt 480
His Asn Ser Ala Ala Asp Met Ser Ser Ala Ser Asn Asn Phe Val Phe
145                 150                 155                 160

cta aaa aat gaa gga tta ctt gac ata tca ttt atg ctg aac gca tgc 528
Leu Lys Asn Glu Gly Leu Leu Asp Ile Ser Phe Met Leu Asn Ala Cys
                165                 170                 175

tat gac gta gta ggc gaa ggc ata cct ttt tct cct tat ata tgc gca 576
Tyr Asp Val Val Gly Glu Gly Ile Pro Phe Ser Pro Tyr Ile Cys Ala
            180                 185                 190

ggt atc ggt act gat tta gta tcc atg ttt gaa gct aca aat cct aaa 624
Gly Ile Gly Thr Asp Leu Val Ser Met Phe Glu Ala Thr Asn Pro Lys
        195                 200                 205

att tct tac caa gga aag tta ggt tta agc tac tct ata agc cca gaa 672
Ile Ser Tyr Gln Gly Lys Leu Gly Leu Ser Tyr Ser Ile Ser Pro Glu
    210                 215                 220

gct tct gtg ttt att ggt ggg cac ttt cat aag gta tta ggg aac gaa 720
Ala Ser Val Phe Ile Gly Gly His Phe His Lys Val Leu Gly Asn Glu
225                 230                 235                 240

ttt aga gat att cct act ata ata cct act gga tca aca ctt gca gga 768
Phe Arg Asp Ile Pro Thr Ile Ile Pro Thr Gly Ser Thr Leu Ala Gly
                245                 250                 255

aaa gga aac tac cct gca ata gta ata ctg gat gta tgc cac ttt gga 816
Lys Gly Asn Tyr Pro Ala Ile Val Ile Leu Asp Val Cys His Phe Gly
            260                 265                 270

ata gaa ctt gga gga agg ttt gta ttc taa 846
Ile Glu Leu Gly Gly Arg Phe Val Phe
        275                 280

<210>  2

<211>  281

<212>  PRT

<213>  Ehrlichia chaffeensis

<400>  2

Met Asn Tyr Lys Lys Val Phe Ile Thr Ser Ala Leu Ile Ser Leu Ile
1               5                   10                  15

Ser Ser Leu Pro Gly Val Ser Phe Ser Asp Pro Ala Gly Ser Gly Ile
            20                  25                  30

Asn Gly Asn Phe Tyr Ile Ser Gly Lys Tyr Met Pro Ser Ala Ser His
        35                  40                  45

Phe Gly Val Phe Ser Ala Lys Glu Glu Arg Asn Thr Thr Val Gly Val
    50                  55                  60

Phe Gly Leu Lys Gln Asn Trp Asp Gly Ser Ala Ile Ser Asn Ser Ser
65                  70                  75                  80

Pro Asn Asp Val Phe Thr Val Ser Asn Tyr Ser Phe Lys Tyr Glu Asn
                85                  90                  95

Asn Pro Phe Leu Gly Phe Ala Gly Ala Ile Gly Tyr Ser Met Asp Gly
            100                 105                 110

Pro Arg Ile Glu Leu Glu Val Ser Tyr Glu Thr Phe Asp Val Lys Asn
        115                 120                 125

Gln Gly Asn Asn Tyr Lys Asn Glu Ala His Arg Tyr Cys Ala Leu Ser
    130                 135                 140

His Asn Ser Ala Ala Asp Met Ser Ser Ala Ser Asn Asn Phe Val Phe
145                 150                 155                 160

Leu Lys Asn Glu Gly Leu Leu Asp Ile Ser Phe Met Leu Asn Ala Cys
                165                 170                 175

Tyr Asp Val Val Gly Glu Gly Ile Pro Phe Ser Pro Tyr Ile Cys Ala
            180                 185                 190

Gly Ile Gly Thr Asp Leu Val Ser Met Phe Glu Ala Thr Asn Pro Lys
        195                 200                 205

Ile Ser Tyr Gln Gly Lys Leu Gly Leu Ser Tyr Ser Ile Ser Pro Glu
    210                 215                 220

Ala Ser Val Phe Ile Gly Gly His Phe His Lys Val Leu Gly Asn Glu
225                 230                 235                 240

Phe Arg Asp Ile Pro Thr Ile Ile Pro Thr Gly Ser Thr Leu Ala Gly
                245                 250                 255

Lys Gly Asn Tyr Pro Ala Ile Val Ile Leu Asp Val Cys His Phe Gly
            260                 265                 270

Ile Glu Leu Gly Gly Arg Phe Val Phe
        275                 280

<210>  3

<211>  852

<212>  DNA

<213>  Ehrlichia chaffeensis

<220>

<221>  CDS

<222>  (1)..(852)

<400>  3
atg aat tac aag aaa att ttt gta agc agt gca tta att tca tta atg        48
Met Asn Tyr Lys Lys Ile Phe Val Ser Ser Ala Leu Ile Ser Leu Met
1               5                   10                  15

tca atc tta cct tac caa tct ttt gca gat cct gta act tca aat gat        96
Ser Ile Leu Pro Tyr Gln Ser Phe Ala Asp Pro Val Thr Ser Asn Asp
            20                  25                  30

aca gga atc aac gac agc aga gaa ggc ttc tac att agt gta aag tat       144
Thr Gly Ile Asn Asp Ser Arg Glu Gly Phe Tyr Ile Ser Val Lys Tyr
        35                  40                  45

aat cca agc ata tca cac ttc aga aaa ttc tca gct gaa gaa gct ccc       192
Asn Pro Ser Ile Ser His Phe Arg Lys Phe Ser Ala Glu Glu Ala Pro
    50                  55                  60

atc aat gga aat act tct atc act aaa aag gtt ttc ggg ctg aaa aaa       240
Ile Asn Gly Asn Thr Ser Ile Thr Lys Lys Val Phe Gly Leu Lys Lys
65                  70                  75                  80

gac gga gat ata gca caa tct gcg aat ttt aac agg aca gat cca gcc       288
Asp Gly Asp Ile Ala Gln Ser Ala Asn Phe Asn Arg Thr Asp Pro Ala
                85                  90                  95

ctc gag ttt cag aat aac cta ata tca gga ttc tca gga agt att ggt       336
Leu Glu Phe Gln Asn Asn Leu Ile Ser Gly Phe Ser Gly Ser Ile Gly
            100                 105                 110

tat gct atg gat ggg cca aga ata gaa ctt gaa gct gca tac caa aaa       384
Tyr Ala Met Asp Gly Pro Arg Ile Glu Leu Glu Ala Ala Tyr Gln Lys
        115                 120                 125

ttt gat gca aaa aat cct gac aac aat gac act aat agc ggt gac tac 432
Phe Asp Ala Lys Asn Pro Asp Asn Asn Asp Thr Asn Ser Gly Asp Tyr
    130                 135                 140

tat aaa tac ttt gga cta tct cgt gaa gac gca ata gca gat aag aaa 480
Tyr Lys Tyr Phe Gly Leu Ser Arg Glu Asp Ala Ile Ala Asp Lys Lys
145                 150                 155                 160

tat gtt gtc ctt aaa aat gaa ggc atc act ttt atg tca tta atg gtt 528
Tyr Val Val Leu Lys Asn Glu Gly Ile Thr Phe Met Ser Leu Met Val
                165                 170                 175

aac act tgc tat gac att aca gct gaa gga gta cct ttc ata ccg tat 576
Asn Thr Cys Tyr Asp Ile Thr Ala Glu Gly Val Pro Phe Ile Pro Tyr
            180                 185                 190

gca tgt gca ggt gta gga gca gac ctt ata aac gta ttt aag gat ttt 624
Ala Cys Ala Gly Val Gly Ala Asp Leu Ile Asn Val Phe Lys Asp Phe
        195                 200                 205

aat tta aaa ttc tca tac caa ggg aaa ata ggt att agc tat cca atc 672
Asn Leu Lys Phe Ser Tyr Gln Gly Lys Ile Gly Ile Ser Tyr Pro Ile
    210                 215                 220

aca cca gaa gtt tcc gct ttt att gga gga tac tac cac gga gtt ata 720
Thr Pro Glu Val Ser Ala Phe Ile Gly Gly Tyr Tyr His Gly Val Ile
225                 230                 235                 240

gga aat aat ttt aac aaa ata cct gta ata aca cct gta gta tta gaa 768
Gly Asn Asn Phe Asn Lys Ile Pro Val Ile Thr Pro Val Val Leu Glu
                245                 250                 255

gga gct cct caa aca aca tct gcg cta gta act att gac act gga tac 816
Gly Ala Pro Gln Thr Thr Ser Ala Leu Val Thr Ile Asp Thr Gly Tyr
            260                 265                 270

ttt ggc gga gaa gtt gga gta agg ttc acc ttc tag 852
Phe Gly Gly Glu Val Gly Val Arg Phe Thr Phe
        275                 280

<210> 4

<211> 283

<212> PRT

<213> Ehrlichia chaffeensis

<400> 4

Met Asn Tyr Lys Lys Ile Phe Val Ser Ser Ala Leu Ile Ser Leu Met
1               5                   10                  15

Ser Ile Leu Pro Tyr Gln Ser Phe Ala Asp Pro Val Thr Ser Asn Asp
            20                  25                  30

Thr Gly Ile Asn Asp Ser Arg Glu Gly Phe Tyr Ile Ser Val Lys Tyr
        35                  40                  45

Asn Pro Ser Ile Ser His Phe Arg Lys Phe Ser Ala Glu Glu Ala Pro
    50                  55                  60

Ile Asn Gly Asn Thr Ser Ile Thr Lys Lys Val Phe Gly Leu Lys Lys
65                  70                  75                  80

Asp Gly Asp Ile Ala Gln Ser Ala Asn Phe Asn Arg Thr Asp Pro Ala
                85                  90                  95

Leu Glu Phe Gln Asn Asn Leu Ile Ser Gly Phe Ser Gly Ser Ile Gly
            100                 105                 110

Tyr Ala Met Asp Gly Pro Arg Ile Glu Leu Glu Ala Ala Tyr Gln Lys
        115                 120                 125

Phe Asp Ala Lys Asn Pro Asp Asn Asn Asp Thr Asn Ser Gly Asp Tyr
    130                 135                 140

Tyr Lys Tyr Phe Gly Leu Ser Arg Glu Asp Ala Ile Ala Asp Lys Lys
145                 150                 155                 160

Tyr Val Val Leu Lys Asn Glu Gly Ile Thr Phe Met Ser Leu Met Val
                165                 170                 175

Asn Thr Cys Tyr Asp Ile Thr Ala Glu Gly Val Pro Phe Ile Pro Tyr
            180                 185                 190

Ala Cys Ala Gly Val Gly Ala Asp Leu Ile Asn Val Phe Lys Asp Phe
        195                 200                 205

Asn Leu Lys Phe Ser Tyr Gln Gly Lys Ile Gly Ile Ser Tyr Pro Ile
    210                 215                 220

Thr Pro Glu Val Ser Ala Phe Ile Gly Gly Tyr Tyr His Gly Val Ile
225                 230                 235                 240

Gly Asn Asn Phe Asn Lys Ile Pro Val Ile Thr Pro Val Val Leu Glu
                245                 250                 255

Gly Ala Pro Gln Thr Thr Ser Ala Leu Val Thr Ile Asp Thr Gly Tyr
            260                 265                 270

Phe Gly Gly Glu Val Gly Val Arg Phe Thr Phe
        275                 280

<210>  5

<211>  843

<212>  DNA

<213>  Ehrlichia chaffeensis


<220>

<221>  CDS

<222>  (1)..(843)

<400>  5
atg aac tgc aaa aaa ttt ttt ata aca act gca ttg gca ttg cca atg        48
Met Asn Cys Lys Lys Phe Phe Ile Thr Thr Ala Leu Ala Leu Pro Met
1               5                   10                  15

tct ttc tta cct gga ata tta ctt tct gaa cca gta caa gat gac agt        96
Ser Phe Leu Pro Gly Ile Leu Leu Ser Glu Pro Val Gln Asp Asp Ser
            20                  25                  30

gtg agt ggc aat ttc tat att agt ggc aag tac atg cca agt gct tct       144
Val Ser Gly Asn Phe Tyr Ile Ser Gly Lys Tyr Met Pro Ser Ala Ser
        35                  40                  45

cat ttt gga gtt ttc tct gcc aaa gaa gaa aaa aat cct act gtc gcg       192
His Phe Gly Val Phe Ser Ala Lys Glu Glu Lys Asn Pro Thr Val Ala
    50                  55                  60

ttg tat ggt ttg aaa caa gat tgg aac ggt gtt agt gct tca agt cat       240
Leu Tyr Gly Leu Lys Gln Asp Trp Asn Gly Val Ser Ala Ser Ser His
65                  70                  75                  80

gct gat gcg gac ttt aat aac aaa ggt tat tct ttt aaa tac gaa aac       288
Ala Asp Ala Asp Phe Asn Asn Lys Gly Tyr Ser Phe Lys Tyr Glu Asn
                85                  90                  95

aat cca ttt cta ggt ttt gca gga gct att ggt tat tca atg ggt ggt       336
Asn Pro Phe Leu Gly Phe Ala Gly Ala Ile Gly Tyr Ser Met Gly Gly
            100                 105                 110

cca aga ata gag ttt gaa gtg tcc tat gaa aca ttt gac gtg aaa aat       384
Pro Arg Ile Glu Phe Glu Val Ser Tyr Glu Thr Phe Asp Val Lys Asn
        115                 120                 125

caa ggt ggt aat tac aaa aat gat gct cac aga tac tgt gcc tta gat       432
Gln Gly Gly Asn Tyr Lys Asn Asp Ala His Arg Tyr Cys Ala Leu Asp
    130                 135                 140

cgt aaa gca agc agc act aat gcc aca gct agt cac tac gtg cta cta       480
Arg Lys Ala Ser Ser Thr Asn Ala Thr Ala Ser His Tyr Val Leu Leu
145                 150                 155                 160

aaa aat gaa gga cta ctt gat ata tca ctt atg ttg aat gca tgc tat       528
Lys Asn Glu Gly Leu Leu Asp Ile Ser Leu Met Leu Asn Ala Cys Tyr
                165                 170                 175

gac gta gta agt gaa gga ata cct tcc tct cct tac ata tgt gca ggt       576
Asp Val Val Ser Glu Gly Ile Pro Ser Ser Pro Tyr Ile Cys Ala Gly
            180                 185                 190

gtt ggt acc gat tta ata tcc atg ttt gaa gct ata aac cct aaa att       624
Val Gly Thr Asp Leu Ile Ser Met Phe Glu Ala Ile Asn Pro Lys Ile
        195                 200                 205

tct tat caa gga aag tta ggt ttg agt tac tct ata aac cca gaa gct       672
Ser Tyr Gln Gly Lys Leu Gly Leu Ser Tyr Ser Ile Asn Pro Glu Ala
    210                 215                 220

tct gtc ttt gtt ggt gga cat ttt cat aaa gtt gca ggt aat gaa ttc       720
Ser Val Phe Val Gly Gly His Phe His Lys Val Ala Gly Asn Glu Phe
225                 230                 235                 240

agg gac att tct act ctt aaa gcg ttt gct aca cca tca tct gca gct        768
Arg Asp Ile Ser Thr Leu Lys Ala Phe Ala Thr Pro Ser Ser Ala Ala
                245                 250                 255

act cca gac tta gca aca gta aca ctg agt gtg tgt cac ttt gga gta        816
Thr Pro Asp Leu Ala Thr Val Thr Leu Ser Val Cys His Phe Gly Val
            260                 265                 270

gaa ctt gga gga aga ttt aac ttc taa                                    843
Glu Leu Gly Gly Arg Phe Asn Phe
        275                 280

<210>  6

<211>  280

<212>  PRT

<213>  Ehrlichia chaffeensis

<400>  6

Met Asn Cys Lys Lys Phe Phe Ile Thr Thr Ala Leu Ala Leu Pro Met
1               5                   10                  15

Ser Phe Leu Pro Gly Ile Leu Leu Ser Glu Pro Val Gln Asp Asp Ser
            20                  25                  30

Val Ser Gly Asn Phe Tyr Ile Ser Gly Lys Tyr Met Pro Ser Ala Ser
        35                  40                  45

His Phe Gly Val Phe Ser Ala Lys Glu Glu Lys Asn Pro Thr Val Ala
    50                  55                  60

Leu Tyr Gly Leu Lys Gln Asp Trp Asn Gly Val Ser Ala Ser Ser His
65                  70                  75                  80

Ala Asp Ala Asp Phe Asn Asn Lys Gly Tyr Ser Phe Lys Tyr Glu Asn
                85                  90                  95

Asn Pro Phe Leu Gly Phe Ala Gly Ala Ile Gly Tyr Ser Met Gly Gly
            100                 105                 110

Pro Arg Ile Glu Phe Glu Val Ser Tyr Glu Thr Phe Asp Val Lys Asn
        115                 120                 125

Gln Gly Gly Asn Tyr Lys Asn Asp Ala His Arg Tyr Cys Ala Leu Asp
    130                 135                 140

Arg Lys Ala Ser Ser Thr Asn Ala Thr Ala Ser His Tyr Val Leu Leu
145                 150                 155                 160

Lys Asn Glu Gly Leu Leu Asp Ile Ser Leu Met Leu Asn Ala Cys Tyr
                165                 170                 175

Asp Val Val Ser Glu Gly Ile Pro Ser Ser Pro Tyr Ile Cys Ala Gly
            180                 185                 190

Val Gly Thr Asp Leu Ile Ser Met Phe Glu Ala Ile Asn Pro Lys Ile
        195                 200                 205

Ser Tyr Gln Gly Lys Leu Gly Leu Ser Tyr Ser Ile Asn Pro Glu Ala
    210                 215                 220

Ser Val Phe Val Gly Gly His Phe His Lys Val Ala Gly Asn Glu Phe
225                 230                 235                 240

Arg Asp Ile Ser Thr Leu Lys Ala Phe Ala Thr Pro Ser Ser Ala Ala
                245                 250                 255

Thr Pro Asp Leu Ala Thr Val Thr Leu Ser Val Cys His Phe Gly Val
            260                 265                 270

Glu Leu Gly Gly Arg Phe Asn Phe
        275                 280

<210>  7

<211>  861

<212>  DNA

<213>  Ehrlichia chaffeensis



<220>

<221>  CDS

<222>  (1)..(861)



<400>  7
atg aac tgc gaa aaa ttt ttt ata aca act gca tta aca tta cta atg        48
Met Asn Cys Glu Lys Phe Phe Ile Thr Thr Ala Leu Thr Leu Leu Met
1               5                   10                  15

tcc ttc tta cct gga ata tca ctt tct gat cca gta cag gat gac aac        96
Ser Phe Leu Pro Gly Ile Ser Leu Ser Asp Pro Val Gln Asp Asp Asn
            20                  25                  30

att agt ggt aat ttc tac atc agt gga aag tat atg cca agc gct tcg       144
Ile Ser Gly Asn Phe Tyr Ile Ser Gly Lys Tyr Met Pro Ser Ala Ser
        35                  40                  45

cat ttt gga gtt ttt tct gcc aag gaa gaa aga aat aca aca gtt gga       192
His Phe Gly Val Phe Ser Ala Lys Glu Glu Arg Asn Thr Thr Val Gly
    50                  55                  60

gta ttt gga ata gag caa gat tgg gat aga tgt gta ata tct aga acc       240
Val Phe Gly Ile Glu Gln Asp Trp Asp Arg Cys Val Ile Ser Arg Thr
65                  70                  75                  80

act tta agc cat ata ttc acc gtt cca aat tat tca ttt aag tat gaa          288
Thr Leu Ser His Ile Phe Thr Val Pro Asn Tyr Ser Phe Lys Tyr Glu
                85                  90                  95

aat aat cta ttt tca gga ttt gca gga gct att ggc tac tca atg gat          336
Asn Asn Leu Phe Ser Gly Phe Ala Gly Ala Ile Gly Tyr Ser Met Asp
            100                 105                 110

ggc cca aga ata gag ctt gaa gta tct tat gaa gca ttc gat gtt aaa          384
Gly Pro Arg Ile Glu Leu Glu Val Ser Tyr Glu Ala Phe Asp Val Lys
        115                 120                 125

aat caa ggt aac aat tat aag aac gaa gca cat aga tat tat gct ctg          432
Asn Gln Gly Asn Asn Tyr Lys Asn Glu Ala His Arg Tyr Tyr Ala Leu
    130                 135                 140

tcc cat ctt ctc ggc aca gag aca cag ata gat ggt gca ggc agt gcg          480
Ser His Leu Leu Gly Thr Glu Thr Gln Ile Asp Gly Ala Gly Ser Ala
145                 150                 155                 160

tct gtc ttt cta ata aat gaa gga cta ctt gat aaa tca ttt atg ctg          528
Ser Val Phe Leu Ile Asn Glu Gly Leu Leu Asp Lys Ser Phe Met Leu
                165                 170                 175

aac gca tgt tat gat gta ata agt gaa ggc ata cct ttt tct cct tat          576
Asn Ala Cys Tyr Asp Val Ile Ser Glu Gly Ile Pro Phe Ser Pro Tyr
            180                 185                 190

ata tgt gca ggt att ggt att gat tta gta tcc atg ttt gaa gct ata          624
Ile Cys Ala Gly Ile Gly Ile Asp Leu Val Ser Met Phe Glu Ala Ile
        195                 200                 205

aat cct aaa att tct tat caa gga aaa tta ggc tta agt tac cct ata          672
Asn Pro Lys Ile Ser Tyr Gln Gly Lys Leu Gly Leu Ser Tyr Pro Ile
    210                 215                 220

agc cca gaa gct tct gtg ttt att ggt gga cat ttt cat aag gtg ata          720
Ser Pro Glu Ala Ser Val Phe Ile Gly Gly His Phe His Lys Val Ile
225                 230                 235                 240

gga aac gaa ttt aga gat att cct act atg ata cct agt gaa tca gcg          768
Gly Asn Glu Phe Arg Asp Ile Pro Thr Met Ile Pro Ser Glu Ser Ala
                245                 250                 255

ctt gca gga aaa gga aac tac cct gca ata gta aca ctg gac gtg ttc          816
Leu Ala Gly Lys Gly Asn Tyr Pro Ala Ile Val Thr Leu Asp Val Phe
            260                 265                 270

tac ttt ggc ata gaa ctt gga gga agg ttt aac ttc caa ctt tga              861
Tyr Phe Gly Ile Glu Leu Gly Gly Arg Phe Asn Phe Gln Leu
        275                 280                 285

<210>  8

<211>  286

<212>  PRT

<213>  Ehrlichia chaffeensis

<400>  8

Met Asn Cys Glu Lys Phe Phe Ile Thr Thr Ala Leu Thr Leu Leu Met
1               5                   10                  15

Ser Phe Leu Pro Gly Ile Ser Leu Ser Asp Pro Val Gln Asp Asp Asn
            20                  25                  30

Ile Ser Gly Asn Phe Tyr Ile Ser Gly Lys Tyr Met Pro Ser Ala Ser
        35                  40                  45

His Phe Gly Val Phe Ser Ala Lys Glu Glu Arg Asn Thr Thr Val Gly
    50                  55                  60

Val Phe Gly Ile Glu Gln Asp Trp Asp Arg Cys Val Ile Ser Arg Thr
65                  70                  75                  80

Thr Leu Ser His Ile Phe Thr Val Pro Asn Tyr Ser Phe Lys Tyr Glu
                85                  90                  95

Asn Asn Leu Phe Ser Gly Phe Ala Gly Ala Ile Gly Tyr Ser Met Asp
            100                 105                 110

Gly Pro Arg Ile Glu Leu Glu Val Ser Tyr Glu Ala Phe Asp Val Lys
        115                 120                 125

Asn Gln Gly Asn Asn Tyr Lys Asn Glu Ala His Arg Tyr Tyr Ala Leu
    130                 135                 140

Ser His Leu Leu Gly Thr Glu Thr Gln Ile Asp Gly Ala Gly Ser Ala
145                 150                 155                 160

Ser Val Phe Leu Ile Asn Glu Gly Leu Leu Asp Lys Ser Phe Met Leu
                165                 170                 175

Asn Ala Cys Tyr Asp Val Ile Ser Glu Gly Ile Pro Phe Ser Pro Tyr
            180                 185                 190

Ile Cys Ala Gly Ile Gly Ile Asp Leu Val Ser Met Phe Glu Ala Ile
        195                 200                 205

Asn Pro Lys Ile Ser Tyr Gln Gly Lys Leu Gly Leu Ser Tyr Pro Ile
    210                 215                 220

Ser Pro Glu Ala Ser Val Phe Ile Gly Gly His Phe His Lys Val Ile
225                 230                 235                 240

Gly Asn Glu Phe Arg Asp Ile Pro Thr Met Ile Pro Ser Glu Ser Ala
                245                 250                 255

Leu Ala Gly Lys Gly Asn Tyr Pro Ala Ile Val Thr Leu Asp Val Phe
            260                 265                 270

Tyr Phe Gly Ile Glu Leu Gly Gly Arg Phe Asn Phe Gln Leu
        275                 280                 285

<210>  9

<211>  837

<212>  DNA

<213>  Ehrlichia chaffeensis

<220>

<221>  CDS

<222>  (1)..(837)

<400>  9
atg aat tgc aaa aaa ttt ttt ata aca act gca tta gta tca cta atg        48
Met Asn Cys Lys Lys Phe Phe Ile Thr Thr Ala Leu Val Ser Leu Met
1               5                   10                  15

tcc ttt cta cct gga ata tca ttt tct gat cca gtg caa ggt gac aat        96
Ser Phe Leu Pro Gly Ile Ser Phe Ser Asp Pro Val Gln Gly Asp Asn
            20                  25                  30

att agt ggt aat ttc tat gtt agt ggc aag tat atg cca agt gct tcg       144
Ile Ser Gly Asn Phe Tyr Val Ser Gly Lys Tyr Met Pro Ser Ala Ser
        35                  40                  45

cat ttt ggc atg ttt tct gcc aaa gaa gaa aaa aat cct act gtt gca       192
His Phe Gly Met Phe Ser Ala Lys Glu Glu Lys Asn Pro Thr Val Ala
    50                  55                  60

ttg tat ggc tta aaa caa gat tgg gaa ggg att agc tca tca agt cac       240
Leu Tyr Gly Leu Lys Gln Asp Trp Glu Gly Ile Ser Ser Ser Ser His
65                  70                  75                  80

aat gat aat cat ttc aat aac aag ggt tat tca ttt aaa tat gaa aat       288
Asn Asp Asn His Phe Asn Asn Lys Gly Tyr Ser Phe Lys Tyr Glu Asn
                85                  90                  95

aac cca ttt tta ggg ttt gca gga gct att ggt tat tca atg ggt ggt       336
Asn Pro Phe Leu Gly Phe Ala Gly Ala Ile Gly Tyr Ser Met Gly Gly
            100                 105                 110

cca aga gta gag ttt gaa gtg tcc tat gaa aca ttt gac gtt aaa aat       384
Pro Arg Val Glu Phe Glu Val Ser Tyr Glu Thr Phe Asp Val Lys Asn
        115                 120                 125

cag ggt aat aac tat aaa aat gat gct cac aga tac tgt gct tta ggt       432
Gln Gly Asn Asn Tyr Lys Asn Asp Ala His Arg Tyr Cys Ala Leu Gly
    130                 135                 140

caa caa gac aac agc gga ata cct aaa act agt aaa tac gta ctg tta       480
Gln Gln Asp Asn Ser Gly Ile Pro Lys Thr Ser Lys Tyr Val Leu Leu
145                 150                 155                 160

aaa agc gaa gga ttg ctt gac ata tca ttt atg cta aat gca tgc tat       528
Lys Ser Glu Gly Leu Leu Asp Ile Ser Phe Met Leu Asn Ala Cys Tyr
                165                 170                 175

gat ata ata aac gag agc ata cct ttg tct cct tac ata tgt gca ggt       576
Asp Ile Ile Asn Glu Ser Ile Pro Leu Ser Pro Tyr Ile Cys Ala Gly
            180                 185                 190

gtt ggt act gat tta ata tcc atg ttt gaa gct aca aat cct aaa att        624
Val Gly Thr Asp Leu Ile Ser Met Phe Glu Ala Thr Asn Pro Lys Ile
        195                 200                 205

tct tac caa ggg aag tta ggt cta agt tac tct ata aac cca gaa gct        672
Ser Tyr Gln Gly Lys Leu Gly Leu Ser Tyr Ser Ile Asn Pro Glu Ala
    210                 215                 220

tct gta ttt att ggt gga cat ttt cat aag gtg ata gga aac gaa ttt        720
Ser Val Phe Ile Gly Gly His Phe His Lys Val Ile Gly Asn Glu Phe
225                 230                 235                 240

agg gac att cct act ctg aaa gca ttt gtt acg tca tca gct act cca        768
Arg Asp Ile Pro Thr Leu Lys Ala Phe Val Thr Ser Ser Ala Thr Pro
                245                 250                 255

gat cta gca ata gta aca cta agt gta tgt cat ttt gga ata gaa ctt        816
Asp Leu Ala Ile Val Thr Leu Ser Val Cys His Phe Gly Ile Glu Leu
            260                 265                 270

gga gga agg ttt aac ttc taa                                            837
Gly Gly Arg Phe Asn Phe
        275

<210>  10

<211>  278

<212>  PRT

<213>  Ehrlichia chaffeensis

<400>  10

Met Asn Cys Lys Lys Phe Phe Ile Thr Thr Ala Leu Val Ser Leu Met
1               5                   10                  15

Ser Phe Leu Pro Gly Ile Ser Phe Ser Asp Pro Val Gln Gly Asp Asn
            20                  25                  30

Ile Ser Gly Asn Phe Tyr Val Ser Gly Lys Tyr Met Pro Ser Ala Ser
        35                  40                  45

His Phe Gly Met Phe Ser Ala Lys Glu Glu Lys Asn Pro Thr Val Ala
    50                  55                  60

Leu Tyr Gly Leu Lys Gln Asp Trp Glu Gly Ile Ser Ser Ser Ser His
65                  70                  75                  80

Asn Asp Asn His Phe Asn Asn Lys Gly Tyr Ser Phe Lys Tyr Glu Asn
                85                  90                  95

Asn Pro Phe Leu Gly Phe Ala Gly Ala Ile Gly Tyr Ser Met Gly Gly
            100                 105                 110

Pro Arg Val Glu Phe Glu Val Ser Tyr Glu Thr Phe Asp Val Lys Asn
        115                 120                 125

Gln Gly Asn Asn Tyr Lys Asn Asp Ala His Arg Tyr Cys Ala Leu Gly
    130                 135                 140

Gln Gln Asp Asn Ser Gly Ile Pro Lys Thr Ser Lys Tyr Val Leu Leu
145                 150                 155                 160

Lys Ser Glu Gly Leu Leu Asp Ile Ser Phe Met Leu Asn Ala Cys Tyr
                165                 170                 175

Asp Ile Ile Asn Glu Ser Ile Pro Leu Ser Pro Tyr Ile Cys Ala Gly
            180                 185                 190

Val Gly Thr Asp Leu Ile Ser Met Phe Glu Ala Thr Asn Pro Lys Ile
        195                 200                 205

Ser Tyr Gln Gly Lys Leu Gly Leu Ser Tyr Ser Ile Asn Pro Glu Ala
    210                 215                 220

Ser Val Phe Ile Gly Gly His Phe His Lys Val Ile Gly Asn Glu Phe
225                 230                 235                 240

Arg Asp Ile Pro Thr Leu Lys Ala Phe Val Thr Ser Ser Ala Thr Pro
                245                 250                 255

Asp Leu Ala Ile Val Thr Leu Ser Val Cys His Phe Gly Ile Glu Leu
            260                 265                 270

Gly Gly Arg Phe Asn Phe
        275

<210>  11

<211>  843

<212>  DNA

<213>  Ehrlichia chaffeensis

<220>

<221>  CDS

<222>  (1)..(843)

<400>  11
atg aat tgc aaa aaa ttt ttt ata aca act aca tta gta tcg cta atg        48
Met Asn Cys Lys Lys Phe Phe Ile Thr Thr Thr Leu Val Ser Leu Met
1               5                   10                  15

tcc ttc tta cct gga ata tca ttt tct gat gca gta cag aac gac aat        96
Ser Phe Leu Pro Gly Ile Ser Phe Ser Asp Ala Val Gln Asn Asp Asn
            20                  25                  30

gtt ggt ggt aat ttc tat atc agt ggg aaa tat gta cca agt gtt tca          144
Val Gly Gly Asn Phe Tyr Ile Ser Gly Lys Tyr Val Pro Ser Val Ser
        35                  40                  45

cat ttt ggc gta ttc tct gct aaa cag gaa aga aat aca aca acc gga          192
His Phe Gly Val Phe Ser Ala Lys Gln Glu Arg Asn Thr Thr Thr Gly
    50                  55                  60

gta ttt gga tta aag caa gat tgg gat ggc agc aca ata tct aaa aat          240
Val Phe Gly Leu Lys Gln Asp Trp Asp Gly Ser Thr Ile Ser Lys Asn
65                  70                  75                  80

tct cca gaa aat aca ttt aac gtt cca aat tat tca ttt aaa tat gaa          288
Ser Pro Glu Asn Thr Phe Asn Val Pro Asn Tyr Ser Phe Lys Tyr Glu
                85                  90                  95

aat aat cca ttt cta ggt ttt gca gga gct gtt ggt tat tta atg aat          336
Asn Asn Pro Phe Leu Gly Phe Ala Gly Ala Val Gly Tyr Leu Met Asn
            100                 105                 110

ggt cca aga ata gag tta gaa atg tcc tat gaa aca ttt gat gtg aaa          384
Gly Pro Arg Ile Glu Leu Glu Met Ser Tyr Glu Thr Phe Asp Val Lys
        115                 120                 125

aac cag ggt aat aac tat aag aac gat gct cac aaa tat tat gct tta          432
Asn Gln Gly Asn Asn Tyr Lys Asn Asp Ala His Lys Tyr Tyr Ala Leu
    130                 135                 140

acc cat aac agt ggg gga aag cta agc aat gca ggt gat aag ttt gtt          480
Thr His Asn Ser Gly Gly Lys Leu Ser Asn Ala Gly Asp Lys Phe Val
145                 150                 155                 160

ttt cta aaa aat gaa gga cta ctt gat ata tca ctt atg ttg aat gca          528
Phe Leu Lys Asn Glu Gly Leu Leu Asp Ile Ser Leu Met Leu Asn Ala
                165                 170                 175

tgc tat gat gta ata agt gaa gga ata cct ttc tct cct tac ata tgt          576
Cys Tyr Asp Val Ile Ser Glu Gly Ile Pro Phe Ser Pro Tyr Ile Cys
            180                 185                 190

gca ggt gtt ggt act gat tta ata tcc atg ttt gaa gct ata aac cct          624
Ala Gly Val Gly Thr Asp Leu Ile Ser Met Phe Glu Ala Ile Asn Pro
        195                 200                 205

aaa att tct tat caa gga aag tta ggt ttg agt tac tcc ata agc cca          672
Lys Ile Ser Tyr Gln Gly Lys Leu Gly Leu Ser Tyr Ser Ile Ser Pro
    210                 215                 220

gaa gct tct gtt ttt gtt ggt gga cat ttt cat aag gtg ata ggg aat          720
Glu Ala Ser Val Phe Val Gly Gly His Phe His Lys Val Ile Gly Asn
225                 230                 235                 240

gaa ttc aga gat att cct gct atg ata ccc agt acc tca act ctc aca          768
Glu Phe Arg Asp Ile Pro Ala Met Ile Pro Ser Thr Ser Thr Leu Thr
                245                 250                 255

ggt aat cac ttt act ata gta aca cta agt gta tgc cac ttt gga gtg          816
Gly Asn His Phe Thr Ile Val Thr Leu Ser Val Cys His Phe Gly Val
            260                 265                 270

gaa ctt gga gga agg ttt aac ttt taa                                      843
Glu Leu Gly Gly Arg Phe Asn Phe
        275                 280

<210>  12

<211>  280

<212>  PRT

<213>  Ehrlichia chaffeensis

<400>  12

Met Asn Cys Lys Lys Phe Phe Ile Thr Thr Thr Leu Val Ser Leu Met
1               5                   10                  15

Ser Phe Leu Pro Gly Ile Ser Phe Ser Asp Ala Val Gln Asn Asp Asn
            20                  25                  30

Val Gly Gly Asn Phe Tyr Ile Ser Gly Lys Tyr Val Pro Ser Val Ser
        35                  40                  45

His Phe Gly Val Phe Ser Ala Lys Gln Glu Arg Asn Thr Thr Thr Gly
    50                  55                  60

Val Phe Gly Leu Lys Gln Asp Trp Asp Gly Ser Thr Ile Ser Lys Asn
65                  70                  75                  80

Ser Pro Glu Asn Thr Phe Asn Val Pro Asn Tyr Ser Phe Lys Tyr Glu
                85                  90                  95

Asn Asn Pro Phe Leu Gly Phe Ala Gly Ala Val Gly Tyr Leu Met Asn
            100                 105                 110

Gly Pro Arg Ile Glu Leu Glu Met Ser Tyr Glu Thr Phe Asp Val Lys
        115                 120                 125

Asn Gln Gly Asn Asn Tyr Lys Asn Asp Ala His Lys Tyr Tyr Ala Leu
    130                 135                 140

Thr His Asn Ser Gly Gly Lys Leu Ser Asn Ala Gly Asp Lys Phe Val
145                 150                 155                 160

Phe Leu Lys Asn Glu Gly Leu Leu Asp Ile Ser Leu Met Leu Asn Ala
                165                 170                 175

Cys Tyr Asp Val Ile Ser Glu Gly Ile Pro Phe Ser Pro Tyr Ile Cys
            180                 185                 190

Ala Gly Val Gly Thr Asp Leu Ile Ser Met Phe Glu Ala Ile Asn Pro
        195                 200                 205

Lys Ile Ser Tyr Gln Gly Lys Leu Gly Leu Ser Tyr Ser Ile Ser Pro
    210                 215                 220

Glu Ala Ser Val Phe Val Gly Gly His Phe His Lys Val Ile Gly Asn
225                 230                 235                 240

Glu Phe Arg Asp Ile Pro Ala Met Ile Pro Ser Thr Ser Thr Leu Thr
                245                 250                 255

Gly Asn His Phe Thr Ile Val Thr Leu Ser Val Cys His Phe Gly Val
            260                 265                 270

Glu Leu Gly Gly Arg Phe Asn Phe
        275                 280

<210>  13

<211>  894

<212>  DNA

<213>  Ehrlichia chaffeensis


<400>  13
atggaaaatc tcatgaataa gaaaaacaaa ttctttacaa taagtacagc aatggtatgc      60

ttattgttat tacctggtat atcattttca gaaactataa acaacagtgc taaaaaacag     120

cctgggttat atatcagtgg gcagtacaaa cctagtgttt cagtttttag taatttttca     180

gtaaaagaaa ctaatgttcc cacaaagcag ttaatagcac ttaaaaaaga cattaattct     240

gttgcagttg gtagtaatgc tactacaggt attagcaatc caggtaattt cacaattcct     300

tatactgcag aatttcaaga taatgttgcc aatttcaatg gggctgttgg ttactctttt     360

cctgatagtc taagaattga aatagaggga tttcatgaaa aatttgatgt caaaaaccct     420

ggaggttaca cacaagtaaa agatgcgtac cgttattttg cactagcacg tgatttaaaa     480

gatggcttct ttgaacctaa agcggaagat acaggtgttt atcatactgt tatgaaaaat     540

gatggattat ctattttatc tactatggtt aacgtctgtt acgatttttc tgtagatgaa     600

ttaccagtct taccttatat atgtgcaggt atgggtataa acgccataga attcttcgac     660

gctttacatg taaaatttgc ttaccaaggc aaactaggta ttagctatca actatttact     720

aaagtaaatt tattccttga tgggtattac catcaagtaa taggcaatca atccaaaaac     780

ttaaacgtaa accatgttta cacacttaaa gaatctccta aagtcacatc tgcagtagct     840

acacttgaca ttgcatactt tggtgccgaa gttggaataa gattcacatt ttaa           894

<210>  14

<211>  280

<212>  PRT

<213>  Ehrlichia chaffeensis


<400>  14

Met Val Cys Leu Leu Leu Leu Pro Gly Ile Ser Phe Ser Glu Thr Ile

1               5                   10                  15

Asn Asn Ser Ala Lys Lys Gln Pro Gly Leu Tyr Ile Ser Gly Gln Tyr
            20                  25                  30

Lys Pro Ser Val Ser Val Phe Ser Asn Phe Ser Val Lys Glu Thr Asn
        35                  40                  45

Val Pro Thr Lys Gln Leu Ile Ala Leu Lys Lys Asp Ile Asn Ser Val
    50                  55                  60

Ala Val Gly Ser Asn Ala Thr Thr Gly Ile Ser Asn Pro Gly Asn Phe
65                  70                  75                  80

Thr Ile Pro Tyr Thr Ala Glu Phe Gln Asp Asn Val Ala Asn Phe Asn
                85                  90                  95

Gly Ala Val Gly Tyr Ser Phe Pro Asp Ser Leu Arg Ile Glu Ile Glu
            100                 105                 110

Gly Phe His Glu Lys Phe Asp Val Lys Asn Pro Gly Gly Tyr Thr Gln
        115                 120                 125

Val Lys Asp Ala Tyr Arg Tyr Phe Ala Leu Ala Arg Asp Leu Lys Asp
    130                 135                 140

Gly Phe Phe Glu Pro Lys Ala Glu Asp Thr Gly Val Tyr His Thr Val
145                 150                 155                 160

Met Lys Asn Asp Gly Leu Ser Ile Leu Ser Thr Met Val Asn Val Cys
                165                 170                 175

Tyr Asp Phe Ser Val Asp Glu Leu Pro Val Leu Pro Tyr Ile Cys Ala
            180                 185                 190

Gly Met Gly Ile Asn Ala Ile Glu Phe Phe Asp Ala Leu His Val Lys
        195                 200                 205

Phe Ala Tyr Gln Gly Lys Leu Gly Ile Ser Tyr Gln Leu Phe Thr Lys
    210                 215                 220

Val Asn Leu Phe Leu Asp Gly Tyr Tyr His Gln Val Ile Gly Asn Gln
225                 230                 235                 240

Phe Lys Asn Leu Asn Val Asn His Val Tyr Thr Leu Lys Glu Ser Pro
                245                 250                 255

Lys Val Thr Ser Ala Val Ala Thr Leu Asp Ile Ala Tyr Phe Gly Gly
            260                 265                 270

Glu Val Gly Ile Arg Phe Thr Phe
        275                 280

<210>  15

<211>  591

<212>  DNA

<213>  Ehrlichia chaffeensis

<220>

<221>  CDS

<222>  (1)..(591)


<400>  15
atg ata tat aaa gaa aaa ctt act aga gtg gga gaa tat atc tta gca      48
Met Ile Tyr Lys Glu Lys Leu Thr Arg Val Gly Glu Tyr Ile Leu Ala
1               5                   10                  15

tat tta tca ttt att ctt tct act tat atc ttt cta gtg ctg gta aat      96
Tyr Leu Ser Phe Ile Leu Ser Thr Tyr Ile Phe Leu Val Leu Val Asn
            20                  25                  30

att att aga tat aac agc ctt gct ata tgt gtt atc agt cta cta aga      144
Ile Ile Arg Tyr Asn Ser Leu Ala Ile Cys Val Ile Ser Leu Leu Arg
        35                  40                  45

act aat atc ttt aac gtt agc aca aaa aaa tta ata aaa gat aaa tgt      192
Thr Asn Ile Phe Asn Val Ser Thr Lys Lys Leu Ile Lys Asp Lys Cys
    50                  55                  60

cgt gat act aag ttt agt aac atg aaa tgt tat ttg tac ggt aaa ccg      240
Arg Asp Thr Lys Phe Ser Asn Met Lys Cys Tyr Leu Tyr Gly Lys Pro
65                  70                  75                  80

tta aat tta caa att ttt tat gga ata ttt tcc ttt att aga aac ttt      288
Leu Asn Leu Gln Ile Phe Tyr Gly Ile Phe Ser Phe Ile Arg Asn Phe
                85                  90                  95

caa aat aac aca cta ata att cct aat gat agt aaa tgc ggc ttc tat      336
Gln Asn Asn Thr Leu Ile Ile Pro Asn Asp Ser Lys Cys Gly Phe Tyr
            100                 105                 110

acc acg tta tgg gat aat cca gca cta cat tat aca tat aca ctt act      384
Thr Thr Leu Trp Asp Asn Pro Ala Leu His Tyr Thr Tyr Thr Leu Thr
        115                 120                 125

ggc agt gag tac cgt aat ttt ttt gac att cta tat gaa aac att atc      432
Gly Ser Glu Tyr Arg Asn Phe Phe Asp Ile Leu Tyr Glu Asn Ile Ile
    130                 135                 140

tgt caa tgt aaa tta ctt att aac tat aac cgt tct gta tta aac caa      480
Cys Gln Cys Lys Leu Leu Ile Asn Tyr Asn Arg Ser Val Leu Asn Gln
145                 150                 155                 160

cat aat aaa aat act ctc gta ata ata cca ata cct aat gct aga gag      528
His Asn Lys Asn Thr Leu Val Ile Ile Pro Ile Pro Asn Ala Arg Glu
                165                 170                 175

ttc agt aat gaa att cga gta agg aat ata tca ata aat aag gaa agt      576
Phe Ser Asn Glu Ile Arg Val Arg Asn Ile Ser Ile Asn Lys Glu Ser
            180                 185                 190

tct tat gag tgc taa                                                  591
Ser Tyr Glu Cys
        195


<210>  16

<211>  196

<212>  PRT

<213>  Ehrlichia chaffeensis

<400>  16

Met Ile Tyr Lys Glu Lys Leu Thr Arg Val Gly Glu Tyr Ile Leu Ala
1               5                   10                  15

Tyr Leu Ser Phe Ile Leu Ser Thr Tyr Ile Phe Leu Val Leu Val Asn
            20                  25                  30

Ile Ile Arg Tyr Asn Ser Leu Ala Ile Cys Val Ile Ser Leu Leu Arg
        35                  40                  45

Thr Asn Ile Phe Asn Val Ser Thr Lys Lys Leu Ile Lys Asp Lys Cys
    50                  55                  60

Arg Asp Thr Lys Phe Ser Asn Met Lys Cys Tyr Leu Tyr Gly Lys Pro
65                  70                  75                  80

Leu Asn Leu Gln Ile Phe Tyr Gly Ile Phe Ser Phe Ile Arg Asn Phe
                85                  90                  95

Gln Asn Asn Thr Leu Ile Ile Pro Asn Asp Ser Lys Cys Gly Phe Tyr
            100                 105                 110

Thr Thr Leu Trp Asp Asn Pro Ala Leu His Tyr Thr Tyr Thr Leu Thr
        115                 120                 125

Gly Ser Glu Tyr Arg Asn Phe Phe Asp Ile Leu Tyr Glu Asn Ile Ile
    130                 135                 140

Cys Gln Cys Lys Leu Leu Ile Asn Tyr Asn Arg Ser Val Leu Asn Gln
145                 150                 155                 160

His Asn Lys Asn Thr Leu Val Ile Ile Pro Ile Pro Asn Ala Arg Glu
                165                 170                 175

Phe Ser Asn Glu Ile Arg Val Arg Asn Ile Ser Ile Asn Lys Glu Ser
            180                 185                 190

Ser Tyr Glu Cys
        195

<210>  17

<211>  493

<212>  DNA

<213>  Ehrlichia chaffeensis

<220>

<221>  CDS

<222>  (1)..(492)

<400>  17
atg aat aaa aaa aac aag ttt att ata gct aca gca ttg gta tat tta        48
Met Asn Lys Lys Asn Lys Phe Ile Ile Ala Thr Ala Leu Val Tyr Leu
1               5                   10                  15

ctg tca tta cct agt gta tcg ttt tca gag gtt aca aac agc agt att        96
Leu Ser Leu Pro Ser Val Ser Phe Ser Glu Val Thr Asn Ser Ser Ile
            20                  25                  30

aaa aaa cac tct ggg tta tat att agt gga caa tac aaa cca agt gtt       144
Lys Lys His Ser Gly Leu Tyr Ile Ser Gly Gln Tyr Lys Pro Ser Val
        35                  40                  45

tct gtt ttt agt agt ttc tca att aaa gaa act aac act atc aca aaa       192
Ser Val Phe Ser Ser Phe Ser Ile Lys Glu Thr Asn Thr Ile Thr Lys
    50                  55                  60

aat ctt ata gcg tta aaa aaa gat att aac tct ctt gaa gtt aac gcc       240
Asn Leu Ile Ala Leu Lys Lys Asp Ile Asn Ser Leu Glu Val Asn Ala
65                  70                  75                  80

gat gct agt caa ggt att agt cat cca gga aat ttt act ata cct tat       288
Asp Ala Ser Gln Gly Ile Ser His Pro Gly Asn Phe Thr Ile Pro Tyr
                85                  90                  95

ata gca gca ttt gaa gat aat gct ttt aat ttc aac ggt gct att ggt       336
Ile Ala Ala Phe Glu Asp Asn Ala Phe Asn Phe Asn Gly Ala Ile Gly
            100                 105                 110

tac att act gaa ggt cta agg att gaa ata gaa ggt tcc tat gaa gaa       384
Tyr Ile Thr Glu Gly Leu Arg Ile Glu Ile Glu Gly Ser Tyr Glu Glu
        115                 120                 125

ttt gat gct aaa aac cct gga ggt tat ggt cta aat gat gcc ttt cgg       432
Phe Asp Ala Lys Asn Pro Gly Gly Tyr Gly Leu Asn Asp Ala Phe Arg
    130                 135                 140

tac ttt gct tta gca cgt gat atg gaa agc aac aag ttc caa cca aaa       480
Tyr Phe Ala Leu Ala Arg Asp Met Glu Ser Asn Lys Phe Gln Pro Lys
145                 150                 155                 160

gca caa agc tca c                                                     493
Ala Gln Ser Ser

<210>  18

<211>  164

<212>  PRT

<213>  Ehrlichia chaffeensis

<400>  18

Met Asn Lys Lys Asn Lys Phe Ile Ile Ala Thr Ala Leu Val Tyr Leu
1               5                   10                  15

Leu Ser Leu Pro Ser Val Ser Phe Ser Glu Val Thr Asn Ser Ser Ile
            20                  25                  30

Lys Lys His Ser Gly Leu Tyr Ile Ser Gly Gln Tyr Lys Pro Ser Val
        35                  40                  45

Ser Val Phe Ser Ser Phe Ser Ile Lys Glu Thr Asn Thr Ile Thr Lys
    50                  55                  60

Asn Leu Ile Ala Leu Lys Lys Asp Ile Asn Ser Leu Glu Val Asn Ala
65                  70                  75                  80

Asp Ala Ser Gln Gly Ile Ser His Pro Gly Asn Phe Thr Ile Pro Tyr
                85                  90                  95

Ile Ala Ala Phe Glu Asp Asn Ala Phe Asn Phe Asn Gly Ala Ile Gly
            100                 105                 110

Tyr Ile Thr Glu Gly Leu Arg Ile Glu Ile Glu Gly Ser Tyr Glu Glu
        115                 120                 125

Phe Asp Ala Lys Asn Pro Gly Gly Tyr Gly Leu Asn Asp Ala Phe Arg
    130                 135                 140

Tyr Phe Ala Leu Ala Arg Asp Met Glu Ser Asn Lys Phe Gln Pro Lys
145                 150                 155                 160

Ala Gln Ser Ser

<210>  19

<211>  396

<212>  DNA

<213>  Ehrlichia chaffeensis

<220>

<221>  CDS

<222>  (1)..(396)

<400>  19
tct aga ata cat gat gaa aat tat gct att aca aca aat aat aaa tta      48
Ser Arg Ile His Asp Glu Asn Tyr Ala Ile Thr Thr Asn Asn Lys Leu
1               5                   10                  15

tcc atc gca tct att atg gtt aac acc tgc tat gat att tca att aat      96
Ser Ile Ala Ser Ile Met Val Asn Thr Cys Tyr Asp Ile Ser Ile Asn
            20                  25                  30

aat aca tca ata gta ccg tat tta tgc aca ggc att ggt gaa gat ctt      144

Asn Thr Ser Ile Val Pro Tyr Leu Cys Thr Gly Ile Gly Glu Asp Leu
        35                  40                  45

gta ggg ctt ttt aat aca ata cat ttt aaa ctt gca tat caa ggg aaa        192
Val Gly Leu Phe Asn Thr Ile His Phe Lys Leu Ala Tyr Gln Gly Lys
    50                  55                  60

gtt gga atg agt tat ttg ata aat aac aat atc cta tta ttt tct gac        240
Val Gly Met Ser Tyr Leu Ile Asn Asn Asn Ile Leu Leu Phe Ser Asp
65                  70                  75                  80

ata tat tat cat aaa gtc atg ggt aac aga ttt aaa aat ttg tac atg        288
Ile Tyr Tyr His Lys Val Met Gly Asn Arg Phe Lys Asn Leu Tyr Met
                85                  90                  95

caa tat gta gct gat cct aat att tct gaa gaa act ata cct ata tta        336
Gln Tyr Val Ala Asp Pro Asn Ile Ser Glu Glu Thr Ile Pro Ile Leu
            100                 105                 110

gca aaa ctt gat att ggt tat ttt gga agt gaa att gga ata agg ttt        384
Ala Lys Leu Asp Ile Gly Tyr Phe Gly Ser Glu Ile Gly Ile Arg Phe
        115                 120                 125

atg ttt aac taa                                                        396
Met Phe Asn
    130

<210>  20

<211>  131

<212>  PRT

<213>  Ehrlichia chaffeensis

<400>  20

Ser Arg Ile His Asp Glu Asn Tyr Ala Ile Thr Thr Asn Asn Lys Leu
1               5                   10                  15

Ser Ile Ala Ser Ile Met Val Asn Thr Cys Tyr Asp Ile Ser Ile Asn
            20                  25                  30

Asn Thr Ser Ile Val Pro Tyr Leu Cys Thr Gly Ile Gly Glu Asp Leu
        35                  40                  45

Val Gly Leu Phe Asn Thr Ile His Phe Lys Leu Ala Tyr Gln Gly Lys
    50                  55                  60

Val Gly Met Ser Tyr Leu Ile Asn Asn Asn Ile Leu Leu Phe Ser Asp
65                  70                  75                  80

Ile Tyr Tyr His Lys Val Met Gly Asn Arg Phe Lys Asn Leu Tyr Met
                85                  90                  95

Gln Tyr Val Ala Asp Pro Asn Ile Ser Glu Glu Thr Ile Pro Ile Leu
            100                 105                 110

Ala Lys Leu Asp Ile Gly Tyr Phe Gly Ser Glu Ile Gly Ile Arg Phe
        115                 120                 125

Met Phe Asn
    130

<210>  21

<211>  888

<212>  DNA

<213>  Ehrlichia chaffeensis

<220>

<221>  CDS

<222>  (1)..(888)

<400>  21
atg aca aag aaa ttt aat ttt gta aat gtt ata tta aca ttt ttg tta       48
Met Thr Lys Lys Phe Asn Phe Val Asn Val Ile Leu Thr Phe Leu Leu
1               5                   10                  15

ttt ctt ttc cca ctt aag tca ttt aca aca tat gca aat aat aac aca       96
Phe Leu Phe Pro Leu Lys Ser Phe Thr Thr Tyr Ala Asn Asn Asn Thr
            20                  25                  30

atc act caa aaa gtt gga ttg tac ata agt ggt caa tat aag cca agt      144
Ile Thr Gln Lys Val Gly Leu Tyr Ile Ser Gly Gln Tyr Lys Pro Ser
        35                  40                  45

att cct cat ttc aag aat ttt tca gta gaa gaa aat gac aaa gta gta      192
Ile Pro His Phe Lys Asn Phe Ser Val Glu Glu Asn Asp Lys Val Val
    50                  55                  60

gat ttg ata ggt ctt aca act gat gtt aca tat atc aca gaa cat ata      240
Asp Leu Ile Gly Leu Thr Thr Asp Val Thr Tyr Ile Thr Glu His Ile
65                  70                  75                  80

tta cga gat aat aca aaa ttc aac act cat tat att gca aag ttc aag      288
Leu Arg Asp Asn Thr Lys Phe Asn Thr His Tyr Ile Ala Lys Phe Lys
                85                  90                  95

aac aat ttt ata aat ttc agc agt gca att ggt tat tat tct ggg caa      336
Asn Asn Phe Ile Asn Phe Ser Ser Ala Ile Gly Tyr Tyr Ser Gly Gln
            100                 105                 110

gaa cca agg tta gaa ata gaa agc tct tat ggg gat ttt gat gtt gta      384
Glu Pro Arg Leu Glu Ile Glu Ser Ser Tyr Gly Asp Phe Asp Val Val
        115                 120                 125

aat tat aaa aat tat gca gta caa gat gtt aat aga tat ttt gct tta      432
Asn Tyr Lys Asn Tyr Ala Val Gln Asp Val Asn Arg Tyr Phe Ala Leu
    130                 135                 140

gta cgt gaa aaa aat ggt tca aat ttc tct cca aaa cca cat gaa act      480
Val Arg Glu Lys Asn Gly Ser Asn Phe Ser Pro Lys Pro His Glu Thr
145                 150                 155                 160

agt caa ccc tct gac agt aat cct aaa aag tct ttt tat act tta atg      528

Ser Gln Pro Ser Asp Ser Asn Pro Lys Lys Ser Phe Tyr Thr Leu Met
                165                 170                 175

aag aat aat ggg gta ttt gtt gca tca gta ata atc aac ggt tgt tat      576
Lys Asn Asn Gly Val Phe Val Ala Ser Val Ile Ile Asn Gly Cys Tyr
            180                 185                 190

gat ttt tct ttt aat aac aca aca ata tca cct tac gta tgt ata gga      624
Asp Phe Ser Phe Asn Asn Thr Thr Ile Ser Pro Tyr Val Cys Ile Gly
        195                 200                 205

gtt gga gga gat ttt ata gag ttt ttt gaa gta atg cat atc aag ttt      672
Val Gly Gly Asp Phe Ile Glu Phe Phe Glu Val Met His Ile Lys Phe
    210                 215                 220

gct tgc caa agt aag gtt ggt att agc tat cca ata tct ccc tct att      720
Ala Cys Gln Ser Lys Val Gly Ile Ser Tyr Pro Ile Ser Pro Ser Ile
225                 230                 235                 240

act att ttt gct gat gca cat tat cac aag gtc ata aat aat aaa ttt      768
Thr Ile Phe Ala Asp Ala His Tyr His Lys Val Ile Asn Asn Lys Phe
                245                 250                 255

aac aac cta cat gtt aag tat tca tat gaa ctt aaa aac tca cct acc      816
Asn Asn Leu His Val Lys Tyr Ser Tyr Glu Leu Lys Asn Ser Pro Thr
            260                 265                 270

att acc tct gca aca gcc aaa cta aac att gaa tat ttt ggt ggt gaa      864
Ile Thr Ser Ala Thr Ala Lys Leu Asn Ile Glu Tyr Phe Gly Gly Glu
        275                 280                 285

gtt ggg atg aga ttt ata ttt taa                                      888
Val Gly Met Arg Phe Ile Phe
    290                 295

<210>  22

<211>  295

<212>  PRT

<213>  Ehrlichia chaffeensis

<400>  22

Met Thr Lys Lys Phe Asn Phe Val Asn Val Ile Leu Thr Phe Leu Leu
1               5                   10                  15

Phe Leu Phe Pro Leu Lys Ser Phe Thr Thr Tyr Ala Asn Asn Asn Thr
            20                  25                  30

Ile Thr Gln Lys Val Gly Leu Tyr Ile Ser Gly Gln Tyr Lys Pro Ser
        35                  40                  45

Ile Pro His Phe Lys Asn Phe Ser Val Glu Glu Asn Asp Lys Val Val
    50                  55                  60

Asp Leu Ile Gly Leu Thr Thr Asp Val Thr Tyr Ile Thr Glu His Ile
65                  70                  75                  80

Leu Arg Asp Asn Thr Lys Phe Asn Thr His Tyr Ile Ala Lys Phe Lys
                85                  90                  95

Asn Asn Phe Ile Asn Phe Ser Ser Ala Ile Gly Tyr Tyr Ser Gly Gln
            100                 105                 110

Glu Pro Arg Leu Glu Ile Glu Ser Ser Tyr Gly Asp Phe Asp Val Val
        115                 120                 125

Asn Tyr Lys Asn Tyr Ala Val Gln Asp Val Asn Arg Tyr Phe Ala Leu
    130                 135                 140

Val Arg Glu Lys Asn Gly Ser Asn Phe Ser Pro Lys Pro His Glu Thr
145                 150                 155                 160

Ser Gln Pro Ser Asp Ser Asn Pro Lys Lys Ser Phe Tyr Thr Leu Met
                165                 170                 175

Lys Asn Asn Gly Val Phe Val Ala Ser Val Ile Ile Asn Gly Cys Tyr
            180                 185                 190

Asp Phe Ser Phe Asn Asn Thr Thr Ile Ser Pro Tyr Val Cys Ile Gly
        195                 200                 205

Val Gly Gly Asp Phe Ile Glu Phe Phe Glu Val Met His Ile Lys Phe
    210                 215                 220

Ala Cys Gln Ser Lys Val Gly Ile Ser Tyr Pro Ile Ser Pro Ser Ile
225                 230                 235                 240

Thr Ile Phe Ala Asp Ala His Tyr His Lys Val Ile Asn Asn Lys Phe
                245                 250                 255

Asn Asn Leu His Val Lys Tyr Ser Tyr Glu Leu Lys Asn Ser Pro Thr
            260                 265                 270

Ile Thr Ser Ala Thr Ala Lys Leu Asn Ile Glu Tyr Phe Gly Gly Glu
        275                 280                 285

Val Gly Met Arg Phe Ile Phe
    290                 295

<210>  23

<211>  840

<212>  DNA

<213>  Ehrlichia chaffeensis


<220>

<221>  CDS

<222>  (1)..(840)


<400>  23
atg agc aaa aaa aag ttt att aca ata gga aca gta ctt gca tct cta      48
Met Ser Lys Lys Lys Phe Ile Thr Ile Gly Thr Val Leu Ala Ser Leu
1               5                   10                  15

tta tca ttc tta tct att gaa tcc ttt tca gct ata aat cat aat cat      96
Leu Ser Phe Leu Ser Ile Glu Ser Phe Ser Ala Ile Asn His Asn His
            20                  25                  30

aca gga aat aac act agt ggt ata tat att aca ggg cag tat aga cca      144
Thr Gly Asn Asn Thr Ser Gly Ile Tyr Ile Thr Gly Gln Tyr Arg Pro
        35                  40                  45

gga gta tcc cat ttt agc aat ttc tca gta aaa gaa act aat gtt gat      192
Gly Val Ser His Phe Ser Asn Phe Ser Val Lys Glu Thr Asn Val Asp
    50                  55                  60

aca ata caa cta gta gga tat aaa aaa agt gcg tct tct atc gat cct      240
Thr Ile Gln Leu Val Gly Tyr Lys Lys Ser Ala Ser Ser Ile Asp Pro
65                  70                  75                  80

aac act tat tca aac ttt caa ggt cca tat act gtt aca ttt caa gat      288
Asn Thr Tyr Ser Asn Phe Gln Gly Pro Tyr Thr Val Thr Phe Gln Asp
                85                  90                  95

aat gct gct agt ttc agt gga gca att gga tat tct tac ccc gaa agt      336
Asn Ala Ala Ser Phe Ser Gly Ala Ile Gly Tyr Ser Tyr Pro Glu Ser
            100                 105                 110

cta aga ctt gaa ctt gaa ggt tct tac gaa aaa ttt gat gtc aaa gat      384
Leu Arg Leu Glu Leu Glu Gly Ser Tyr Glu Lys Phe Asp Val Lys Asp
        115                 120                 125

cct aaa gac tac tca gca aaa gat gct ttt agg ttt ttt gct cta gca      432
Pro Lys Asp Tyr Ser Ala Lys Asp Ala Phe Arg Phe Phe Ala Leu Ala
    130                 135                 140

cgt aat acg tct act act gtt cct gat gct caa aaa tat aca gtt atg      480
Arg Asn Thr Ser Thr Thr Val Pro Asp Ala Gln Lys Tyr Thr Val Met
145                 150                 155                 160

aag aat aat ggc tta tct gtt gca tca atc atg atc aat ggt tgt tat      528
Lys Asn Asn Gly Leu Ser Val Ala Ser Ile Met Ile Asn Gly Cys Tyr
                165                 170                 175

gat cta tct ttt aat aat tta gtc gta tca cct tat ata tgt gca ggt      576
Asp Leu Ser Phe Asn Asn Leu Val Val Ser Pro Tyr Ile Cys Ala Gly
            180                 185                 190

aat ggt gaa gat ttc att gaa ttt ttt gat act ttg cac att aaa ctt      624
Asn Gly Glu Asp Phe Ile Glu Phe Phe Asp Thr Leu His Ile Lys Leu
        195                 200                 205

gct tat caa gga aaa cta ggt att agt tat tac ttc ttt cct aag att      672
Ala Tyr Gln Gly Lys Leu Gly Ile Ser Tyr Tyr Phe Phe Pro Lys Ile
    210                 215                 220

aat gta ttt gct ggt ggg tac tat cat aga gtt ata ggg aat aaa ttt      720
Asn Val Phe Ala Gly Gly Tyr Tyr His Arg Val Ile Gly Asn Lys Phe
225                 230                 235                 240

aaa aat tta aat gtt aac cat gtt gtt aca ctt gat gaa ttt cct aaa      768

Lys Asn Leu Asn Val Asn His Val Val Thr Leu Asp Glu Phe Pro Lys
                245                 250                 255

gca act tct gca gta gct aca ctt ata gtt gct tat ttt ggt ggt gaa       816
Ala Thr Ser Ala Val Ala Thr Leu Ile Val Ala Tyr Phe Gly Gly Glu
            260                 265                 270

gct gga gta aag ttt aca ttt taa                                       840
Ala Gly Val Lys Phe Thr Phe
        275

<210>  24

<211>  279

<212>  PRT

<213>  Ehrlichia chaffeensis

<400>  24

Met Ser Lys Lys Lys Phe Ile Thr Ile Gly Thr Val Leu Ala Ser Leu
1               5                   10                  15

Leu Ser Phe Leu Ser Ile Glu Ser Phe Ser Ala Ile Asn His Asn His
            20                  25                  30

Thr Gly Asn Asn Thr Ser Gly Ile Tyr Ile Thr Gly Gln Tyr Arg Pro
        35                  40                  45

Gly Val Ser His Phe Ser Asn Phe Ser Val Lys Glu Thr Asn Val Asp
    50                  55                  60

Thr Ile Gln Leu Val Gly Tyr Lys Lys Ser Ala Ser Ser Ile Asp Pro
65                  70                  75                  80

Asn Thr Tyr Ser Asn Phe Gln Gly Pro Tyr Thr Val Thr Phe Gln Asp
                85                  90                  95

Asn Ala Ala Ser Phe Ser Gly Ala Ile Gly Tyr Ser Tyr Pro Glu Ser
            100                 105                 110

Leu Arg Leu Glu Leu Glu Gly Ser Tyr Glu Lys Phe Asp Val Lys Asp
        115                 120                 125

Pro Lys Asp Tyr Ser Ala Lys Asp Ala Phe Arg Phe Phe Ala Leu Ala
    130                 135                 140

Arg Asn Thr Ser Thr Thr Val Pro Asp Ala Gln Lys Tyr Thr Val Met
145                 150                 155                 160

Lys Asn Asn Gly Leu Ser Val Ala Ser Ile Met Ile Asn Gly Cys Tyr
                165                 170                 175

Asp Leu Ser Phe Asn Asn Leu Val Val Ser Pro Tyr Ile Cys Ala Gly
            180                 185                 190

Asn Gly Glu Asp Phe Ile Glu Phe Phe Asp Thr Leu His Ile Lys Leu
        195                 200                 205

Ala Tyr Gln Gly Lys Leu Gly Ile Ser Tyr Tyr Phe Phe Pro Lys Ile
    210                 215                 220

Asn Val Phe Ala Gly Gly Tyr Tyr His Arg Val Ile Gly Asn Lys Phe
225                 230                 235                 240

Lys Asn Leu Asn Val Asn His Val Val Thr Leu Asp Glu Phe Pro Lys
                245                 250                 255

Ala Thr Ser Ala Val Ala Thr Leu Ile Val Ala Tyr Phe Gly Gly Glu
            260                 265                 270

Ala Gly Val Lys Phe Thr Phe
        275

<210>  25

<211>  852

<212>  DNA

<213>  Ehrlichia chaffeensis


<220>

<221>  CDS

<222>  (1)..(852)


<400>  25
atg agt gct aaa aaa aag ctt ttt ata ata ggg tca gtg tta gta tgt        48
Met Ser Ala Lys Lys Lys Leu Phe Ile Ile Gly Ser Val Leu Val Cys
1               5                   10                  15

tta gtg tca tac tta cct act aaa tct ttg tca aac tta aat aat att        96
Leu Val Ser Tyr Leu Pro Thr Lys Ser Leu Ser Asn Leu Asn Asn Ile
            20                  25                  30

aat aat aac act aag tgc act ggg cta tat gtc agt gga caa tat aaa       144
Asn Asn Asn Thr Lys Cys Thr Gly Leu Tyr Val Ser Gly Gln Tyr Lys
        35                  40                  45

cct act gtt tct cac ttt agt aat ttt tca ctt aaa gaa act tat act       192
Pro Thr Val Ser His Phe Ser Asn Phe Ser Leu Lys Glu Thr Tyr Thr
    50                  55                  60

gac act aaa gag tta tta gga cta gca aaa gat att aag tct att aca       240
Asp Thr Lys Glu Leu Leu Gly Leu Ala Lys Asp Ile Lys Ser Ile Thr
65                  70                  75                  80

gat ata aca aca aat aaa aaa ttc aac att cct tat aac aca aaa ttt       288

Asp Ile Thr Thr Asn Lys Lys Phe Asn Ile Pro Tyr Asn Thr Lys Phe
                85                  90                  95

caa gat aat gct gtt agc ttc agt gca gct gtt gga tat att tcc caa        336
Gln Asp Asn Ala Val Ser Phe Ser Ala Ala Val Gly Tyr Ile Ser Gln
            100                 105                 110

gac agt cca agg gtt gag gta gaa tgg tct tat gaa gaa ttt gac gtt        384
Asp Ser Pro Arg Val Glu Val Glu Trp Ser Tyr Glu Glu Phe Asp Val
        115                 120                 125

aaa aat cct ggt aat tac gta gta agt gaa gcc ttc agg tat att gct        432
Lys Asn Pro Gly Asn Tyr Val Val Ser Glu Ala Phe Arg Tyr Ile Ala
    130                 135                 140

tta gca aga gga att gat aat ctt caa aaa tat cct gaa aca aat aag        480
Leu Ala Arg Gly Ile Asp Asn Leu Gln Lys Tyr Pro Glu Thr Asn Lys
145                 150                 155                 160

tat gtt gtt ata aag aac aat ggc tta tct gtc gca tcc att ata atc        528
Tyr Val Val Ile Lys Asn Asn Gly Leu Ser Val Ala Ser Ile Ile Ile
                165                 170                 175

aat ggc tgt tat gat ttt tct tta aac aat tta aaa gta tca cct tac        576
Asn Gly Cys Tyr Asp Phe Ser Leu Asn Asn Leu Lys Val Ser Pro Tyr
            180                 185                 190

ata tgc gta ggg ttt ggt ggg gac att ata gaa ttt ttt agt gct gta        624
Ile Cys Val Gly Phe Gly Gly Asp Ile Ile Glu Phe Phe Ser Ala Val
        195                 200                 205

agt ttt aaa ttt gct tat caa ggt aag gta ggt atc agt tat cca tta        672
Ser Phe Lys Phe Ala Tyr Gln Gly Lys Val Gly Ile Ser Tyr Pro Leu
    210                 215                 220

ttc tct aat atg att ata ttt gct gac gga tat tac cat aag gtc ata        720
Phe Ser Asn Met Ile Ile Phe Ala Asp Gly Tyr Tyr His Lys Val Ile
225                 230                 235                 240

gga aat aaa ttt aac aat tta aat gtt caa cac gtt gtt agt ctt aac        768
Gly Asn Lys Phe Asn Asn Leu Asn Val Gln His Val Val Ser Leu Asn
                245                 250                 255

agt cat cct aag tct act ttt gca gta gct act ctt aat gtt gag tat        816
Ser His Pro Lys Ser Thr Phe Ala Val Ala Thr Leu Asn Val Glu Tyr
            260                 265                 270

ttc ggt agt gaa ttt ggg tta aaa ttt ata ttt taa                        852
Phe Gly Ser Glu Phe Gly Leu Lys Phe Ile Phe
        275                 280

<210>  26

<211>  283

<212>  PRT

<213>  Ehrlichia chaffeensis

<400>  26

Met Ser Ala Lys Lys Lys Leu Phe Ile Ile Gly Ser Val Leu Val Cys
1               5                   10                  15

Leu Val Ser Tyr Leu Pro Thr Lys Ser Leu Ser Asn Leu Asn Asn Ile
            20                  25                  30

Asn Asn Asn Thr Lys Cys Thr Gly Leu Tyr Val Ser Gly Gln Tyr Lys
        35                  40                  45

Pro Thr Val Ser His Phe Ser Asn Phe Ser Leu Lys Glu Thr Tyr Thr
    50                  55                  60

Asp Thr Lys Glu Leu Leu Gly Leu Ala Lys Asp Ile Lys Ser Ile Thr
65                  70                  75                  80

Asp Ile Thr Thr Asn Lys Lys Phe Asn Ile Pro Tyr Asn Thr Lys Phe
                85                  90                  95

Gln Asp Asn Ala Val Ser Phe Ser Ala Ala Val Gly Tyr Ile Ser Gln
            100                 105                 110

Asp Ser Pro Arg Val Glu Val Glu Trp Ser Tyr Glu Glu Phe Asp Val
        115                 120                 125

Lys Asn Pro Gly Asn Tyr Val Val Ser Glu Ala Phe Arg Tyr Ile Ala
    130                 135                 140

Leu Ala Arg Gly Ile Asp Asn Leu Gln Lys Tyr Pro Glu Thr Asn Lys
145                 150                 155                 160

Tyr Val Val Ile Lys Asn Asn Gly Leu Ser Val Ala Ser Ile Ile Ile
                165                 170                 175

Asn Gly Cys Tyr Asp Phe Ser Leu Asn Asn Leu Lys Val Ser Pro Tyr
            180                 185                 190

Ile Cys Val Gly Phe Gly Gly Asp Ile Ile Glu Phe Phe Ser Ala Val
        195                 200                 205

Ser Phe Lys Phe Ala Tyr Gln Gly Lys Val Gly Ile Ser Tyr Pro Leu
    210                 215                 220

Phe Ser Asn Met Ile Ile Phe Ala Asp Gly Tyr Tyr His Lys Val Ile
225                 230                 235                 240

Gly Asn Lys Phe Asn Asn Leu Asn Val Gln His Val Val Ser Leu Asn
                245                 250                 255

Ser His Pro Lys Ser Thr Phe Ala Val Ala Thr Leu Asn Val Glu Tyr
            260                 265                 270

Phe Gly Ser Glu Phe Gly Leu Lys Phe Ile Phe
        275                 280

<210>  27

<211>  828

<212>  DNA

<213>  Ehrlichia chaffeensis



<220>

<221>  CDS

<222>  (1)..(828)



<400>  27
atg agt aaa aaa aat ttt att aca ata gga gca aca ctt att cat atg        48
Met Ser Lys Lys Asn Phe Ile Thr Ile Gly Ala Thr Leu Ile His Met
1               5                   10                  15

ttg tta cct aac ata tct ttt cca gaa act att aac aat aac act gat        96
Leu Leu Pro Asn Ile Ser Phe Pro Glu Thr Ile Asn Asn Asn Thr Asp
            20                  25                  30

aaa ctt tct ggg tta tat ata agt ggg caa tat aaa cca ggg att tct       144
Lys Leu Ser Gly Leu Tyr Ile Ser Gly Gln Tyr Lys Pro Gly Ile Ser
        35                  40                  45

cat ttc agc aaa ttt tca gtc aaa gaa atc tat aat gat aac att caa       192
His Phe Ser Lys Phe Ser Val Lys Glu Ile Tyr Asn Asp Asn Ile Gln
    50                  55                  60

cta att ggg tta aga cac aac gca att tct act agt acc ctt aat att       240
Leu Ile Gly Leu Arg His Asn Ala Ile Ser Thr Ser Thr Leu Asn Ile
65                  70                  75                  80

aat aca gat ttt aat atc ccc tat aaa gta aca ttt caa aat aac att       288
Asn Thr Asp Phe Asn Ile Pro Tyr Lys Val Thr Phe Gln Asn Asn Ile
                85                  90                  95

acc agc ttt agt gga gct att ggt tat tct gat ccc aca ggg gca aga       336
Thr Ser Phe Ser Gly Ala Ile Gly Tyr Ser Asp Pro Thr Gly Ala Arg
            100                 105                 110

ttt gag ctt gaa ggt tct tat gaa gaa ttt gat gtg aca gat cct gga       384
Phe Glu Leu Glu Gly Ser Tyr Glu Glu Phe Asp Val Thr Asp Pro Gly
        115                 120                 125

gac tgc tta ata aaa gat acc tat aga tat ttc gct tta gct aga aac       432
Asp Cys Leu Ile Lys Asp Thr Tyr Arg Tyr Phe Ala Leu Ala Arg Asn
    130                 135                 140

cca tca ggt tct agc cct acc tca aac aac tat act gtt atg aga aat       480
Pro Ser Gly Ser Ser Pro Thr Ser Asn Asn Tyr Thr Val Met Arg Asn
145                 150                 155                 160

gat ggt gtt tcc att act tct gtt ata ttt aat ggc tgt tat gac atc       528
Asp Gly Val Ser Ile Thr Ser Val Ile Phe Asn Gly Cys Tyr Asp Ile
                165                 170                 175

ttt tta aag gat tta gaa gta tca cct tat gta tgt gtt ggt gta ggt       576
Phe Leu Lys Asp Leu Glu Val Ser Pro Tyr Val Cys Val Gly Val Gly
            180                 185                 190

gga gat ttt ata gaa ttt ttt gac gca tta cac att aaa tta gca tac       624

Gly Asp Phe Ile Glu Phe Phe Asp Ala Leu His Ile Lys Leu Ala Tyr
        195                 200                 205

caa ggc aag tta ggt atc aat tat cac tta tcg act caa gca agc gta        672
Gln Gly Lys Leu Gly Ile Asn Tyr His Leu Ser Thr Gln Ala Ser Val
    210                 215                 220

ttt att gat gga tat tat cat aag gtt ata gga aat caa ttc aac aat        720
Phe Ile Asp Gly Tyr Tyr His Lys Val Ile Gly Asn Gln Phe Asn Asn
225                 230                 235                 240

cta aat gtt caa cac gtg gct agt aca gat ttt gga cct gta tac gca        768
Leu Asn Val Gln His Val Ala Ser Thr Asp Phe Gly Pro Val Tyr Ala
                245                 250                 255

gta gcc aca ctt aac att ggt tat ttt ggt ggt gaa atc gga att aga        816
Val Ala Thr Leu Asn Ile Gly Tyr Phe Gly Gly Glu Ile Gly Ile Arg
            260                 265                 270

ctt aca ttt taa                                                        828
Leu Thr Phe
        275

<210>  28

<211>  275

<212>  PRT

<213>  Ehrlichia chaffeensis

<400>  28

Met Ser Lys Lys Asn Phe Ile Thr Ile Gly Ala Thr Leu Ile His Met
1               5                   10                  15

Leu Leu Pro Asn Ile Ser Phe Pro Glu Thr Ile Asn Asn Asn Thr Asp
            20                  25                  30

Lys Leu Ser Gly Leu Tyr Ile Ser Gly Gln Tyr Lys Pro Gly Ile Ser
        35                  40                  45

His Phe Ser Lys Phe Ser Val Lys Glu Ile Tyr Asn Asp Asn Ile Gln
    50                  55                  60

Leu Ile Gly Leu Arg His Asn Ala Ile Ser Thr Ser Thr Leu Asn Ile
65                  70                  75                  80

Asn Thr Asp Phe Asn Ile Pro Tyr Lys Val Thr Phe Gln Asn Asn Ile
                85                  90                  95

Thr Ser Phe Ser Gly Ala Ile Gly Tyr Ser Asp Pro Thr Gly Ala Arg
            100                 105                 110

Phe Glu Leu Glu Gly Ser Tyr Glu Glu Phe Asp Val Thr Asp Pro Gly
        115                 120                 125

Asp Cys Leu Ile Lys Asp Thr Tyr Arg Tyr Phe Ala Leu Ala Arg Asn
    130                 135                 140

Pro Ser Gly Ser Ser Pro Thr Ser Asn Asn Tyr Thr Val Met Arg Asn
145                 150                 155                 160

Asp Gly Val Ser Ile Thr Ser Val Ile Phe Asn Gly Cys Tyr Asp Ile
                165                 170                 175

Phe Leu Lys Asp Leu Glu Val Ser Pro Tyr Val Cys Val Gly Val Gly
            180                 185                 190

Gly Asp Phe Ile Glu Phe Phe Asp Ala Leu His Ile Lys Leu Ala Tyr
        195                 200                 205

Gln Gly Lys Leu Gly Ile Asn Tyr His Leu Ser Thr Gln Ala Ser Val
    210                 215                 220

Phe Ile Asp Gly Tyr Tyr His Lys Val Ile Gly Asn Gln Phe Asn Asn
225                 230                 235                 240

Leu Asn Val Gln His Val Ala Ser Thr Asp Phe Gly Pro Val Tyr Ala
                245                 250                 255

Val Ala Thr Leu Asn Ile Gly Tyr Phe Gly Gly Glu Ile Gly Ile Arg
            260                 265                 270

Leu Thr Phe
        275

<210>  29

<211>  858

<212>  DNA

<213>  Ehrlichia chaffeensis

<220>

<221>  CDS

<222>  (1)..(858)

<400>  29
atg aat aat aga aaa agt ttt ttt ata ata ggt gca tca tta cta gca        48
Met Asn Asn Arg Lys Ser Phe Phe Ile Ile Gly Ala Ser Leu Leu Ala
1               5                   10                  15

agc tta tta ttc aca tct gag gcc tct tct aca gga aat gta agt aac        96
Ser Leu Leu Phe Thr Ser Glu Ala Ser Ser Thr Gly Asn Val Ser Asn
            20                  25                  30

cat act tat ttt aaa cct agg tta tat atc agt gga caa tat aga cca       144

His Thr Tyr Phe Lys Pro Arg Leu Tyr Ile Ser Gly Gln Tyr Arg Pro
        35                  40                  45

gga gtt tct cat ttt agc aaa ttt tca gtc aaa gaa acc aac tac aat        192
Gly Val Ser His Phe Ser Lys Phe Ser Val Lys Glu Thr Asn Tyr Asn
    50                  55                  60

act act caa cta gtt ggg ctt aaa aag gac atc agt gtc ata ggg aac        240
Thr Thr Gln Leu Val Gly Leu Lys Lys Asp Ile Ser Val Ile Gly Asn
65                  70                  75                  80

agt aat atc aca acc tac aca aat ttc aac ttt cct tac att gca gaa        288
Ser Asn Ile Thr Thr Tyr Thr Asn Phe Asn Phe Pro Tyr Ile Ala Glu
                85                  90                  95

ttt caa gac aat gcc ata agt ttc agt ggg gca att gga tac ttg tat        336
Phe Gln Asp Asn Ala Ile Ser Phe Ser Gly Ala Ile Gly Tyr Leu Tyr
            100                 105                 110

tcc gag aat ttt aga att gaa gta gag gct tct tat gaa gaa ttt gat        384
Ser Glu Asn Phe Arg Ile Glu Val Glu Ala Ser Tyr Glu Glu Phe Asp
        115                 120                 125

gtt aaa aat cca gaa gga tct gct aca gac gca tac agg tat ttt gca        432
Val Lys Asn Pro Glu Gly Ser Ala Thr Asp Ala Tyr Arg Tyr Phe Ala
    130                 135                 140

cta gca cgt gct atg gat ggc act aat aaa tct agt cct gat gac aca        480
Leu Ala Arg Ala Met Asp Gly Thr Asn Lys Ser Ser Pro Asp Asp Thr
145                 150                 155                 160

aga aaa ttc act gtc atg aga aat gac ggg ttt tca att tca tca gta        528
Arg Lys Phe Thr Val Met Arg Asn Asp Gly Phe Ser Ile Ser Ser Val
                165                 170                 175

atg ata aat ggg tgt tac aat ttt aca tta gat gat ata cca gta gta        576
Met Ile Asn Gly Cys Tyr Asn Phe Thr Leu Asp Asp Ile Pro Val Val
            180                 185                 190

ccg tat gta tgc gca gga ata gga gga gat ttc ata gag ttt ttt aat        624
Pro Tyr Val Cys Ala Gly Ile Gly Gly Asp Phe Ile Glu Phe Phe Asn
        195                 200                 205

gat tta cat gtt aag ttt cgt cat caa ggc aag gta ggt att agt tat        672
Asp Leu His Val Lys Phe Arg His Gln Gly Lys Val Gly Ile Ser Tyr
    210                 215                 220

tct ata tcc cct gaa gta agt tta ttt ctt aac gga tat tac cat aaa        720
Ser Ile Ser Pro Glu Val Ser Leu Phe Leu Asn Gly Tyr Tyr His Lys
225                 230                 235                 240

gta aca ggt aac aga ttt aaa aac tta cac gtt caa cac gta agt gat        768
Val Thr Gly Asn Arg Phe Lys Asn Leu His Val Gln His Val Ser Asp
                245                 250                 255

tta agt gac gct cct aag ttc aca tct gca gtt gct aca ctc aat gtt        816
Leu Ser Asp Ala Pro Lys Phe Thr Ser Ala Val Ala Thr Leu Asn Val
            260                 265                 270

ggg tac ttt ggt ggc gaa att gga gta aga ttt ata ttt taa                858
Gly Tyr Phe Gly Gly Glu Ile Gly Val Arg Phe Ile Phe
        275                 280                 285

<210>  30

<211>  285

<212>  PRT

<213>  Ehrlichia chaffeensis

<400>  30

Met Asn Asn Arg Lys Ser Phe Phe Ile Ile Gly Ala Ser Leu Leu Ala
1               5                   10                  15

Ser Leu Leu Phe Thr Ser Glu Ala Ser Ser Thr Gly Asn Val Ser Asn
            20                  25                  30

His Thr Tyr Phe Lys Pro Arg Leu Tyr Ile Ser Gly Gln Tyr Arg Pro
        35                  40                  45

Gly Val Ser His Phe Ser Lys Phe Ser Val Lys Glu Thr Asn Tyr Asn
    50                  55                  60

Thr Thr Gln Leu Val Gly Leu Lys Lys Asp Ile Ser Val Ile Gly Asn
65                  70                  75                  80

Ser Asn Ile Thr Thr Tyr Thr Asn Phe Asn Phe Pro Tyr Ile Ala Glu
                85                  90                  95

Phe Gln Asp Asn Ala Ile Ser Phe Ser Gly Ala Ile Gly Tyr Leu Tyr
            100                 105                 110

Ser Glu Asn Phe Arg Ile Glu Val Glu Ala Ser Tyr Glu Glu Phe Asp
        115                 120                 125

Val Lys Asn Pro Glu Gly Ser Ala Thr Asp Ala Tyr Arg Tyr Phe Ala
    130                 135                 140

Leu Ala Arg Ala Met Asp Gly Thr Asn Lys Ser Ser Pro Asp Asp Thr
145                 150                 155                 160

Arg Lys Phe Thr Val Met Arg Asn Asp Gly Phe Ser Ile Ser Ser Val
                165                 170                 175

Met Ile Asn Gly Cys Tyr Asn Phe Thr Leu Asp Asp Ile Pro Val Val
            180                 185                 190

Pro Tyr Val Cys Ala Gly Ile Gly Gly Asp Phe Ile Glu Phe Phe Asn
        195                 200                 205

Asp Leu His Val Lys Phe Arg His Gln Gly Lys Val Gly Ile Ser Tyr
    210                 215                 220

Ser Ile Ser Pro Glu Val Ser Leu Phe Leu Asn Gly Tyr Tyr His Lys
225                 230                 235                 240

Val Thr Gly Asn Arg Phe Lys Asn Leu His Val Gln His Val Ser Asp
            245                 250                 255

Leu Ser Asp Ala Pro Lys Phe Thr Ser Ala Val Ala Thr Leu Asn Val
        260                 265                 270

Gly Tyr Phe Gly Gly Glu Ile Gly Val Arg Phe Ile Phe
    275                 280                 285

<210>  31

<211>  867

<212>  DNA

<213>  Ehrlichia canis

<220>

<221>  CDS

<222>  (1)..(867)

<400>  31
atg aat tgc aaa aga ttt ttc ata gca agt gca ttg ata tca cta atg        48
Met Asn Cys Lys Arg Phe Phe Ile Ala Ser Ala Leu Ile Ser Leu Met
1               5                   10                  15

tct ttc tta cct agc gta tct ttt tct gaa tca ata cat gaa gat aat        96
Ser Phe Leu Pro Ser Val Ser Phe Ser Glu Ser Ile His Glu Asp Asn
            20                  25                  30

ata aat ggt aac ttt tac att agt gca aag tat atg cca agt gcc tca       144
Ile Asn Gly Asn Phe Tyr Ile Ser Ala Lys Tyr Met Pro Ser Ala Ser
        35                  40                  45

cac ttt ggc gta ttt tca gtt aaa gaa gag aaa aac aca aca act gga       192
His Phe Gly Val Phe Ser Val Lys Glu Glu Lys Asn Thr Thr Thr Gly
    50                  55                  60

gtt ttc gga tta aaa caa gat tgg gac gga gca aca ata aag gat gca       240
Val Phe Gly Leu Lys Gln Asp Trp Asp Gly Ala Thr Ile Lys Asp Ala
65                  70                  75                  80

agc agc agc cac aca ata gac cca agt aca ata ttc tcc att tca aat       288
Ser Ser Ser His Thr Ile Asp Pro Ser Thr Ile Phe Ser Ile Ser Asn
                85                  90                  95

tat tca ttt aaa tat gaa aac aat cca ttt tta ggg ttt gca gga gct       336
Tyr Ser Phe Lys Tyr Glu Asn Asn Pro Phe Leu Gly Phe Ala Gly Ala
            100                 105                 110

att ggc tac tca atg ggt ggt cca agg gta gag ttt gaa gtg tct tac       384
Ile Gly Tyr Ser Met Gly Gly Pro Arg Val Glu Phe Glu Val Ser Tyr
        115                 120                 125

gaa ata ttt gat gta aaa aac caa ggt aac agt tac aag aac gat gct       432
Glu Ile Phe Asp Val Lys Asn Gln Gly Asn Ser Tyr Lys Asn Asp Ala
    130                 135                 140

cac aaa tat tgc gct tta tca aga cac acc gga ggt atg cca caa gcc       480

His Lys Tyr Cys Ala Leu Ser Arg His Thr Gly Gly Met Pro Gln Ala
145                 150                 155                 160

ggt cat caa aat aaa ttt gtc ttc cta aaa aat gaa gga tta ctt gac       528
Gly His Gln Asn Lys Phe Val Phe Leu Lys Asn Glu Gly Leu Leu Asp
                165                 170                 175

ata tca ctt atg ata aac gca tgt tat gat ata aca atc gac agc atg       576
Ile Ser Leu Met Ile Asn Ala Cys Tyr Asp Ile Thr Ile Asp Ser Met
            180                 185                 190

cca ttt tct cca tat ata tgt gca ggt att ggt agt gac tta gtt tcg       624
Pro Phe Ser Pro Tyr Ile Cys Ala Gly Ile Gly Ser Asp Leu Val Ser
        195                 200                 205

atg ttt gaa act aca aat cct aaa att tct tat caa gga aaa tta ggt       672
Met Phe Glu Thr Thr Asn Pro Lys Ile Ser Tyr Gln Gly Lys Leu Gly
    210                 215                 220

gta agt tac tcc ata agc cca gaa gca tct gtt ttt gtt gga gga cac       720
Val Ser Tyr Ser Ile Ser Pro Glu Ala Ser Val Phe Val Gly Gly His
225                 230                 235                 240

ttt cac aga gtt ata ggt aat gaa ttt aaa gac att cct gca ata act       768
Phe His Arg Val Ile Gly Asn Glu Phe Lys Asp Ile Pro Ala Ile Thr
                245                 250                 255

cct gct gga gca aca gaa att aaa ggc aca cag ttt aca aca gta aca       816
Pro Ala Gly Ala Thr Glu Ile Lys Gly Thr Gln Phe Thr Thr Val Thr
            260                 265                 270

tta aac ata tgc cac ttc gga cta gag ctt gga ggc agg ttt act ttt       864
Leu Asn Ile Cys His Phe Gly Leu Glu Leu Gly Gly Arg Phe Thr Phe
        275                 280                 285

taa                                                                   867

<210>  32

<211>  288

<212>  PRT

<213>  Ehrlichia canis

<400>  32

Met Asn Cys Lys Arg Phe Phe Ile Ala Ser Ala Leu Ile Ser Leu Met
1               5                   10                  15

Ser Phe Leu Pro Ser Val Ser Phe Ser Glu Ser Ile His Glu Asp Asn
            20                  25                  30

Ile Asn Gly Asn Phe Tyr Ile Ser Ala Lys Tyr Met Pro Ser Ala Ser
        35                  40                  45

His Phe Gly Val Phe Ser Val Lys Glu Glu Lys Asn Thr Thr Thr Gly
    50                  55                  60

Val Phe Gly Leu Lys Gln Asp Trp Asp Gly Ala Thr Ile Lys Asp Ala
65                  70                  75                  80

Ser Ser Ser His Thr Ile Asp Pro Ser Thr Ile Phe Ser Ile Ser Asn
                85                  90                  95

Tyr Ser Phe Lys Tyr Glu Asn Asn Pro Phe Leu Gly Phe Ala Gly Ala
            100                 105                 110

Ile Gly Tyr Ser Met Gly Gly Pro Arg Val Glu Phe Glu Val Ser Tyr
        115                 120                 125

Glu Ile Phe Asp Val Lys Asn Gln Gly Asn Ser Tyr Lys Asn Asp Ala
    130                 135                 140

His Lys Tyr Cys Ala Leu Ser Arg His Thr Gly Gly Met Pro Gln Ala
145                 150                 155                 160

Gly His Gln Asn Lys Phe Val Phe Leu Lys Asn Glu Gly Leu Leu Asp
                165                 170                 175

Ile Ser Leu Met Ile Asn Ala Cys Tyr Asp Ile Thr Ile Asp Ser Met
            180                 185                 190

Pro Phe Ser Pro Tyr Ile Cys Ala Gly Ile Gly Ser Asp Leu Val Ser
        195                 200                 205

Met Phe Glu Thr Thr Asn Pro Lys Ile Ser Tyr Gln Gly Lys Leu Gly
    210                 215                 220

Val Ser Tyr Ser Ile Ser Pro Glu Ala Ser Val Phe Val Gly Gly His
225                 230                 235                 240

Phe His Arg Val Ile Gly Asn Glu Phe Lys Asp Ile Pro Ala Ile Thr
                245                 250                 255

Pro Ala Gly Ala Thr Glu Ile Lys Gly Thr Gln Phe Thr Thr Val Thr
            260                 265                 270

Leu Asn Ile Cys His Phe Gly Leu Glu Leu Gly Gly Arg Phe Thr Phe
        275                 280                 285

<210>  33

<211>  864

<212>  DNA

<213>  Ehrlichia chaffeensis


<220>

<221>  CDS

<222>  (1)..(864)

<400>  33
atg aaa tat aaa aaa act ttt aca gta act gca tta gta tta tta act      48
Met Lys Tyr Lys Lys Thr Phe Thr Val Thr Ala Leu Val Leu Leu Thr
1               5                   10                  15

tcc ttt aca cat ttt ata cct ttt tat agt cca gca cgt gcc agt aca      96
Ser Phe Thr His Phe Ile Pro Phe Tyr Ser Pro Ala Arg Ala Ser Thr
            20                  25                  30

att cac aac ttc tac att agt gga aaa tat atg cca aca gcg tca cat      144
Ile His Asn Phe Tyr Ile Ser Gly Lys Tyr Met Pro Thr Ala Ser His
        35                  40                  45

ttt gga att ttt tca gct aaa gaa gaa caa agt ttt act aag gta tta      192
Phe Gly Ile Phe Ser Ala Lys Glu Glu Gln Ser Phe Thr Lys Val Leu
    50                  55                  60

gtt ggg tta gat caa cga tta tca cat aat att ata aac aat aat gat      240
Val Gly Leu Asp Gln Arg Leu Ser His Asn Ile Ile Asn Asn Asn Asp
65                  70                  75                  80

aca gca aag agt ctt aag gtt caa aat tat tca ttt aaa tac aaa aat      288
Thr Ala Lys Ser Leu Lys Val Gln Asn Tyr Ser Phe Lys Tyr Lys Asn
                85                  90                  95

aac cca ttt cta gga ttt gca aga gct att ggt tat tca ata ggc aat      336
Asn Pro Phe Leu Gly Phe Ala Arg Ala Ile Gly Tyr Ser Ile Gly Asn
            100                 105                 110

tca aga ata gaa cta gaa gta tca cat gaa ata ttt gat act aaa aac      384
Ser Arg Ile Glu Leu Glu Val Ser His Glu Ile Phe Asp Thr Lys Asn
        115                 120                 125

cca gga aac aat tat tta aat gac tct cac aaa tat tgc gct tta tct      432
Pro Gly Asn Asn Tyr Leu Asn Asp Ser His Lys Tyr Cys Ala Leu Ser
    130                 135                 140

cat gga agt cac ata tgc agt gat gga aat agc gga gat tgg tac act      480
His Gly Ser His Ile Cys Ser Asp Gly Asn Ser Gly Asp Trp Tyr Thr
145                 150                 155                 160

gca aaa act gat aag ttt gta ctt ctg aaa aat gaa ggt tta ctt gac      528
Ala Lys Thr Asp Lys Phe Val Leu Leu Lys Asn Glu Gly Leu Leu Asp
                165                 170                 175

gtc tca ttt atg tta aac gca tgt tat gac ata aca act gaa aaa atg      576
Val Ser Phe Met Leu Asn Ala Cys Tyr Asp Ile Thr Thr Glu Lys Met
            180                 185                 190

cct ttt tca cct tat ata tgt gca ggt att ggt act gat ctc ata tct      624
Pro Phe Ser Pro Tyr Ile Cys Ala Gly Ile Gly Thr Asp Leu Ile Ser
        195                 200                 205

atg ttt gag aca aca caa aac aaa ata tct tat caa gga aag tta ggt      672
Met Phe Glu Thr Thr Gln Asn Lys Ile Ser Tyr Gln Gly Lys Leu Gly
    210                 215                 220

tta aac tat act ata aac tca aga gtt tct gtt ttt gca ggt ggg cac      720
Leu Asn Tyr Thr Ile Asn Ser Arg Val Ser Val Phe Ala Gly Gly His
225                 230                 235                 240

ttt cat aaa gta ata ggt aat gaa ttt aaa ggt att cct act cta tta      768
Phe His Lys Val Ile Gly Asn Glu Phe Lys Gly Ile Pro Thr Leu Leu
                245                 250                 255

cct gat gga tca aac att aaa gta caa cag tct gca aca gta aca tta        816
Pro Asp Gly Ser Asn Ile Lys Val Gln Gln Ser Ala Thr Val Thr Leu
            260                 265                 270

gat gtg tgc cat ttc ggg tta gag att gga agt aga ttt ttc ttt taa        864
Asp Val Cys His Phe Gly Leu Glu Ile Gly Ser Arg Phe Phe Phe
        275                 280                 285

<210>  34

<211>  287

<212>  PRT

<213>  Ehrlichia chaffeensis

<400>  34

Met Lys Tyr Lys Lys Thr Phe Thr Val Thr Ala Leu Val Leu Leu Thr
1               5                   10                  15

Ser Phe Thr His Phe Ile Pro Phe Tyr Ser Pro Ala Arg Ala Ser Thr
            20                  25                  30

Ile His Asn Phe Tyr Ile Ser Gly Lys Tyr Met Pro Thr Ala Ser His
        35                  40                  45

Phe Gly Ile Phe Ser Ala Lys Glu Glu Gln Ser Phe Thr Lys Val Leu
    50                  55                  60

Val Gly Leu Asp Gln Arg Leu Ser His Asn Ile Ile Asn Asn Asn Asp
65                  70                  75                  80

Thr Ala Lys Ser Leu Lys Val Gln Asn Tyr Ser Phe Lys Tyr Lys Asn
                85                  90                  95

Asn Pro Phe Leu Gly Phe Ala Arg Ala Ile Gly Tyr Ser Ile Gly Asn
            100                 105                 110

Ser Arg Ile Glu Leu Glu Val Ser His Glu Ile Phe Asp Thr Lys Asn
        115                 120                 125

Pro Gly Asn Asn Tyr Leu Asn Asp Ser His Lys Tyr Cys Ala Leu Ser
    130                 135                 140

His Gly Ser His Ile Cys Ser Asp Gly Asn Ser Gly Asp Trp Tyr Thr
145                 150                 155                 160

Ala Lys Thr Asp Lys Phe Val Leu Leu Lys Asn Glu Gly Leu Leu Asp
                165                 170                 175

Val Ser Phe Met Leu Asn Ala Cys Tyr Asp Ile Thr Thr Glu Lys Met
            180                 185                 190

Pro Phe Ser Pro Tyr Ile Cys Ala Gly Ile Gly Thr Asp Leu Ile Ser
        195                 200                 205

Met Phe Glu Thr Thr Gln Asn Lys Ile Ser Tyr Gln Gly Lys Leu Gly
    210                 215                 220

Leu Asn Tyr Thr Ile Asn Ser Arg Val Ser Val Phe Ala Gly Gly His
225                 230                 235                 240

Phe His Lys Val Ile Gly Asn Glu Phe Lys Gly Ile Pro Thr Leu Leu
                245                 250                 255

Pro Asp Gly Ser Asn Ile Lys Val Gln Gln Ser Ala Thr Val Thr Leu
            260                 265                 270

Asp Val Cys His Phe Gly Leu Glu Ile Gly Ser Arg Phe Phe Phe
        275                 280                 285

<210>  35

<211>  924

<212>  DNA

<213>  Ehrlichia canis

<220>

<221>  CDS

<222>  (1)..(924)

<400>  35
atg ttt tat act aat ata tat att ctg gct tgt att tac ttt gca ctt      48
Met Phe Tyr Thr Asn Ile Tyr Ile Leu Ala Cys Ile Tyr Phe Ala Leu
1               5                   10                  15

cca cta ttg tta att tat ttt cac tat ttt agg tgt aat atg aat tgc      96
Pro Leu Leu Leu Ile Tyr Phe His Tyr Phe Arg Cys Asn Met Asn Cys
            20                  25                  30

aaa aaa att ctt ata aca act gca tta ata tca tta atg tac tct att      144
Lys Lys Ile Leu Ile Thr Thr Ala Leu Ile Ser Leu Met Tyr Ser Ile
        35                  40                  45

cca agc ata tct ttt tct gat act ata caa gat ggt aac atg ggt ggt      192
Pro Ser Ile Ser Phe Ser Asp Thr Ile Gln Asp Gly Asn Met Gly Gly
    50                  55                  60

aac ttc tat att agt gga aag tat gta cca agt gtc tca cat ttt ggt      240
Asn Phe Tyr Ile Ser Gly Lys Tyr Val Pro Ser Val Ser His Phe Gly
65                  70                  75                  80

agc ttc tca gct aaa gaa gaa agc aaa tca act gtt gga gtt ttt gga      288
Ser Phe Ser Ala Lys Glu Glu Ser Lys Ser Thr Val Gly Val Phe Gly
                85                  90                  95

tta aaa cat gat tgg gat gga agt cca ata ctt aag aat aaa cac gct 336
Leu Lys His Asp Trp Asp Gly Ser Pro Ile Leu Lys Asn Lys His Ala
            100                 105                 110

gac ttt act gtt cca aac tat tcg ttc aga tac gag aac aat cca ttt 384
Asp Phe Thr Val Pro Asn Tyr Ser Phe Arg Tyr Glu Asn Asn Pro Phe
        115                 120                 125

cta ggg ttt gca gga gct atc ggt tac tca atg ggt ggc cca aga ata 432
Leu Gly Phe Ala Gly Ala Ile Gly Tyr Ser Met Gly Gly Pro Arg Ile
    130                 135                 140

gaa ttc gaa ata tct tat gaa gca ttc gac gta aaa agt cct aat atc 480
Glu Phe Glu Ile Ser Tyr Glu Ala Phe Asp Val Lys Ser Pro Asn Ile
145                 150                 155                 160

aat tat caa aat gac gcg cac agg tac tgc gct cta tct cat cac aca 528
Asn Tyr Gln Asn Asp Ala His Arg Tyr Cys Ala Leu Ser His His Thr
                165                 170                 175

tcg gca gcc atg gaa gct gat aaa ttt gtc ttc tta aaa aac gaa ggg 576
Ser Ala Ala Met Glu Ala Asp Lys Phe Val Phe Leu Lys Asn Glu Gly
            180                 185                 190

tta att gac ata tca ctt gca ata aat gca tgt tat gat ata ata aat 624
Leu Ile Asp Ile Ser Leu Ala Ile Asn Ala Cys Tyr Asp Ile Ile Asn
        195                 200                 205

gac aaa gta cct gtt tct cct tat ata tgc gca ggt att ggt act gat 672
Asp Lys Val Pro Val Ser Pro Tyr Ile Cys Ala Gly Ile Gly Thr Asp
    210                 215                 220

ttg att tct atg ttt gaa gct aca agt cct aaa att tcc tac caa gga 720
Leu Ile Ser Met Phe Glu Ala Thr Ser Pro Lys Ile Ser Tyr Gln Gly
225                 230                 235                 240

aaa ctg ggc att agt tac tct att aat ccg gaa acc tct gtt ttc atc 768
Lys Leu Gly Ile Ser Tyr Ser Ile Asn Pro Glu Thr Ser Val Phe Ile
                245                 250                 255

ggt ggg cat ttc cac agg atc ata ggt aat gag ttt aga gat att cct 816
Gly Gly His Phe His Arg Ile Ile Gly Asn Glu Phe Arg Asp Ile Pro
            260                 265                 270

gca ata gta cct agt aac tca act aca ata agt gga cca caa ttt gca 864
Ala Ile Val Pro Ser Asn Ser Thr Thr Ile Ser Gly Pro Gln Phe Ala
        275                 280                 285

aca gta aca cta aat gtg tgt cac ttt ggt tta gaa ctt gga gga aga 912
Thr Val Thr Leu Asn Val Cys His Phe Gly Leu Glu Leu Gly Gly Arg
    290                 295                 300

ttt aac ttc taa 924
Phe Asn Phe
305

<210> 36

<211> 307

<212> PRT

<213> Ehrlichia canis

<400> 36

Met Phe Tyr Thr Asn Ile Tyr Ile Leu Ala Cys Ile Tyr Phe Ala Leu
1               5                   10                  15

Pro Leu Leu Leu Ile Tyr Phe His Tyr Phe Arg Cys Asn Met Asn Cys
            20                  25                  30

Lys Lys Ile Leu Ile Thr Thr Ala Leu Ile Ser Leu Met Tyr Ser Ile
        35                  40                  45

Pro Ser Ile Ser Phe Ser Asp Thr Ile Gln Asp Gly Asn Met Gly Gly
    50                  55                  60

Asn Phe Tyr Ile Ser Gly Lys Tyr Val Pro Ser Val Ser His Phe Gly
65                  70                  75                  80

Ser Phe Ser Ala Lys Glu Glu Ser Lys Ser Thr Val Gly Val Phe Gly
                85                  90                  95

Leu Lys His Asp Trp Asp Gly Ser Pro Ile Leu Lys Asn Lys His Ala
            100                 105                 110

Asp Phe Thr Val Pro Asn Tyr Ser Phe Arg Tyr Glu Asn Asn Pro Phe
        115                 120                 125

Leu Gly Phe Ala Gly Ala Ile Gly Tyr Ser Met Gly Gly Pro Arg Ile
    130                 135                 140

Glu Phe Glu Ile Ser Tyr Glu Ala Phe Asp Val Lys Ser Pro Asn Ile
145                 150                 155                 160

Asn Tyr Gln Asn Asp Ala His Arg Tyr Cys Ala Leu Ser His His Thr
                165                 170                 175

Ser Ala Ala Met Glu Ala Asp Lys Phe Val Phe Leu Lys Asn Glu Gly
            180                 185                 190

Leu Ile Asp Ile Ser Leu Ala Ile Asn Ala Cys Tyr Asp Ile Ile Asn
        195                 200                 205

Asp Lys Val Pro Val Ser Pro Tyr Ile Cys Ala Gly Ile Gly Thr Asp
    210                 215                 220

Leu Ile Ser Met Phe Glu Ala Thr Ser Pro Lys Ile Ser Tyr Gln Gly
225                 230                 235                 240

Lys Leu Gly Ile Ser Tyr Ser Ile Asn Pro Glu Thr Ser Val Phe Ile
                245                 250                 255

Gly Gly His Phe His Arg Ile Ile Gly Asn Glu Phe Arg Asp Ile Pro
            260                 265                 270

Ala Ile Val Pro Ser Asn Ser Thr Thr Ile Ser Gly Pro Gln Phe Ala
        275                 280                 285

Thr Val Thr Leu Asn Val Cys His Phe Gly Leu Glu Leu Gly Gly Arg
    290                 295                 300

Phe Asn Phe
305

<210>  37

<211>  843

<212>  DNA

<213>  Ehrlichia canis

<220>

<221>  CDS

<222>  (1)..(843)

<400>  37
atg aat tgc aaa aaa att ctt ata aca act gca tta atg tca tta atg        48
Met Asn Cys Lys Lys Ile Leu Ile Thr Thr Ala Leu Met Ser Leu Met
1               5                   10                  15

tac tat gct cca agc ata tct ttt tct gat act ata caa gac gat aac        96
Tyr Tyr Ala Pro Ser Ile Ser Phe Ser Asp Thr Ile Gln Asp Asp Asn
            20                  25                  30

act ggt agc ttc tac atc agt gga aaa tat gta cca agt gtt tca cat       144
Thr Gly Ser Phe Tyr Ile Ser Gly Lys Tyr Val Pro Ser Val Ser His
        35                  40                  45

ttt ggt gtt ttc tca gct aaa gaa gaa aga aac tca act gtt gga gtt       192
Phe Gly Val Phe Ser Ala Lys Glu Glu Arg Asn Ser Thr Val Gly Val
    50                  55                  60

ttt gga tta aaa cat gat tgg aat gga ggt aca ata tct aac tct tct       240
Phe Gly Leu Lys His Asp Trp Asn Gly Gly Thr Ile Ser Asn Ser Ser
65                  70                  75                  80

cca gaa aat ata ttc aca gtt caa aat tat tcg ttt aaa tac gaa aac       288
Pro Glu Asn Ile Phe Thr Val Gln Asn Tyr Ser Phe Lys Tyr Glu Asn
                85                  90                  95

aac cca ttc tta ggg ttt gca gga gct att ggt tat tca atg ggt ggc       336
Asn Pro Phe Leu Gly Phe Ala Gly Ala Ile Gly Tyr Ser Met Gly Gly
            100                 105                 110

cca aga ata gaa ctt gaa gtt ctg tac gag aca ttc gat gtg aaa aat       384
Pro Arg Ile Glu Leu Glu Val Leu Tyr Glu Thr Phe Asp Val Lys Asn
        115                 120                 125

cag aac aat aat tat aag aac ggc gca cac aga tac tgt gct tta tct       432
Gln Asn Asn Asn Tyr Lys Asn Gly Ala His Arg Tyr Cys Ala Leu Ser
    130                 135                 140

cat cat agt tca gca aca aac atg tcc tcc gca agt aac aaa ttt gtt 480
His His Ser Ser Ala Thr Asn Met Ser Ser Ala Ser Asn Lys Phe Val
145                 150                 155                 160

ttc tta aaa aat gaa ggg tta att gac tta tca ttt atg ata aat gca 528
Phe Leu Lys Asn Glu Gly Leu Ile Asp Leu Ser Phe Met Ile Asn Ala
                165                 170                 175

tgc tat gac ata ata att gaa gga atg cct ttt tca cct tat att tgt 576
Cys Tyr Asp Ile Ile Ile Glu Gly Met Pro Phe Ser Pro Tyr Ile Cys
            180                 185                 190

gca ggt gtt ggt act gat gtt gtt tcc atg ttt gaa gct ata aat cct 624
Ala Gly Val Gly Thr Asp Val Val Ser Met Phe Glu Ala Ile Asn Pro
        195                 200                 205

aaa att tct tac caa gga aaa cta gga tta ggt tat agt ata agt tca 672
Lys Ile Ser Tyr Gln Gly Lys Leu Gly Leu Gly Tyr Ser Ile Ser Ser
    210                 215                 220

gaa gcc tct gtt ttt atc ggt gga cac ttt cac aga gtc ata ggt aat 720
Glu Ala Ser Val Phe Ile Gly Gly His Phe His Arg Val Ile Gly Asn
225                 230                 235                 240

gaa ttt aga gac atc cct gct atg gtt cct agt gga tca aat ctt cca 768
Glu Phe Arg Asp Ile Pro Ala Met Val Pro Ser Gly Ser Asn Leu Pro
                245                 250                 255

gaa aac caa ttt gca ata gta aca cta aat gtg tgt cac ttt ggt tta 816
Glu Asn Gln Phe Ala Ile Val Thr Leu Asn Val Cys His Phe Gly Leu
            260                 265                 270

gaa ctt gga gga aga ttt aac ttc tga 843
Glu Leu Gly Gly Arg Phe Asn Phe
        275                 280

<210> 38

<211> 280

<212> PRT

<213> Ehrlichia canis

<400> 38

Met Asn Cys Lys Lys Ile Leu Ile Thr Thr Ala Leu Met Ser Leu Met
1               5                   10                  15

Tyr Tyr Ala Pro Ser Ile Ser Phe Ser Asp Thr Ile Gln Asp Asp Asn
            20                  25                  30

Thr Gly Ser Phe Tyr Ile Ser Gly Lys Tyr Val Pro Ser Val Ser His
        35                  40                  45

Phe Gly Val Phe Ser Ala Lys Glu Glu Arg Asn Ser Thr Val Gly Val
    50                  55                  60

Phe Gly Leu Lys His Asp Trp Asn Gly Gly Thr Ile Ser Asn Ser Ser
65                  70                  75                  80

Pro Glu Asn Ile Phe Thr Val Gln Asn Tyr Ser Phe Lys Tyr Glu Asn
                85                  90                  95

Asn Pro Phe Leu Gly Phe Ala Gly Ala Ile Gly Tyr Ser Met Gly Gly
            100                 105                 110

Pro Arg Ile Glu Leu Glu Val Leu Tyr Glu Thr Phe Asp Val Lys Asn
        115                 120                 125

Gln Asn Asn Asn Tyr Lys Asn Gly Ala His Arg Tyr Cys Ala Leu Ser
    130                 135                 140

His His Ser Ser Ala Thr Asn Met Ser Ser Ala Ser Asn Lys Phe Val
145                 150                 155                 160

Phe Leu Lys Asn Glu Gly Leu Ile Asp Leu Ser Phe Met Ile Asn Ala
                165                 170                 175

Cys Tyr Asp Ile Ile Ile Glu Gly Met Pro Phe Ser Pro Tyr Ile Cys
            180                 185                 190

Ala Gly Val Gly Thr Asp Val Val Ser Met Phe Glu Ala Ile Asn Pro
        195                 200                 205

Lys Ile Ser Tyr Gln Gly Lys Leu Gly Leu Gly Tyr Ser Ile Ser Ser
    210                 215                 220

Glu Ala Ser Val Phe Ile Gly Gly His Phe His Arg Val Ile Gly Asn
225                 230                 235                 240

Glu Phe Arg Asp Ile Pro Ala Met Val Pro Ser Gly Ser Asn Leu Pro
                245                 250                 255

Glu Asn Gln Phe Ala Ile Val Thr Leu Asn Val Cys His Phe Gly Leu
            260                 265                 270

Glu Leu Gly Gly Arg Phe Asn Phe
        275                 280

<210>  39

<211>  852

<212>  DNA

<213>  Ehrlichia canis


<220>

<221>  CDS

<222>  (1)..(852)

<400>  39
atg aat tgt aaa aaa gtt ttc aca ata agt gca ttg ata tca tcc ata      48
Met Asn Cys Lys Lys Val Phe Thr Ile Ser Ala Leu Ile Ser Ser Ile
1               5                   10                  15

tac ttc cta cct aat gtc tca tac tct aac cca gta tat ggt aac agt      96
Tyr Phe Leu Pro Asn Val Ser Tyr Ser Asn Pro Val Tyr Gly Asn Ser
            20                  25                  30

atg tat ggt aat ttt tac ata tca gga aag tac atg cca agt gtt cct      144
Met Tyr Gly Asn Phe Tyr Ile Ser Gly Lys Tyr Met Pro Ser Val Pro
        35                  40                  45

cat ttt gga att ttt tca gct gaa gaa gag aaa aaa aag aca act gta      192
His Phe Gly Ile Phe Ser Ala Glu Glu Glu Lys Lys Lys Thr Thr Val
    50                  55                  60

gta tat ggc tta aaa gga aaa ctg gca gga gat gca ata tct agt caa      240
Val Tyr Gly Leu Lys Gly Lys Leu Ala Gly Asp Ala Ile Ser Ser Gln
65                  70                  75                  80

agt cca gat gat aat ttt acc att cga aat tac tca ttc aag tat gca      288
Ser Pro Asp Asp Asn Phe Thr Ile Arg Asn Tyr Ser Phe Lys Tyr Ala
                85                  90                  95

agc aac aag ttt tta ggg ttt gca gta gct att ggt tac tcg ata ggc      336
Ser Asn Lys Phe Leu Gly Phe Ala Val Ala Ile Gly Tyr Ser Ile Gly
            100                 105                 110

agt cca aga ata gaa gtt gag atg tct tat gaa gca ttt gat gtg aaa      384
Ser Pro Arg Ile Glu Val Glu Met Ser Tyr Glu Ala Phe Asp Val Lys
        115                 120                 125

aat cca ggt gat aat tac aaa aac ggt gct tac agg tat tgt gct tta      432
Asn Pro Gly Asp Asn Tyr Lys Asn Gly Ala Tyr Arg Tyr Cys Ala Leu
    130                 135                 140

tct cat caa gat gat gcg gat gat gac atg act agt gca act gac aaa      480
Ser His Gln Asp Asp Ala Asp Asp Asp Met Thr Ser Ala Thr Asp Lys
145                 150                 155                 160

ttt gta tat tta att aat gaa gga tta ctt aac ata tca ttt atg aca      528
Phe Val Tyr Leu Ile Asn Glu Gly Leu Leu Asn Ile Ser Phe Met Thr
                165                 170                 175

aac ata tgt tat gaa aca gca agc aaa aat ata cct ctc tct cct tac      576
Asn Ile Cys Tyr Glu Thr Ala Ser Lys Asn Ile Pro Leu Ser Pro Tyr
            180                 185                 190

ata tgt gca ggt att ggt act gat tta att cac atg ttt gaa act aca      624
Ile Cys Ala Gly Ile Gly Thr Asp Leu Ile His Met Phe Glu Thr Thr
        195                 200                 205

cat cct aaa att tct tat caa gga aag cta ggg ttg gcc tac ttc gta      672
His Pro Lys Ile Ser Tyr Gln Gly Lys Leu Gly Leu Ala Tyr Phe Val
    210                 215                 220

agt gca gag tct tcg gtt tct ttt ggt ata tat ttt cat aaa att ata      720
Ser Ala Glu Ser Ser Val Ser Phe Gly Ile Tyr Phe His Lys Ile Ile
225                 230                 235                 240

aat aat aag ttt aaa aat gtt cca gcc atg gta cct att aac tca gac      768
Asn Asn Lys Phe Lys Asn Val Pro Ala Met Val Pro Ile Asn Ser Asp
                245                 250                 255

gag ata gta gga cca cag ttt gca aca gta aca tta aat gta tgc tac 816
Glu Ile Val Gly Pro Gln Phe Ala Thr Val Thr Leu Asn Val Cys Tyr
            260                 265                 270

ttt gga tta gaa ctt gga tgt agg ttc aac ttc taa 852
Phe Gly Leu Glu Leu Gly Cys Arg Phe Asn Phe
        275                 280

<210> 40

<211> 283

<212> PRT

<213> Ehrlichia canis

<400> 40

Met Asn Cys Lys Lys Val Phe Thr Ile Ser Ala Leu Ile Ser Ser Ile
1               5                   10                  15

Tyr Phe Leu Pro Asn Val Ser Tyr Ser Asn Pro Val Tyr Gly Asn Ser
            20                  25                  30

Met Tyr Gly Asn Phe Tyr Ile Ser Gly Lys Tyr Met Pro Ser Val Pro
        35                  40                  45

His Phe Gly Ile Phe Ser Ala Glu Glu Glu Lys Lys Lys Thr Thr Val
    50                  55                  60

Val Tyr Gly Leu Lys Gly Lys Leu Ala Gly Asp Ala Ile Ser Ser Gln
65                  70                  75                  80

Ser Pro Asp Asp Asn Phe Thr Ile Arg Asn Tyr Ser Phe Lys Tyr Ala
                85                  90                  95

Ser Asn Lys Phe Leu Gly Phe Ala Val Ala Ile Gly Tyr Ser Ile Gly
            100                 105                 110

Ser Pro Arg Ile Glu Val Glu Met Ser Tyr Glu Ala Phe Asp Val Lys
        115                 120                 125

Asn Pro Gly Asp Asn Tyr Lys Asn Gly Ala Tyr Arg Tyr Cys Ala Leu
    130                 135                 140

Ser His Gln Asp Asp Ala Asp Asp Asp Met Thr Ser Ala Thr Asp Lys
145                 150                 155                 160

Phe Val Tyr Leu Ile Asn Glu Gly Leu Leu Asn Ile Ser Phe Met Thr
                165                 170                 175

Asn Ile Cys Tyr Glu Thr Ala Ser Lys Asn Ile Pro Leu Ser Pro Tyr
            180                 185                 190

Ile Cys Ala Gly Ile Gly Thr Asp Leu Ile His Met Phe Glu Thr Thr
        195                 200                 205

His Pro Lys Ile Ser Tyr Gln Gly Lys Leu Gly Leu Ala Tyr Phe Val
    210                 215                 220

Ser Ala Glu Ser Ser Val Ser Phe Gly Ile Tyr Phe His Lys Ile Ile
225                 230                 235                 240

Asn Asn Lys Phe Lys Asn Val Pro Ala Met Val Pro Ile Asn Ser Asp
                245                 250                 255

Glu Ile Val Gly Pro Gln Phe Ala Thr Val Thr Leu Asn Val Cys Tyr
            260                 265                 270

Phe Gly Leu Glu Leu Gly Cys Arg Phe Asn Phe
        275                 280

<210>  41

<211>  831

<212>  DNA

<213>  Ehrlichia canis

<220>

<221>  CDS

<222>  (1)..(831)

<400>  41
atg aac tgt aaa aaa ttt ctt ata aca act aca ttg gta tca cta aca        48
Met Asn Cys Lys Lys Phe Leu Ile Thr Thr Thr Leu Val Ser Leu Thr
1               5                   10                  15

att ctt tta cct ggc ata tct ttc tcc aaa cca ata cat gaa aac aat        96
Ile Leu Leu Pro Gly Ile Ser Phe Ser Lys Pro Ile His Glu Asn Asn
            20                  25                  30

act aca gga aac ttt tac att att gga aaa tat gta cca agt att tca       144
Thr Thr Gly Asn Phe Tyr Ile Ile Gly Lys Tyr Val Pro Ser Ile Ser
        35                  40                  45

cat ttt ggg aac ttt tca gct aaa gaa gaa aaa aac aca act act gga       192
His Phe Gly Asn Phe Ser Ala Lys Glu Glu Lys Asn Thr Thr Thr Gly
    50                  55                  60

att ttt gga tta aaa gaa tca tgg act ggt ggt atc atc ctt gat aaa       240
Ile Phe Gly Leu Lys Glu Ser Trp Thr Gly Gly Ile Ile Leu Asp Lys
65                  70                  75                  80

gaa cat gca gct ttt aat atc cca aat tat tca ttt aaa tat gaa aat       288
Glu His Ala Ala Phe Asn Ile Pro Asn Tyr Ser Phe Lys Tyr Glu Asn
                85                  90                  95

aat cca ttt tta gga ttt gca ggg gta att ggc tat tca ata ggt agt 336
Asn Pro Phe Leu Gly Phe Ala Gly Val Ile Gly Tyr Ser Ile Gly Ser
            100                 105                 110

cca aga ata gaa ttt gaa gta tca tac gag aca ttc gat gta caa aat 384
Pro Arg Ile Glu Phe Glu Val Ser Tyr Glu Thr Phe Asp Val Gln Asn
        115                 120                 125

cca gga gat aag ttt aac aat gat gca cat aag tat tgt gct tta tcc 432
Pro Gly Asp Lys Phe Asn Asn Asp Ala His Lys Tyr Cys Ala Leu Ser
    130                 135                 140

aat gat tcc agt aaa aca atg aaa agt ggt aaa ttc gtt ttt ctc aaa 480
Asn Asp Ser Ser Lys Thr Met Lys Ser Gly Lys Phe Val Phe Leu Lys
145                 150                 155                 160

aat gaa gga tta agt gac ata tca ctc atg tta aat gta tgt tat gat 528
Asn Glu Gly Leu Ser Asp Ile Ser Leu Met Leu Asn Val Cys Tyr Asp
                165                 170                 175

ata ata aac aaa aga atg cct ttt tca cct tac ata tgt gca ggc att 576
Ile Ile Asn Lys Arg Met Pro Phe Ser Pro Tyr Ile Cys Ala Gly Ile
            180                 185                 190

ggt act gac tta ata ttc atg ttt gac gct ata aac cat aaa gct gct 624
Gly Thr Asp Leu Ile Phe Met Phe Asp Ala Ile Asn His Lys Ala Ala
        195                 200                 205

tat caa gga aaa tta ggt ttt aat tat cca ata agc cca gaa gct aac 672
Tyr Gln Gly Lys Leu Gly Phe Asn Tyr Pro Ile Ser Pro Glu Ala Asn
    210                 215                 220

att tct atg ggt gtg cac ttt cac aaa gta aca aac aac gag ttt aga 720
Ile Ser Met Gly Val His Phe His Lys Val Thr Asn Asn Glu Phe Arg
225                 230                 235                 240

gtt cct gtt cta tta act gct gga gga ctc gct cca gat aat cta ttt 768
Val Pro Val Leu Leu Thr Ala Gly Gly Leu Ala Pro Asp Asn Leu Phe
                245                 250                 255

gca ata gta aag ttg agt ata tgt cat ttt ggg tta gaa ttt ggg tac 816
Ala Ile Val Lys Leu Ser Ile Cys His Phe Gly Leu Glu Phe Gly Tyr
            260                 265                 270

agg gtc agt ttt taa 831
Arg Val Ser Phe
        275

<210> 42

<211> 276

<212> PRT

<213> Ehrlichia canis

<400> 42

Met Asn Cys Lys Lys Phe Leu Ile Thr Thr Thr Leu Val Ser Leu Thr
1               5                   10                  15

Ile Leu Leu Pro Gly Ile Ser Phe Ser Lys Pro Ile His Glu Asn Asn
            20                  25                  30

Thr Thr Gly Asn Phe Tyr Ile Ile Gly Lys Tyr Val Pro Ser Ile Ser
        35                  40                  45

His Phe Gly Asn Phe Ser Ala Lys Glu Glu Lys Asn Thr Thr Thr Gly
    50                  55                  60

Ile Phe Gly Leu Lys Glu Ser Trp Thr Gly Gly Ile Ile Leu Asp Lys
65                  70                  75                  80

Glu His Ala Ala Phe Asn Ile Pro Asn Tyr Ser Phe Lys Tyr Glu Asn
                85                  90                  95

Asn Pro Phe Leu Gly Phe Ala Gly Val Ile Gly Tyr Ser Ile Gly Ser
            100                 105                 110

Pro Arg Ile Glu Phe Glu Val Ser Tyr Glu Thr Phe Asp Val Gln Asn
        115                 120                 125

Pro Gly Asp Lys Phe Asn Asn Asp Ala His Lys Tyr Cys Ala Leu Ser
    130                 135                 140

Asn Asp Ser Ser Lys Thr Met Lys Ser Gly Lys Phe Val Phe Leu Lys
145                 150                 155                 160

Asn Glu Gly Leu Ser Asp Ile Ser Leu Met Leu Asn Val Cys Tyr Asp
                165                 170                 175

Ile Ile Asn Lys Arg Met Pro Phe Ser Pro Tyr Ile Cys Ala Gly Ile
            180                 185                 190

Gly Thr Asp Leu Ile Phe Met Phe Asp Ala Ile Asn His Lys Ala Ala
        195                 200                 205

Tyr Gln Gly Lys Leu Gly Phe Asn Tyr Pro Ile Ser Pro Glu Ala Asn
    210                 215                 220

Ile Ser Met Gly Val His Phe His Lys Val Thr Asn Asn Glu Phe Arg
225                 230                 235                 240

Val Pro Val Leu Leu Thr Ala Gly Gly Leu Ala Pro Asp Asn Leu Phe
                245                 250                 255

Ala Ile Val Lys Leu Ser Ile Cys His Phe Gly Leu Glu Phe Gly Tyr
            260                 265                 270

Arg Val Ser Phe
        275

<210>  43

<211>  882

<212>  DNA

<213>  Ehrlichia canis



<220>

<221>  CDS

<222>  (1)..(882)



<400>  43
atg aat aat aaa ctc aaa ttt act ata ata aac aca gta tta gta tgc        48
Met Asn Asn Lys Leu Lys Phe Thr Ile Ile Asn Thr Val Leu Val Cys
1               5                   10                  15

tta ttg tca tta cct aat ata tct tcc tca aag gcc ata aac aat aac        96
Leu Leu Ser Leu Pro Asn Ile Ser Ser Ser Lys Ala Ile Asn Asn Asn
            20                  25                  30

gct aaa aag tac tac gga tta tat atc agt gga caa tat aaa ccc agt       144
Ala Lys Lys Tyr Tyr Gly Leu Tyr Ile Ser Gly Gln Tyr Lys Pro Ser
        35                  40                  45

gtt tct gtt ttc agt aat ttt tca gtt aaa gaa acc aat gtc ata act       192
Val Ser Val Phe Ser Asn Phe Ser Val Lys Glu Thr Asn Val Ile Thr
    50                  55                  60

aaa aac ctt ata gct tta aaa aaa gat gtt gac tct att gaa acc aag       240
Lys Asn Leu Ile Ala Leu Lys Lys Asp Val Asp Ser Ile Glu Thr Lys
65                  70                  75                  80

act gat gcc agt gta ggt att agt aac cca tca aat ttt act atc ccc       288
Thr Asp Ala Ser Val Gly Ile Ser Asn Pro Ser Asn Phe Thr Ile Pro
                85                  90                  95

tat aca gct gta ttt caa gat aat tct gtc aat ttc aat gga act att       336
Tyr Thr Ala Val Phe Gln Asp Asn Ser Val Asn Phe Asn Gly Thr Ile
            100                 105                 110

ggt tac acc ttt gct gaa ggt aca aga gtt gaa ata gaa ggt tct tat       384
Gly Tyr Thr Phe Ala Glu Gly Thr Arg Val Glu Ile Glu Gly Ser Tyr
        115                 120                 125

gag gaa ttt gat gtt aaa aac cct gga ggc tat aca cta agt gat gcc       432
Glu Glu Phe Asp Val Lys Asn Pro Gly Gly Tyr Thr Leu Ser Asp Ala
    130                 135                 140

tat cgc tat ttt gca tta gca cgt gaa atg aaa ggt aat agt ttt aca       480
Tyr Arg Tyr Phe Ala Leu Ala Arg Glu Met Lys Gly Asn Ser Phe Thr
145                 150                 155                 160

cct aaa gaa aaa gtt tct aat agt ttt ttt cac act gta atg aga aat       528
Pro Lys Glu Lys Val Ser Asn Ser Phe Phe His Thr Val Met Arg Asn
                165                 170                 175

gat gga tta tct ata ata tct gtt ata gta aat gtt tgc tac gat ttc       576
Asp Gly Leu Ser Ile Ile Ser Val Ile Val Asn Val Cys Tyr Asp Phe
            180                 185                 190

tct ttg aac aat ttg tca ata tcg cct tac ata tgt gga gga gca ggg       624
Ser Leu Asn Asn Leu Ser Ile Ser Pro Tyr Ile Cys Gly Gly Ala Gly
        195                 200                 205

gta gat gct ata gaa ttc ttc gat gta tta cac att aag ttt gca tat          672
Val Asp Ala Ile Glu Phe Phe Asp Val Leu His Ile Lys Phe Ala Tyr
    210                 215                 220

caa agc aag cta ggt att gct tat tct cta cca tct aac att agt ctc          720
Gln Ser Lys Leu Gly Ile Ala Tyr Ser Leu Pro Ser Asn Ile Ser Leu
225                 230                 235                 240

ttt gct agt tta tat tac cat aaa gta atg ggc aat caa ttt aaa aat          768
Phe Ala Ser Leu Tyr Tyr His Lys Val Met Gly Asn Gln Phe Lys Asn
                245                 250                 255

tta aat gtc caa gat gtt gct gaa ctt gca agt ata cct aaa att aca          816
Leu Asn Val Gln Asp Val Ala Glu Leu Ala Ser Ile Pro Lys Ile Thr
            260                 265                 270

tcc gca gtt gct aca ctt aat att ggt tat ttt gga ggt gaa att ggt          864
Ser Ala Val Ala Thr Leu Asn Ile Gly Tyr Phe Gly Gly Glu Ile Gly
        275                 280                 285

gca aga ttg aca ttt taa                                                  882
Ala Arg Leu Thr Phe
    290

<210>  44

<211>  293

<212>  PRT

<213>  Ehrlichia canis

<400>  44

Met Asn Asn Lys Leu Lys Phe Thr Ile Ile Asn Thr Val Leu Val Cys
1               5                   10                  15

Leu Leu Ser Leu Pro Asn Ile Ser Ser Ser Lys Ala Ile Asn Asn Asn
            20                  25                  30

Ala Lys Lys Tyr Tyr Gly Leu Tyr Ile Ser Gly Gln Tyr Lys Pro Ser
        35                  40                  45

Val Ser Val Phe Ser Asn Phe Ser Val Lys Glu Thr Asn Val Ile Thr
    50                  55                  60

Lys Asn Leu Ile Ala Leu Lys Lys Asp Val Asp Ser Ile Glu Thr Lys
65                  70                  75                  80

Thr Asp Ala Ser Val Gly Ile Ser Asn Pro Ser Asn Phe Thr Ile Pro
                85                  90                  95

Tyr Thr Ala Val Phe Gln Asp Asn Ser Val Asn Phe Asn Gly Thr Ile
            100                 105                 110

Gly Tyr Thr Phe Ala Glu Gly Thr Arg Val Glu Ile Glu Gly Ser Tyr
        115                 120                 125

Glu Glu Phe Asp Val Lys Asn Pro Gly Gly Tyr Thr Leu Ser Asp Ala
    130                 135                 140

Tyr Arg Tyr Phe Ala Leu Ala Arg Glu Met Lys Gly Asn Ser Phe Thr
145                 150                 155                 160

Pro Lys Glu Lys Val Ser Asn Ser Phe Phe His Thr Val Met Arg Asn
                165                 170                 175

Asp Gly Leu Ser Ile Ile Ser Val Ile Val Asn Val Cys Tyr Asp Phe
            180                 185                 190

Ser Leu Asn Asn Leu Ser Ile Ser Pro Tyr Ile Cys Gly Gly Ala Gly
        195                 200                 205

Val Asp Ala Ile Glu Phe Phe Asp Val Leu His Ile Lys Phe Ala Tyr
    210                 215                 220

Gln Ser Lys Leu Gly Ile Ala Tyr Ser Leu Pro Ser Asn Ile Ser Leu
225                 230                 235                 240

Phe Ala Ser Leu Tyr Tyr His Lys Val Met Gly Asn Gln Phe Lys Asn
                245                 250                 255

Leu Asn Val Gln Asp Val Ala Glu Leu Ala Ser Ile Pro Lys Ile Thr
            260                 265                 270

Ser Ala Val Ala Thr Leu Asn Ile Gly Tyr Phe Gly Gly Glu Ile Gly
        275                 280                 285

Ala Arg Leu Thr Phe
    290

<210>  45

<211>  900

<212>  DNA

<213>  Ehrlichia canis


<220>

<221>  CDS

<222>  (1)..(900)


<400>  45
atg aat agc aag agt aag ttc ttt aca ata tgt aca tcg tta ata tgc        48
Met Asn Ser Lys Ser Lys Phe Phe Thr Ile Cys Thr Ser Leu Ile Cys
1               5                   10                  15

tta tta tca tca cct aac aca tct ctc tca aac ttc ata ggc aat agt 96
Leu Leu Ser Ser Pro Asn Thr Ser Leu Ser Asn Phe Ile Gly Asn Ser
            20                  25                  30

aca aaa cat tct gga tta tat gtt agc gga cat tat aag ccc agc gtt 144
Thr Lys His Ser Gly Leu Tyr Val Ser Gly His Tyr Lys Pro Ser Val
        35                  40                  45

tcc att ttt agc aaa ttt tca gta aaa gaa aca aat aca cat aca gta 192
Ser Ile Phe Ser Lys Phe Ser Val Lys Glu Thr Asn Thr His Thr Val
    50                  55                  60

cag tta gta gct ctt aaa aaa gat gtt aat tct att tct atg aac atc 240
Gln Leu Val Ala Leu Lys Lys Asp Val Asn Ser Ile Ser Met Asn Ile
65                  70                  75                  80

agt aat ggt gct aca ggc att agc aaa gca aca aat ttt aat ctt cct 288
Ser Asn Gly Ala Thr Gly Ile Ser Lys Ala Thr Asn Phe Asn Leu Pro
                85                  90                  95

tat gtt gca gaa ttt caa gac aat gcc ttc aac ttc agt gga gct att 336
Tyr Val Ala Glu Phe Gln Asp Asn Ala Phe Asn Phe Ser Gly Ala Ile
            100                 105                 110

ggt tat tca ctt ttt gaa caa cta aac att gaa gtt gaa ggt tct tat 384
Gly Tyr Ser Leu Phe Glu Gln Leu Asn Ile Glu Val Glu Gly Ser Tyr
        115                 120                 125

gaa gaa ttc gat gcc aaa aat cct ggt ggt tat att tta aat gat gca 432
Glu Glu Phe Asp Ala Lys Asn Pro Gly Gly Tyr Ile Leu Asn Asp Ala
    130                 135                 140

ttc cgc tat ttt gca ttg gca cgt gaa atg gga caa gaa aaa aat gat 480
Phe Arg Tyr Phe Ala Leu Ala Arg Glu Met Gly Gln Glu Lys Asn Asp
145                 150                 155                 160

aat aag cat ctt agt cct aag gag gag cat gat ata agt aaa aca tat 528
Asn Lys His Leu Ser Pro Lys Glu Glu His Asp Ile Ser Lys Thr Tyr
                165                 170                 175

tac aca gtc atg aga aat aat ggg tta tct ata tta tct att atg ata 576
Tyr Thr Val Met Arg Asn Asn Gly Leu Ser Ile Leu Ser Ile Met Ile
            180                 185                 190

aat ggc tgc tat aat cta cct ctc aat gat tta tca ata tca cct tat 624
Asn Gly Cys Tyr Asn Leu Pro Leu Asn Asp Leu Ser Ile Ser Pro Tyr
        195                 200                 205

ttt tgt aca gga ata ggt gta gat gct ata gaa ttt ttt gat gca ctg 672
Phe Cys Thr Gly Ile Gly Val Asp Ala Ile Glu Phe Phe Asp Ala Leu
    210                 215                 220

cat ctt aaa ctt gct ttg caa agt aaa ata gga gct act tac caa tta 720
His Leu Lys Leu Ala Leu Gln Ser Lys Ile Gly Ala Thr Tyr Gln Leu
225                 230                 235                 240

tca gac aac att agt tta ttt aca aat gga tat atc cat caa gta ata 768
Ser Asp Asn Ile Ser Leu Phe Thr Asn Gly Tyr Ile His Gln Val Ile
                245                 250                 255

ggt gat caa ttt aaa aac tta aaa gtc caa tat ata ggt gaa ctt aaa 816
Gly Asp Gln Phe Lys Asn Leu Lys Val Gln Tyr Ile Gly Glu Leu Lys
            260                 265                 270

gag aac ccg aaa att aca tct gca gtt gct act ctc aat gtt gga tac 864
Glu Asn Pro Lys Ile Thr Ser Ala Val Ala Thr Leu Asn Val Gly Tyr
        275                 280                 285

ttt gga ggt gaa att gga gta aga ctc aca ctt taa                                900
Phe Gly Gly Glu Ile Gly Val Arg Leu Thr Leu
    290                 295

<210>  46

<211>  299

<212>  PRT

<213>  Ehrlichia canis

<400>  46

Met Asn Ser Lys Ser Lys Phe Phe Thr Ile Cys Thr Ser Leu Ile Cys
1               5                   10                  15

Leu Leu Ser Ser Pro Asn Thr Ser Leu Ser Asn Phe Ile Gly Asn Ser
            20                  25                  30

Thr Lys His Ser Gly Leu Tyr Val Ser Gly His Tyr Lys Pro Ser Val
        35                  40                  45

Ser Ile Phe Ser Lys Phe Ser Val Lys Glu Thr Asn Thr His Thr Val
    50                  55                  60

Gln Leu Val Ala Leu Lys Lys Asp Val Asn Ser Ile Ser Met Asn Ile
65                  70                  75                  80

Ser Asn Gly Ala Thr Gly Ile Ser Lys Ala Thr Asn Phe Asn Leu Pro
                85                  90                  95

Tyr Val Ala Glu Phe Gln Asp Asn Ala Phe Asn Phe Ser Gly Ala Ile
            100                 105                 110

Gly Tyr Ser Leu Phe Glu Gln Leu Asn Ile Glu Val Glu Gly Ser Tyr
        115                 120                 125

Glu Glu Phe Asp Ala Lys Asn Pro Gly Gly Tyr Ile Leu Asn Asp Ala
    130                 135                 140

Phe Arg Tyr Phe Ala Leu Ala Arg Glu Met Gly Gln Glu Lys Asn Asp
145                 150                 155                 160

Asn Lys His Leu Ser Pro Lys Glu Glu His Asp Ile Ser Lys Thr Tyr
                165                 170                 175

Tyr Thr Val Met Arg Asn Asn Gly Leu Ser Ile Leu Ser Ile Met Ile
            180                 185                 190

Asn Gly Cys Tyr Asn Leu Pro Leu Asn Asp Leu Ser Ile Ser Pro Tyr
        195                 200                 205

Phe Cys Thr Gly Ile Gly Val Asp Ala Ile Glu Phe Phe Asp Ala Leu
    210                 215                 220

His Leu Lys Leu Ala Leu Gln Ser Lys Ile Gly Ala Thr Tyr Gln Leu
225                 230                 235                 240

Ser Asp Asn Ile Ser Leu Phe Thr Asn Gly Tyr Ile His Gln Val Ile
                245                 250                 255

Gly Asp Gln Phe Lys Asn Leu Lys Val Gln Tyr Ile Gly Glu Leu Lys
            260                 265                 270

Glu Asn Pro Lys Ile Thr Ser Ala Val Ala Thr Leu Asn Val Gly Tyr
        275                 280                 285

Phe Gly Gly Glu Ile Gly Val Arg Leu Thr Leu
    290                 295

<210>  47

<211>  495

<212>  DNA

<213>  Ehrlichia canis


<220>

<221>  CDS

<222>  (1)..(495)


<400>  47
atg aat tat aag aaa att cta gta aga agc gcg tta atc tca tta atg        48
Met Asn Tyr Lys Lys Ile Leu Val Arg Ser Ala Leu Ile Ser Leu Met
1               5                   10                  15

tca atc tta cca tat cag ttc ttt gca gat cct cta ggt tca aga act        96
Ser Ile Leu Pro Tyr Gln Phe Phe Ala Asp Pro Leu Gly Ser Arg Thr
            20                  25                  30

aat gat aac aaa gaa ggc ttc tac att agt gca aag tac aat cca agt       144
Asn Asp Asn Lys Glu Gly Phe Tyr Ile Ser Ala Lys Tyr Asn Pro Ser
        35                  40                  45

ata tca cac ttt aga aaa ttc tct gct gaa gaa act cct att aat gga       192
Ile Ser His Phe Arg Lys Phe Ser Ala Glu Glu Thr Pro Ile Asn Gly
    50                  55                  60

aca aat tct ctc act aaa aaa gtt ttc gga cta aag aaa gat ggt gat       240
Thr Asn Ser Leu Thr Lys Lys Val Phe Gly Leu Lys Lys Asp Gly Asp
65                  70                  75                  80

ata aca aaa aaa gac gat ttt aca aga gta gct cca ggc att gat ttt       288
Ile Thr Lys Lys Asp Asp Phe Thr Arg Val Ala Pro Gly Ile Asp Phe
                85                  90                  95

caa aat aac tta ata tca gga ttt tca gga agt att ggt tac tct atg          336
Gln Asn Asn Leu Ile Ser Gly Phe Ser Gly Ser Ile Gly Tyr Ser Met
            100                 105                 110

gac gga cca aga ata gaa ctt gaa gct gca tat cac att tta atc caa          384
Asp Gly Pro Arg Ile Glu Leu Glu Ala Ala Tyr His Ile Leu Ile Gln
        115                 120                 125

aaa cac gat aac aat gat act gat aat ggt gaa tac tat aaa cat ttt          432
Lys His Asp Asn Asn Asp Thr Asp Asn Gly Glu Tyr Tyr Lys His Phe
    130                 135                 140

gca tat ctc gta aag atg cca tgg aag atc agc cat atg ttg ttc tta          480
Ala Tyr Leu Val Lys Met Pro Trp Lys Ile Ser His Met Leu Phe Leu
145                 150                 155                 160

aaa atg acg gca tac                                                      495
Lys Met Thr Ala Tyr
                165

<210>  48

<211>  165

<212>  PRT

<213>  Ehrlichia canis

<400>  48

Met Asn Tyr Lys Lys Ile Leu Val Arg Ser Ala Leu Ile Ser Leu Met
1               5                   10                  15

Ser Ile Leu Pro Tyr Gln Phe Phe Ala Asp Pro Leu Gly Ser Arg Thr
            20                  25                  30

Asn Asp Asn Lys Glu Gly Phe Tyr Ile Ser Ala Lys Tyr Asn Pro Ser
        35                  40                  45

Ile Ser His Phe Arg Lys Phe Ser Ala Glu Glu Thr Pro Ile Asn Gly
    50                  55                  60

Thr Asn Ser Leu Thr Lys Lys Val Phe Gly Leu Lys Lys Asp Gly Asp
65                  70                  75                  80

Ile Thr Lys Lys Asp Asp Phe Thr Arg Val Ala Pro Gly Ile Asp Phe
                85                  90                  95

Gln Asn Asn Leu Ile Ser Gly Phe Ser Gly Ser Ile Gly Tyr Ser Met
            100                 105                 110

Asp Gly Pro Arg Ile Glu Leu Glu Ala Ala Tyr His Ile Leu Ile Gln
        115                 120                 125

Lys His Asp Asn Asn Asp Thr Asp Asn Gly Glu Tyr Tyr Lys His Phe
    130                 135                 140

Ala Tyr Leu Val Lys Met Pro Trp Lys Ile Ser His Met Leu Phe Leu
145                 150                 155                 160

Lys Met Thr Ala Tyr
                165

<210>  49

<211>  34

<212>  DNA

<213>  Artificial/Unknown

<220>

<221>  misc_feature

<222>  ()..()

<223>  Reverse primer

<220>

<221>  misc_feature

<222>  17, 26

<223>  n= a/t/g/c

<400>  49

cgggatccga attcggnath aayggnaayt tyta                                34

<210>  50

<211>  30

<212>  DNA

<213>  Artificial/Unknown

<220>

<221>  misc_feature

<222>  ()..()

<223>  Reverse primer

<400>  50
agcggccgct taraayasra aycttsctcc                                        30

<210>  51

<211>  756

<212>  DNA

<213>  Ehrlichia chaffeensis

<400>  51
ggcataaatg ggaatttcta catcagtgga aaatacatgc caagtgcttc gcattttgga      60

gtattctctg ctaaggaaga aagaaataca acagttggag tgtttggact gaagcaaaat     120

tgggacggaa gcgcaatatc caactcctcc ccaaacgatg tattcactgt ctcaaattat     180

tcatttaaat atgaaaacaa cccgttttta ggttttgcag gagctattgg ttactcaatg     240

gatggtccaa gaatagagct tgaagtatct tatgaaacat ttgatgtaaa aaatcaaggt     300

aacaattata agaatgaagc acatagatat tgtgctctat cccataactc agcagcagac     360

atgagtagtg caagtaataa ttttgtcttt ctaaaaaatg aaggattact tgacatatca     420

tttatgctga acgcatgcta tgacgtagta ggcgaaggca tacctttttc tccttatata     480

tgcgcaggta tcggtactga tttagtatcc atgtttgaag ctacaaatcc taaaatttct     540

taccaaggaa agttaggttt aagctactct ataagcccag aagcttctgt gtttattggt     600

gggcactttc ataaggtaat agggaacgaa tttagagata ttcctactat aatacctact     660

ggatcaacac ttgcaggaaa aggaaactac cctgcaatag taatactgga tgtatgccac     720

tttggaatag aacttggagg aaggtttgta ttctaa                               756

<210>  52

<211>  256

<212>  PRT

<213>  Ehrlichia chaffeensis

<400>  52

Asp Pro Ala Gly Ser Gly Ile Asn Gly Asn Phe Tyr Ile Ser Gly Lys
1               5                   10                  15

Tyr Met Pro Ser Ala Ser His Phe Gly Val Phe Ser Ala Lys Glu Glu
            20                  25                  30

Arg Asn Thr Thr Val Gly Val Phe Gly Leu Lys Gln Asn Trp Asp Gly
        35                  40                  45

Ser Ala Ile Ser Asn Ser Ser Pro Asn Asp Val Phe Thr Val Ser Asn

50                  55                  60

Tyr Ser Phe Lys Tyr Glu Asn Asn Pro Phe Leu Gly Phe Ala Gly Ala
65                  70                  75                  80

Ile Gly Tyr Ser Met Asp Gly Pro Arg Ile Glu Leu Glu Val Ser Tyr
                85                  90                  95

Glu Thr Phe Asp Val Lys Asn Gln Gly Asn Asn Tyr Lys Asn Glu Ala
            100                 105                 110

His Arg Tyr Cys Ala Leu Ser His Asn Ser Ala Ala Asp Met Ser Ser
        115                 120                 125

Ala Ser Asn Asn Phe Val Phe Leu Lys Asn Glu Gly Leu Leu Asp Ile
    130                 135                 140

Ser Phe Met Leu Asn Ala Cys Tyr Asp Val Val Gly Glu Gly Ile Pro
145                 150                 155                 160

Phe Ser Pro Tyr Ile Cys Ala Gly Ile Gly Thr Asp Leu Val Ser Met
                165                 170                 175

Phe Glu Ala Thr Asn Pro Lys Ile Ser Tyr Gln Gly Lys Leu Gly Leu
            180                 185                 190

Ser Tyr Ser Ile Ser Pro Glu Ala Ser Val Phe Ile Gly Gly His Phe
        195                 200                 205

His Lys Val Ile Gly Asn Glu Phe Arg Asp Ile Pro Thr Ile Ile Pro
    210                 215                 220

Thr Gly Ser Thr Leu Ala Gly Lys Gly Asn Tyr Pro Ala Ile Val Ile
225                 230                 235                 240

Leu Asp Val Cys His Phe Gly Ile Glu Leu Gly Gly Arg Phe Val Phe
                245                 250                 255

<210>  53

<211>  817

<212>  DNA

<213>  Ehrlichia canis


<400>  53
tagcagcact aaaaaacagt ttgggttata tgttagtgga caacaccagc ctagtgtttc      60

tatttttagc aatttctcag taaaggaaac taattttcct acaaagtatt ctagcagctt     120

cttaaaaaaa gacattaatt ctgtcgaatt tgacgatagt gttactgctg gcattagtta     180

cccacttaat ttcagtactc cttatatagc tgtatttcaa gataatattt ctaattttaa     240

tggcgctatt gggtacactt ttgttgaagg cccaagaatt gaaatagaag gttcttatga     300

agaattcgat gtcaaagacc ctggaagata tacagaaata caagatgcat accgttactt     360

tgctttagca cgtgatatag actctattcc tactagccca aaaaatagaa cttcacatga     420

tggcaacagt tcatataagg tataccacac tgtaatgaaa aatgaaggac tatctataat     480

atccattatg gtcaatggct gctatgattt ttcttcagat aatttatcaa tattacctta     540

tgtatgtggt ggtataggtg taaatgctat agagtttttc gatgcattac atgttaaatt     600

cgcgtgtcag ggtaaattag gtattactta tccattatct tccaacgtta gtttatttgc 660

tggtggatat tatcaccaag taatgggcaa ccaatttaaa aatctaaatg ttcaacatgt 720

agctgaactt aatgacgcac ccaaagttac atctgcagta gctacacttg acattgggta 780

ttttggtggt gaaattggag caaggcttat attttaa 817

<210> 54

<211> 271

<212> PRT

<213> Ehrlichia canis

<400> 54

Ser Ser Thr Lys Lys Gln Phe Gly Leu Tyr Val Ser Gly Gln His Gln
1               5                   10                  15

Pro Ser Val Ser Ile Phe Ser Asn Phe Ser Val Lys Glu Thr Asn Phe
            20                  25                  30

Pro Thr Lys Tyr Ser Ser Ser Phe Leu Lys Lys Asp Ile Asn Ser Val
        35                  40                  45

Glu Phe Asp Asp Ser Val Thr Ala Gly Ile Ser Tyr Pro Leu Asn Phe
    50                  55                  60

Ser Thr Pro Tyr Ile Ala Val Phe Gln Asp Asn Ile Ser Asn Phe Asn
65                  70                  75                  80

Gly Ala Ile Gly Tyr Thr Phe Val Glu Gly Pro Arg Ile Glu Ile Glu
                85                  90                  95

Gly Ser Tyr Glu Glu Phe Asp Val Lys Asp Pro Gly Arg Tyr Thr Glu
            100                 105                 110

Ile Gln Asp Ala Tyr Arg Tyr Phe Ala Leu Ala Arg Asp Ile Asp Ser
        115                 120                 125

Ile Pro Thr Ser Pro Lys Asn Arg Thr Ser His Asp Gly Asn Ser Ser
    130                 135                 140

Tyr Lys Val Tyr His Thr Val Met Lys Asn Glu Gly Leu Ser Ile Ile
145                 150                 155                 160

Ser Ile Met Val Asn Gly Cys Tyr Asp Phe Ser Ser Asp Asn Leu Ser
                165                 170                 175

Ile Leu Pro Tyr Val Cys Gly Gly Ile Gly Val Asn Ala Ile Glu Phe
            180                 185                 190

Phe Asp Ala Leu His Val Lys Phe Ala Cys Gln Gly Lys Leu Gly Ile
        195                 200                 205

Thr Tyr Pro Leu Ser Ser Asn Val Ser Leu Phe Ala Gly Gly Tyr Tyr
    210                 215                 220

His Gln Val Met Gly Asn Gln Phe Lys Asn Leu Asn Val Gln His Val
225                 230                 235                 240

Ala Glu Leu Asn Asp Ala Pro Lys Val Thr Ser Ala Val Ala Thr Leu

245                 250                 255

Asp Ile Gly Tyr Phe Gly Gly Glu Ile Gly Ala Arg Leu Ile Phe
            260                 265                 270

<210>  55

<211>  882

<212>  DNA

<213>  Ehrlichia canis

<400>  55
atggcaaatt ttatgtacaa aaaatacaaa ctaatgacag caggtgtagt attatttcac      60

atgttatttc tacctcatgt ttctttcgca aaaaatacaa acagcaataa acttggatta     120

tacatcagtg gacagtataa ccctagtgtt tctgttttta gcaatttttc agcaaaagaa     180

accaatgttc atacagtaca actcatggcg cttaaaaaag acattgattc tattgaagtt     240

gatactggaa atagcgcagg tattagcaaa ccacaaaatt tcacagttct ttatactcca     300

aaatttcaag ataatgttgc tggtcttagc ggtgcacttg gattctttta ttctaaagga     360

ttaaggattg aaatggggtt ttcttatgaa aaatttgatg ctaaagacct tggtgagtac     420

accaaaataa aagatgctta tagatatttt gctctagtac gtgaaatgca tgttagtctc     480

atttatccaa aagataataa cacaggaaca cattatactg ttatgagaaa tgatggtata     540

tctatttctt ctgctacagt aaatggctgc tatgattctt ttttccagtt tatctttgtc     600

acctatatgt gtataggcat cggtatagat gctatagaat ttcttaatgc atacatatta     660

agtttgcttg ccaaggtagt taaggtgtta acttattctg tatctcccaa tgttaattta     720

tttgcagatg gatattatca taaagtgatg ggcaataaat ttaaaaattt acctgttcaa     780

tacgttaata ctttagaaga gtatccaaga gttacatctg caattgctac acttgatatt     840

ggctacctcg gtggtgaaat tggcataaga tttatatttt aa                        882

<210>  56

<211>  289

<212>  PRT

<213>  Ehrlichia canis

<400>  56

Met Tyr Lys Lys Tyr Lys Leu Met Thr Ala Gly Val Val Leu Phe His
1               5                   10                  15

Met Leu Phe Leu Pro His Val Ser Phe Ala Lys Asn Thr Asn Ser Asn
            20                  25                  30

Lys Leu Gly Leu Tyr Ile Ser Gly Gln Tyr Asn Pro Ser Val Ser Val
        35                  40                  45

Phe Ser Asn Phe Ser Ala Lys Glu Thr Asn Val His Thr Val Gln Leu

50                  55                  60

Met Ala Leu Lys Lys Asp Ile Asp Ser Ile Glu Val Asp Thr Gly Asn
65                  70                  75                  80

Ser Ala Gly Ile Ser Lys Pro Gln Asn Phe Thr Val Leu Tyr Thr Pro
                85                  90                  95

Lys Phe Gln Asp Asn Val Ala Gly Leu Ser Gly Ala Leu Gly Phe Phe
            100                 105                 110

Tyr Ser Lys Gly Leu Arg Ile Glu Met Gly Phe Ser Tyr Glu Lys Phe
        115                 120                 125

Asp Ala Lys Asp Leu Gly Glu Tyr Thr Lys Ile Lys Asp Ala Tyr Arg
    130                 135                 140

Tyr Phe Ala Leu Val Arg Glu Met His Val Ser Leu Ile Tyr Pro Lys
145                 150                 155                 160

Asp Asn Asn Thr Gly Thr His Tyr Thr Val Met Arg Asn Asp Gly Ile
                165                 170                 175

Ser Ile Ser Ser Ala Thr Val Asn Gly Cys Tyr Asp Ser Phe Phe Gln
            180                 185                 190

Phe Ile Phe Val Thr Tyr Met Cys Ile Gly Ile Gly Ile Asp Ala Ile
        195                 200                 205

Glu Phe Leu Asn Ala Tyr Ile Leu Ser Leu Leu Ala Lys Val Val Lys
    210                 215                 220

Val Leu Thr Tyr Ser Val Ser Pro Asn Val Asn Leu Phe Ala Asp Gly
225                 230                 235                 240

Tyr Tyr His Lys Val Met Gly Asn Lys Phe Lys Asn Leu Pro Val Gln
                245                 250                 255

Tyr Val Asn Thr Leu Glu Glu Tyr Pro Arg Val Thr Ser Ala Ile Ala
            260                 265                 270

Thr Leu Asp Ile Gly Tyr Leu Gly Gly Glu Ile Gly Ile Arg Phe Ile
        275                 280                 285

Phe

<210>  57

<211>  891

<212>  DNA

<213>  Ehrlichia canis

<400>  57
atgggaaatt ctatgaataa taaaagtcaa ttcttaataa gatttatatt tttaacatgc      60

atgctgtcat tacctaatat atctctttca aaagtaaata acgaaaaaca ttctggtttg     120

tatattagcg ggcaatacaa acccagtgtt tctgttttca gtaatttttc agttaaagaa     180

accaactttc atacaaaaca tctcatagct cttaaacaag atgttgattc tgttgaaatt     240

gatactggta gtaatacagc aggtattagt aacccatcta actttacaat cccttatact     300

gcagaatttc aagacaacca tactaactgc aatggctcta ttggttatgc ttttgctgaa 360

ggtccaagaa ttgaaataga attatcatat gaaaaatttg atgttaaaaa tcccacaggg 420

tatactacag taaaagatgc ttatagatac tttgctttag cacgtgaaat aaatatttct 480

ctattccaac caaaacaaaa agaaggtagt ggaatttacc atgtcgtaat gaaaaacgat 540

gggttatcta tcttatccaa tatagttaat atttgctacg atttttcttt aaataattta 600

cctatatcac cttatttatg cggaggaatg ggtataaatg ccatagaatt ctttgacgct 660

ttacatgtga aatttgctta tcaaagcaag gcaggaatta gttatcaact attacgtaaa 720

atcaacttat ttattgatgt atattactac gaagtaataa gtaataaatt taaaaacctg 780

aaagtccaac atgtacatga acttaaagat aatccaaaag tcacatctgc agttgctaca 840

cttgatatag catattttgg tagtgaagct ggcataagaa ttatatttta a 891

<210> 58

<211> 292

<212> PRT

<213> Ehrlichia canis

<400> 58

Met Asn Asn Lys Ser Gln Phe Leu Ile Arg Phe Ile Phe Leu Thr Cys
1               5                   10                  15

Met Leu Ser Leu Pro Asn Ile Ser Leu Ser Lys Val Asn Asn Glu Lys
            20                  25                  30

His Ser Gly Leu Tyr Ile Ser Gly Gln Tyr Lys Pro Ser Val Ser Val
        35                  40                  45

Phe Ser Asn Phe Ser Val Lys Glu Thr Asn Phe His Thr Lys His Leu
    50                  55                  60

Ile Ala Leu Lys Gln Asp Val Asp Ser Val Glu Ile Asp Thr Gly Ser
65                  70                  75                  80

Asn Thr Ala Gly Ile Ser Asn Pro Ser Asn Phe Thr Ile Pro Tyr Thr
                85                  90                  95

Ala Glu Phe Gln Asp Asn His Thr Asn Cys Asn Gly Ser Ile Gly Tyr
            100                 105                 110

Ala Phe Ala Glu Gly Pro Arg Ile Glu Ile Glu Leu Ser Tyr Glu Lys
        115                 120                 125

Phe Asp Val Lys Asn Pro Thr Gly Tyr Thr Thr Val Lys Asp Ala Tyr
    130                 135                 140

Arg Tyr Phe Ala Leu Ala Arg Glu Ile Asn Ile Ser Leu Phe Gln Pro
145                 150                 155                 160

Lys Gln Lys Glu Gly Ser Gly Ile Tyr His Val Val Met Lys Asn Asp
                165                 170                 175

Gly Leu Ser Ile Leu Ser Asn Ile Val Asn Ile Cys Tyr Asp Phe Ser
            180                 185                 190

Leu Asn Asn Leu Pro Ile Ser Pro Tyr Leu Cys Gly Gly Met Gly Ile
        195                 200                 205

Asn Ala Ile Glu Phe Phe Asp Ala Leu His Val Lys Phe Ala Tyr Gln
    210                 215                 220

Ser Lys Ala Gly Ile Ser Tyr Gln Leu Leu Arg Lys Ile Asn Leu Phe
225                 230                 235                 240

Ile Asp Val Tyr Tyr Tyr Glu Val Ile Ser Asn Lys Phe Lys Asn Leu
                245                 250                 255

Lys Val Gln His Val His Glu Leu Lys Asp Asn Pro Lys Val Thr Ser
            260                 265                 270

Ala Val Ala Thr Leu Asp Ile Ala Tyr Phe Gly Ser Glu Ala Gly Ile
        275                 280                 285

Arg Ile Ile Phe
    290

<210>  59

<211>  488

<212>  DNA

<213>  Ehrlichia canis


<400>  59
aagcttctta tgaagaattt gacgttaaaa atcctgaagg atctactaca gactcctata      60

gatatttcgc gttagcacgt ggcatggatg gtaataatat tcctacaagt caaaaattta     120

ctgtaatgag aaacgacggg ttattaatct catctgttat gataaatggc tgttacaatg     180

tcatactaaa tgatatacaa gcagaacctt acatatgtgc aggactagga ggagatttta     240

tagaattctt caatggcttt catgttaagc tagcttatca aggtaaagta ggcattagtt     300

atcaaatatt ccctgaagta agattattta ttgatggata ctaccataaa gtaaaaggca     360

acaagtttaa aaatttacac gttcaacatg taggtgcact tgcagcactc cctaaagtta     420

catctgcagt tgcaacactt aatattggat actttggttg tgaagctgga gtaagattca     480

tattttaa                                                              488

<210>  60

<211>  161

<212>  PRT

<213>  Ehrlichia canis


<400>  60

Ala Ser Tyr Glu Glu Phe Asp Val Lys Asn Pro Glu Gly Ser Thr Thr
1               5                   10                  15

Asp Ser Tyr Arg Tyr Phe Ala Leu Ala Arg Gly Met Asp Gly Asn Asn
            20                  25                  30

Ile Pro Thr Ser Gln Lys Phe Thr Val Met Arg Asn Asp Gly Leu Leu
        35                  40                  45

Ile Ser Ser Val Met Ile Asn Gly Cys Tyr Asn Val Ile Leu Asn Asp
    50                  55                  60

Ile Gln Ala Glu Pro Tyr Ile Cys Ala Gly Leu Gly Gly Asp Phe Ile
65                  70                  75                  80

Glu Phe Phe Asn Gly Phe His Val Lys Leu Ala Tyr Gln Gly Lys Val
                85                  90                  95

Gly Ile Ser Tyr Gln Ile Phe Pro Glu Val Arg Leu Phe Ile Asp Gly
            100                 105                 110

Tyr Tyr His Lys Val Lys Gly Asn Lys Phe Lys Asn Leu His Val Gln
        115                 120                 125

His Val Gly Ala Leu Ala Ala Leu Pro Lys Val Thr Ser Ala Val Ala
    130                 135                 140

Thr Leu Asn Ile Gly Tyr Phe Gly Cys Glu Ala Gly Val Arg Phe Ile
145                 150                 155                 160

Phe

<210>  61

<211>  18

<212>  PRT

<213>  Ehrlichia chaffeensis

<220>

<223>  N-terminal amino acid sequence of P28

<400>  61

Asp Pro Ala Gly Ser Gly Ile Asn Gly Asn Phe Tyr Ser Gly Lys Tyr
1               5                   10                  15

Met Pro

<210>  62

<211>  81

<212>  PRT

<213>  Ehrlichia chaffeensis

<400>  62

Glu Phe Phe Asp Ala Leu His Val Lys Phe Ala Tyr Gln Gly Lys Leu
1               5                   10                  15

Gly Ile Ser Tyr Gln Leu Phe Thr Lys Val Asn Leu Phe Leu Asp Gly
            20                  25                  30

Tyr Tyr His Gln Val Ile Gly Asn Gln Phe Lys Asn Leu Asn Val Asn

35                  40                  45

His Val Tyr Thr Leu Lys Glu Ser Pro Lys Val Thr Ser Ala Val Ala
    50                  55                  60

Thr Leu Asp Ile Ala Tyr Phe Gly Gly Glu Val Gly Ile Arg Phe Thr
65                  70                  75                  80

Phe

<210>  63

<211>  256

<212>  PRT

<213>  Ehrlichia chaffeensis

<400>  63

Asp Pro Ala Gly Ser Gly Ile Asn Gly Asn Phe Tyr Ile Ser Gly Lys
1               5                   10                  15

Tyr Met Pro Ser Ala Ser His Phe Gly Val Phe Ser Ala Lys Glu Glu
            20                  25                  30

Arg Asn Thr Thr Val Gly Val Phe Gly Leu Lys Gln Asn Trp Asp Gly
        35                  40                  45

Ser Ala Ile Ser Asn Ser Ser Pro Asn Asp Val Phe Thr Val Ser Asn
    50                  55                  60

Tyr Ser Phe Lys Tyr Glu Asn Asn Pro Phe Leu Gly Phe Ala Gly Ala
65                  70                  75                  80

Ile Gly Tyr Ser Met Asp Gly Pro Arg Ile Glu Leu Glu Val Ser Tyr
                85                  90                  95

Glu Thr Phe Asp Val Lys Asn Gln Gly Asn Asn Tyr Lys Asn Glu Ala
            100                 105                 110

His Arg Tyr Cys Ala Leu Ser His Asn Ser Ala Ala Asp Met Ser Ser
        115                 120                 125

Ala Ser Asn Asn Phe Val Phe Leu Lys Asn Glu Gly Leu Leu Asp Ile
    130                 135                 140

Ser Phe Met Leu Asn Ala Cys Tyr Asp Val Val Gly Glu Gly Ile Pro
145                 150                 155                 160

Phe Ser Pro Tyr Ile Cys Ala Gly Ile Gly Thr Asp Leu Val Ser Met
                165                 170                 175

Phe Glu Ala Thr Asn Pro Lys Ile Ser Tyr Gln Gly Lys Leu Gly Leu
            180                 185                 190

Ser Tyr Ser Ile Ser Pro Glu Ala Ser Val Phe Ile Gly Gly His Phe
        195                 200                 205

His Lys Val Ile Gly Asn Glu Phe Arg Asp Ile Pro Thr Ile Ile Pro
    210                 215                 220

Thr Gly Ser Thr Leu Ala Gly Lys Gly Asn Tyr Pro Ala Ile Val Ile
225                 230                 235                 240

Leu Asp Val Cys His Phe Gly Ile Glu Leu Gly Gly Arg Phe Val Phe
                245                 250                 255

<210>  64

<211>  284

<212>  PRT

<213>  Cowdria ruminantium



<400>  64

Met Asn Cys Lys Lys Ile Phe Ile Thr Ser Thr Leu Ile Ser Leu Val
1               5                   10                  15

Ser Phe Leu Pro Gly Val Ser Phe Ser Asp Val Ile Gln Glu Glu Asn
            20                  25                  30

Asn Pro Val Gly Ser Val Tyr Ile Ser Ala Lys Tyr Met Pro Thr Ala
        35                  40                  45

Ser His Phe Gly Lys Met Ser Ile Lys Glu Asp Ser Arg Asp Thr Lys
    50                  55                  60

Ala Val Phe Gly Leu Lys Lys Asp Trp Asp Gly Val Lys Thr Pro Ser
65                  70                  75                  80

Gly Asn Thr Asn Ser Ile Phe Thr Glu Lys Asp Tyr Ser Phe Lys Tyr
                85                  90                  95

Glu Asn Asn Pro Phe Leu Gly Phe Ala Gly Ala Val Gly Tyr Ser Met
            100                 105                 110

Asn Gly Pro Arg Ile Glu Phe Glu Val Ser Tyr Glu Thr Phe Asp Val
        115                 120                 125

Arg Asn Pro Gly Gly Asn Tyr Lys Asn Asp Ala His Met Tyr Cys Ala
    130                 135                 140

Leu Asp Thr Ala Ser Ser Ser Thr Ala Gly Ala Thr Thr Ser Val Asn
145                 150                 155                 160

Val Lys Asn Glu Asn Leu Thr Asp Ile Ser Leu Met Leu Asn Ala Cys
                165                 170                 175

Tyr Asp Ile Asn Leu Asp Gly Met Pro Val Ser Pro Tyr Val Cys Ala
            180                 185                 190

Gly Ile Gly Thr Asp Leu Val Ser Val Ile Asn Ala Thr Asn Pro Lys
        195                 200                 205

Leu Ser Tyr Gln Gly Lys Leu Gly Ile Ser Tyr Ser Ile Asn Pro Glu
    210                 215                 220

Ala Ser Ile Phe Ile Gly Gly His Phe His Arg Val Ile Gly Asn Glu
225                 230                 235                 240

Phe Lys Asp Ile Ala Thr Ser Lys Val Phe Thr Ser Ser Gly Asn Ala
                245                 250                 255

Ser Ser Ala Val Ser Pro Gly Phe Ala Ser Ala Ile Leu Asp Val Cys
            260                 265                 270

His Phe Gly Ile Glu Ile Gly Gly Arg Phe Val Phe
        275                 280